# EUROPÄISCHE PATENTSCHRIFT

(11) **EP 0 528 765 B1**
(45) Veröffentlichungstag und Bekanntmachung des Hinweises auf die Patenterteilung: **02.07.1997**
(21) Anmeldenummer: 92810610.3
(22) Anmeldetag: 11.08.1992
(51) Int. Cl.: C07D 513/04, A01N 43/90

(54) **Benzothiazolon Herbizide**
Benzothiazolon herbicide
Herbicides à base de benzothiazolone

(30) Priorität: 19.08.1991 CH 2431/91; 07.04.1992 CH 1124/92
(43) Veröffentlichungstag der Anmeldung: 24.02.1993
(73) Patentinhaber: Novartis AG, 4058 Basel (CH)
(72) Erfinder: Pissiotas, Georg, Dr., W-7850 Lörrach (DE); Moser, Hans, Dr., CH-4312 Magden (CH); Brunner, Hans-Georg, Dr., CH-4415 Lausen (CH)

(56) Entgegenhaltungen:
- EP-A- 0 304 920
- US-A- 4 830 659
- CHEMICAL ABSTRACTS, vol. 116, no. 23, 8. Juni 1992, Columbus, Ohio, US; abstract no. 235647u, M. YAMAGUCHI ET AL. 'Preparation of tetrahydropyridazinothiadiazoles as herbicides' Seite 832 ;

## Beschreibung

Die vorliegende Erfindung betrifft neue, herbizid wirksame Benzothiazolonderivate, Verfahren zu ihrer Herstellung, die sie als Wirkstoffe enthaltende Mittel sowie deren Verwendung zum Bekämpfen von Unkräutern, vor allem selektiv in Nutzpflanzenkulturen.

Herbizid wirksame Benzothiazolonderivate sind beispielsweise aus US-A-4 786 310 und EP-A-0 304 920 bekannt. Herbizid wirksame 9-(2H-1,4-Benzoxazin-3(4H)-on-6-yl)imino-8-thia-1,6-diazabicyclo[4.3.0]nonan-7-one sind aus US-A-4 830 659 bekannt.

Die erfindungsgemäßen Benzothiazolonderivate entsprechen der Formel I 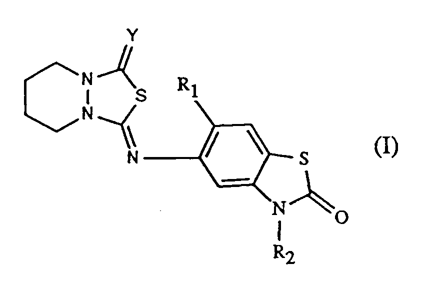 worin
Y Sauerstoff oder Schwefel;
R₁ Wasserstoff oder Fluor und
R₂ Wasserstoff, C₁-C₆-Alkyl, C₂-C₄-Alkenyl, oder C₂-C₆-Alkinyl; durch Halogen substituiertes C₁-C₆-Alkyl, C₂-C₄-Alkenyl, oder C₃-C₆-Alkinyl; C₁-C₄-Alkoxy-C₁-C₄-alkyl, C₁-C₄-Alkoxy-C₁-C₂-alkoxy-C₁-C₂-alkyl, 1-Phenylpropen-3-yl, durch Cyano oder C₃-C₆-Cycloalkyl substituiertes C₁-C₆-Alkyl; Carboxy-C₁-C₄-alkyl, C₁-C₆-Alkoxycarbonyl-C₁-C₄-alkyl, Halogen-C₁-C₆-alkoxycarbonyl-C₁-C₄-alkyl, C₁-C₄-Alkoxy-C₁-C₂-alkoxycarbonyl-C₁-C₄-alkyl, C₁-C₆-Alkoxycarbonyl-C₁-C₂-alkoxycarbonyl-C₁-C₄-alkyl, C₃-C₆-Cycloalkyl-C₁-C₂-alkoxycarbonyl-C₁-C₄-alkyl, C₁-C₅-Alkylaminocarbonyl-C₁-C₄-alkyl, Di-C₁-C₅-alkylaminocarbonyl-C₁-C₄-alkyl, C₃-C₆-Cycloalkyl, C₁-C₄-Alkythio-C₁-C₄-alkyl, Benzyl oder durch Halogen substituiertes Benzyl, C₁-C₄-Alkylsulfonyl, C₃-C₆-Alkenyloxy-C₁-C₄-alkyl, C₁-C₈-Alkylcarbonyl, 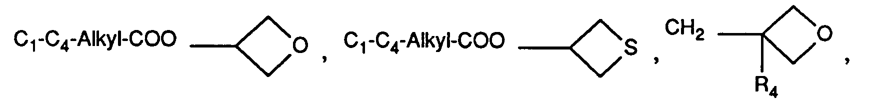 C₁-C₄-Alkylthiocarbonyl-C₁-C₄-alkyl, oder die Gruppe 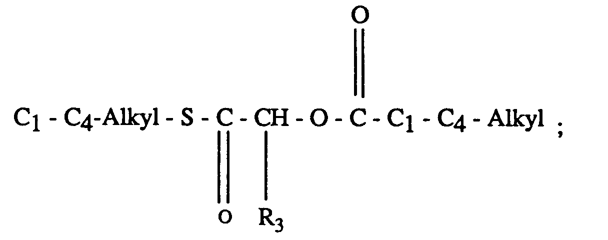
R₃ Wasserstoff oder C₁-C₄-Alkyl; und
R₄ Wasserstoff oder C₁-C₄-Alkyl bedeuten.

Die in der Substituentendefinition R₂ vorkommenden Alkylgruppen können geradkettig oder verzweigt sein und stehen beispielsweise für Methyl, Ethyl, n-Propyl, iso-Propyl, n-Butyl, sek.-Butyl, iso-Butyl, tert.-Butyl, Pentyl oder die isomeren Pentyle, Hexyl oder die isomeren Hexyle.

Halogenalkyl ist beispielsweise Fluormethyl, Difluormethyl, Trifluormethyl, Chlormethyl, Dichlormethyl, Trichlormethyl, 2,2,2-Trifluorethyl, 2-Fluorethyl, 2-Chlorethyl, 2,2,2-Trichlorethyl oder 7-Chlorheptyl; vorzugsweise Trichlormethyl, Difluorchlormethyl, Trifluormethyl und Dichlorfluormethyl.

Alkoxy ist beispielsweise Methoxy, Ethoxy, Propyloxy, i-Propyloxy, n-Butyloxy, iso-Butyloxy, sek.-Butyloxy und tert.-Butyloxy; vorzugsweise Methoxy und Ethoxy.

In den obigen Definitionen ist unter Halogen, Fluor, Chlor, Brom und Jod, vorzugsweise Fluor, Chlor und Brom zu verstehen.

C₁-C₄-Alkylsulfonyl steht beispielsweise für Methylsulfonyl, Ethylsulfonyl, n-Propylsulfonyl, iso-Propylsulfonyl, n-Butylsulfonyl, sek.-Butylsulfonyl, iso-Butylsulfonyl oder tert.-Butylsulfonyl.

Die C₂-C₄-Alkenylreste können in der Z-Form (cis) oder in der E-Form (trans) vorliegen und können geradkettig oder verzweigt sein. Bevorzugt sind Alkenylreste mit einer Kettenlänge von zwei bis drei Kohlenstoffatomen. Beispiele für C₂-C₄-Alkenylreste sind: Vinyl, Allyl, Methallyl, 1-Methylvinyl oder But-2-en-1-yl. Bevorzugt ist Vinyl und Allyl. Bei den C₂-C₄-Alkenylresten, welche durch Halogen substituiert sind, steht Halogen im einzelnen für Fluor, Chlor, Brom und Jod. Bevorzugte Halogenatome, die als Substituenten der C₂-C₄-Alkenylreste auftreten können, sind Fluor und Chlor. Unter den durch Halogen substituierten C₂-C₄-Alkenylresten sind diejenigen bevorzugt, die eine Kettenlänge von zwei bis drei Kohlenstoffatomen besitzen. Speziell bevorzugte, durch Halogen ein- bis dreifach substituierte C₂-C₄-Alkenylreste sind 1-Chlorvinyl, 2-Chlorvinyl, 3-Fluorallyl und 4,4,4-Trifluor-but-2-en-1-yl. Ganz besonders bevorzugt sind 1-Chlorvinyl und 2-Chlorvinyl.

Alkylthio ist beispielsweise Methylthio, Ethylthio, Propylthio, Isopropylthio, n-Butylthio, i-Butylthio, s-Butylthio oder t-Butylthio, vorzugsweise Methylthio und Ethylthio.

Die C₂-C₆-Alkinylreste können geradkettig oder verzweigt sein. Bevorzugt sind Alkenylreste mit einer Kettenlänge von 2 bis 3 Kohlenstoffatomen. C₂-C₆-Alkenylreste stehen beispielsweise für Ethinyl, Propargyl, 1-Propinyl, 3-Butinyl, 1-Methylpropargyl, 3-Pentinyl oder 3-Hexinyl, wobei Ethyl und Propargyl besonders bevorzugt sind.

Die in der Definition des Substituenten R₂ vorkommenden C₃-C₆-Cycloalkylgruppen können substituiert oder unsubstituiert sein und umfassen beispielsweise Cyclopropyl, 2-Fluorcyclopropyl, 2,4-Difluorcyclopropyl, 2-Chlorcyclopropyl, 2,3-Dichlorcyclopropyl, 2-Methylcyclopropyl, 2-Methylthiocyclopropyl, 2,3-Dimethylcyclopropyl, 2-Methoxycyclopropyl, Cyclobutyl, Cyclopentyl, 3-Methylcyclopentyl, 3,4-Dimethoxycyclopentyl, Cyclohexyl, 3-Fluorcyclohexyl, 4-Methylcyclohexyl oder 4-Methylthiocyclohexyl.

Von den Verbindungen der Formel I sind diejenigen bevorzugt, in denen R₁ für Fluor steht, wobei vorzugsweise Y für Sauerstoff steht.

Besonders bevorzugt sind diejenigen Verbindungen der Formel I, in denen R₂ Wasserstoff, C₁-C₄-Alkyl, C₂-C₄-Alkenyl, oder C₂-C₄-Alkinyl; durch Halogen substituiertes C₁-C₄-Alkyl, C₂-C₄-Alkenyl, oder C₃-C₄-Alkinyl; C₁-C₄-Alkoxy-C₁-C₄-alkyl, C₁-C₄-Alkoxy-C₁-C₂-alkoxy-C₁-C₂-alkyl, 1-Phenylpropen-3-yl, durch Cyano oder C₃-C₆-Cycloalkyl substituiertes C₁-C₄-Alkyl, Benzyl oder durch Halogen substituiertes Benzyl, C₃-C₆-Alkenyloxy-C₁-C₄-alkyl, C₁-C₈-Alkylcarbonyl, 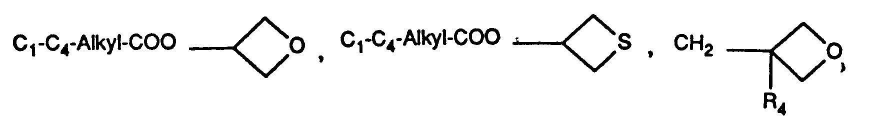 C₁-C₄-Alkylthiocarbonyl-C₁-C₄-alkyl, oder die Gruppe 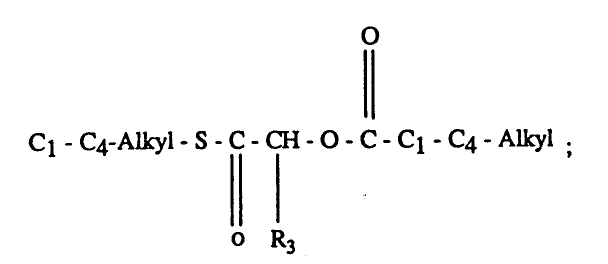 R₃ Wasserstoff oder C₁-C₄-Alkyl; und
R₄ Wasserstoff oder C₁-C₄-Alkyl bedeuten.

Ferner sind Verbindungen der Formel I von besonderem Interesse, in denen R₂ Wasserstoff, C₁-C₄-Alkyl, C₂-C₄-Alkenyl, oder C₂-C₄-Alkinyl; durch Halogen substituiertes C₁-C₄-Alkyl, C₂-C₄-Alkenyl, oder C₃-C₄-Alkinyl; oder C₁-C₂-Alkoxy-C₁-C₂-alkyl bedeuten.

Ganz besonders bevorzugt sind diejenigen Verbindungen der Formel I, in denen R₂ Wasserstoff, C₁-C₄-Alkyl, C₂-C₄-Alkenyl, durch Fluor substituiertes C₁-C₄-Alkyl oder C₂-C₄-Alkenyl bedeuten.

In einer weiteren herausragenden Gruppe von Verbindungen der Formel I bedeutet R₂ C₃-C₆-Cycloalkyl, durch C₃-C₆-Cycloalkyl substituiertes C₁-C₆-Alkyl; C₁-C₄-Alkylthio-C₁-C₄-alkyl, Benzyl, oder durch Halogen substituiertes Benzyl, C₁-C₄-Alkylsulfonyl, C₃-C₆-Alkenyloxy-C₁-C₄-alkyl, C₁-C₈-Alkylcarbonyl, 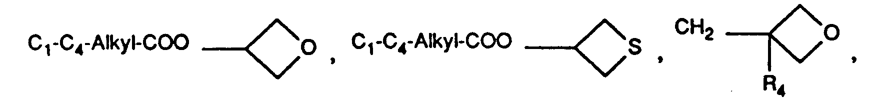 C₁-C₄-Alkylthiocarbonyl-C₁-C₄-alkyl, oder die Gruppe 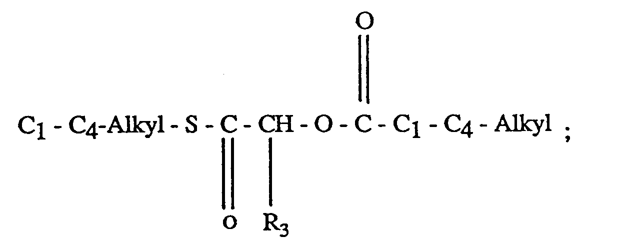 R₃ Wasserstoff oder C₁-C₄-Alkyl; und
R₄ Wasserstoff oder C₁-C₄-Alkyl, wobei diejenigen Verbindungen besonders hervorzuheben sind, in denen Y Sauerstoff, R₁ Fluor und R₃ Wasserstoff oder Methyl bedeuten.

In einer ganz besonders bevorzugten Gruppe von Verbindungen der Formel I stehen Y für Sauerstoff, R₁ für Fluor und R₂ für C₁-C₃-Alkyl, CH₂F, CH₂CH₂F, CH₂CH₂F, CHF₂, Allyl, Propargyl, 1-Methyl-3-propinyl, Methoxycarbonylmethyl, Ethoxycarbonylmethyl, Isopropoxycarbonylmethyl, 1-(Methoxycarbonyl)-ethyl, 1-(Ethoxycarbonyl)-ethyl oder 1-(Isopropoxycarbonyl)-ethyl.
Als bevorzugte Einzelverbindung aus dem Umfang der Formel I sei genannt: 9-[6-Fluor-3-isopropyl-2(3H)-benzothiazolon-5-yl]imino-8-thia-1,6-diazabicyclo[4.3.0]nonan-7-on (Verbindung Nr. 1.001).

In einer weiteren, besonders bevorzugten Untergruppe von Verbindungen der Formel I bedeutet
R₂ durch Halogen substituiertes C₁-C₆-Alkyl, C₂-C₄-Alkenyl, oder C₃-C₆-Alkinyl; C₁-C₄-Alxoxy-C₁-C₄-alkyl, C₁-C₄-Alkoxy-C₁-C₂-alkoxy-C₁-C₂-alkyl, durch Cyano oder C₃-C₆-Cycloalkyl substituiertes C₁-C₆-Alkyl; Carboxy-C₁-C₄-alkyl, C₁-C₆-Alkoxycarbony-C₁-C₄-alkyl, C₁-C₄-Alkoxy-C₁-C₂-alkoxycarbonyl-C₁-C₄-alkyl, C₁-C₆-Alkoxycarbonyl-C₁-C₂-alkoxycarbonyl-C₁-C₄-alkyl, C₃-C₆-Cycloalkyl-C₁-C₂-alkoxycarbonyl-C₁-C₄-alkyl, C₁-C₅-Alkylaminocarbonyl-C₁-C₄-alkyl, Di-C₁-C₅-alkylaminocarbonyl-C₁-C₄-alkyl, C₃-C₆-Cycloalkyl, C₁-C₄-Alkylthio-C₁-C₄-alkyl, C₃-C₆-Alkenyloxy-C₁-C₄-alkyl, 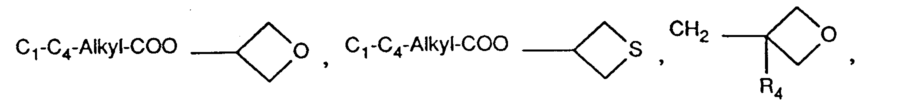 C₁-C₄-Alkylthiocarbonyl-C₁-C₄-alkyl, oder die Gruppe 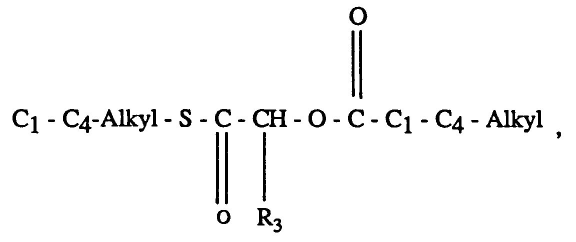 wobei R₁ vorzugsweise für Fluor steht und Y Sauerstoff bedeutet.

Aus dieser Gruppe sind insbesondere diejenigen Verbindungen bevorzugt, worin
R₂ C₁-C₄-Alkoxy-C₁-C₄-alkyl, C₁-C₄-Alkoxy-C₁-C₂-alkoxy-C₁-C₂-alkyl, durch Cyano substituiertes C₁-C₆-Alkyl; Carboxy-C₁-C₄-alkyl, C₁-C₆-Alkoxycarbonyl-C₁-C₄-alkyl, C₁-C₄-Alkoxy-C₁-C₂-alkoxycarbonyl-C₁-C₄-alkyl, C₁-C₆-Alkoxycarbonyl-C₁-C₂-alkoxycarbonyl-C₁-C₄-alkyl, C₃-C₆-Cycloalkyl-C₁-C₂-alkoxycarbonyl-C₁-C₄-alkyl, C₁-C₅-Alkylaminocarbonyl-C₁-C₄-alkyl, Di-C₁-C₅-alkylaminocarbonyl-C₁-C₄-alkyl oder C₃-C₆-Cycloalkyl, ganz besonders bevorzugt aber C₁-C₆-Alkoxycarbonyl-C₁-C₄-alkyl oder C₁-C₄-Alkoxy-C₁-C₂-alkoxycarbonyl-C₁-C₄-alkyl bedeutet.

Die Verbindungen der Formel I werden hergestellt, indem man ein Isothiocyanat der Formel II 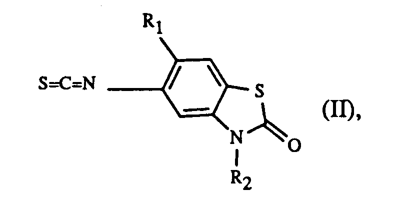 worin die Substituenten R₁ und R₂ die unter Formel I angegebene Bedeutung haben, mit einem Hexahydropyridazin der Formel III 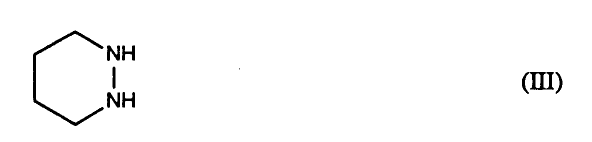 in die Verbindung der Formel IV 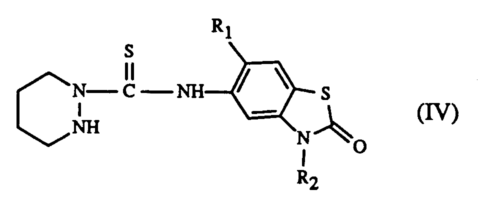 überführt und diese anschließend mit einer Verbindung der Formel V

CYCl₂ (V),

worin Y für Sauerstoff oder Schwefel steht, in Gegenwart einer Base umsetzt.

Die Umsetzung der Isothiocyanate der Formel II mit dem Hexahydropydazin der Formel III wird mit Vorteil in einem reaktionsinerten Lösungsmittel bei Temperaturen von -5°C bis zur Siedetemperatur des Lösungsmittels, insbesondere von 0 bis +50°C, besonders bevorzugt bei Raumtemperatur, durchgeführt. Geeignete Lösungsmittel für diese Reaktion sind beispielsweise Toluol, Xylol, Essigsäureethylester oder Acetonitril.

Die Umsetzung der Verbindung der Formeln IV mit der Verbindung der Formel V erfolgt vorteilhaft in einem reaktionsinerten Lösungsmittel bei niedrigen Temperaturen, vorzugsweise bei 0 bis +50°C, besonders bevorzugt bei 0 bis +15°C. Geeignete Basen für diese Reaktion sind beispielsweise Pyridin, Triethylamin oder N,N-Dimethylanilin. Als Lösungsmittel eignen sich beispielsweise 1,2-Dichlorethan, Dichlormethan oder Toluol.

Das für die erfindungsgemäßen Verbindungen der Formel I, als Ausgangsprodukte verwendeten Hexahydropyridazin der Formel III ist aus EP-A-0 304 920 bekannt. Die Isothiocyanate der Formel II sowie deren Herstellung ist in USP 4,828,605 beschrieben.

Die vorstehend aufgeführten Verfahren können analog zu literaturbekannten Verfahren durchgeführt werden. Die in diesen Verfahren bevorzugten Reaktionsbedingungen, wie Temperatur, molare Verhältnisse der Edukte, Reaktionsführung. Lösungsmittel, gegebenenfalls erforderliche Reagentien, wie Säuren, Basen, wasserbindende Mittel usw., sind dem Fachmann geläufig.

Die Verbindungen der Formel I werden in unveränderter Form, wie aus der Synthese erhältlich, oder vorzugsweise mit den in der Formulierungstechnik üblichen Hilfsmitteln eingesetzt und werden der z.B. zu emulgierbaren Konzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Benetzen, Verstreuen oder Gießen werden gleich wie die Art der Mittel den angestrebten Zielen und den gegebenen Verhältnissen entsprechend gewählt.

Die Formulierungen, d.h. die den Wirkstoff der Formel I und gegebenenfalls einen oder mehrere feste oder flüssige Zusatzstoffe enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen und gegebenenfalls oberflächenaktiven Verbindungen (Tensiden).

Als Lösungsmittel können in Frage kommen: Aromatische Kohlenwasserstoffe, insbesondere die Fraktionen C₈ bis C₁₂, wie Mischungen von Alkylbenzolen, z.B. Xylolgemische oder alkylierte Naphthaline; aliphatische und cycloaliphatische Kohlenwasserstoffe wie Paraffine, Cyclohexan oder Tetrahydronaphthalin; Alkohole, wie Ethanol, Propanol oder Butanol; Glykole sowie deren Ether und Ester, wie Propylenglykol oder Dipropylenglykolether, Ketone wie Cyclohexanon, Isophoron oder Diacetonalkohol, starke polare Lösungsmittel wie N-Methyl-2-pyrrolidon, Dimethylsulfoxid oder Wasser, Pflanzenöle sowie deren Ester, wie Raps-, Ricinus- oder Sojaöl; gegebenenfalls auch Silikonöle.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit; als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali oder gegebenenfalls substituierten Ammoniumsalze von höheren Fettsäuren (C₁₀-C₂₂), wie z.B. die Na- oder K-Salze der Öl- oder Stearinsäure, oder von natürlichen Fettsäuregemischen genannt, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, die Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen, enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht ionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside sind u.a. in folgenden Publikationen beschrieben:
- "Mc Cutcheon's Detergents and Emulsifiers Annual", Mc Publishing Corp., Glen Rock, New Jersey, 1988.
- M. and J. Ash, "Encyclopedia of Surfactants", Vol. I-III, Chemical Publishing Co., New York, 1980-1981.
- Dr. Helmut Stache "Tensid-Taschenbuch", Carl Hanser Verlag, München/Wien 1981.

Die pestiziden Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten.

Insbesondere setzen sich bevorzugte Formulierungen folgendermaßen zusammen: (% = Gewichtsprozent)

### Emulgierbare Konzentrate:

- Aktiver Wirkstoff:: 1 bis 90 %, vorzugsweise 5 bis 20 %
- oberflächenaktives Mittel:: 1 bis 30 %, vorzugsweise 10 bis 20 %
- flüssiges Trägermittel:: 5 bis 94 %, vorzugsweise 70 bis 85 %

### Stäube:

- Aktiver Wirkstoff:: 0,1 bis 10 %, vorzugsweise 0,1 bis 1 %
- festes Trägermittel:: 99,9 bis 90 %, vorzugsweise 99,9 bis 99 %

### Suspensions-Konzentrate:

- Aktiver Wirkstoff:: 5 bis 75 %, vorzugsweise 10 bis 50 %
- Wasser:: 94 bis 24 %, vorzugsweise 88 bis 30 %
- oberflächenaktives Mittel:: 1 bis 40 %, vorzugsweise 2 bis 30 %

### Benetzbare Pulver:

- Aktiver Wirkstoff:: 0,5 bis 90 %, vorzugsweise 1 bis 80 %
- oberflächenaktives Mittel:: 0,5 bis 20 %, vorzugsweise 1 bis 15 %
- festes Trägermaterial:: 5 bis 95 %, vorzugsweise 15 bis 90 %

### Granulate:

- Aktiver Wirkstoff:: 0,5 bis 30 %, vorzugsweise 3 bis 15 %
- festes Trägermittel:: 99,5 bis 70 %, vorzugsweise 97 bis 85 %

Die Wirkstoffe der Formel I werden in der Regel mit Aufwandmengen von 0,001 bis 4 kg/ha insbesondere 0,005 bis 1 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und herbizide Eigenschaften aus, die sie ausgezeichnet zum Einsatz in Kulturen von Nutzpflanzen, insbesondere in Getreide, Baumwolle, Soja, Raps, Mais und Reis befähigen.

Die Erfindung betrifft auch herbizide Mittel, welche einen neuen Wirkstoff der Formel I enthalten, sowie Verfahren zur Hemmung des Pflanzenwuchses.

### Herstellungsbeispiele:

### Beispiel H1: Herstellung von 2-[6-Fluor-3-(1-Methylethyl)-2-benzothiazolon-5-yl-aminothiocarbonyl]-hexahydropyridazin:

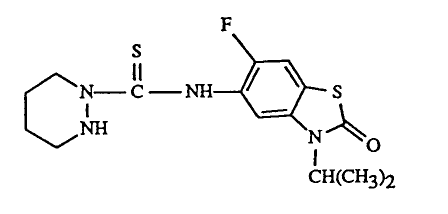

Zu einer Lösung von 3,6 ml Hexahydropyridazin in 50 ml Ethanol gibt man bei einer Temperatur von +5°C eine Lösung von 10,8 g 6-Fluor-3-(1-Methylethyl)-2-benzothiazolon-5-yl-isothiocyanat in 150 ml Ethanol tropfenweise hinzu. Nach 12-stündigem Rühren bei Raumtemperatur wird die Reaktionsmischung eingedampft. Nach dem Reinigen mittels Kieselgelchromatographie (Essigsäureethylester/n-Hexan 1 : 9) erhält man 6,9 g 2-[6-Fluor-3-(1-Methylethyl)-2-benzothiazolon-5-yl-aminothiocarbonyl]-hexahydropyridazin mit einem Schmelzpunkt von 198-199°C.

### Beispiel H2: Herstellung von 9-[6-Fluor-3-isopropyl-2(3H)-benzothiazolon-5-yl]imino-8-thia-1,6-diazabicyclo[4.3.0]nonan-7-on (Verbindung Nr. 1.001):

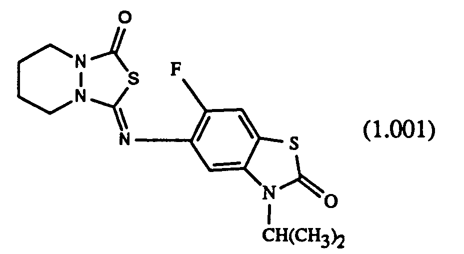

Zu einer Lösung von 4,4 g gemäß Beispiel H1 hergestelltem 2-[6-Fluor-3-(1-Methylethyl)-2-benzothiazolon-5-yl-aminothiocarbonyl]-hexahydropyridazin gibt man bei einer Temperatur von +5°C 7 ml 20%ige toluolische Lösung von Phosgen tropfenweise hinzu. Nach einstündigem Rühren wird die Reaktionsmischung in Eiswasser gegeben. Nach Abtrennen der organischen Phase und Trocknen über Natriumsulfat wird die Lösung eingedampft. Nach chromatographischer Reinigung über Kieselgel (Essigester/n-Hexan 1 : 1) erhält man 2,9 g 9-[3-(1-Methylethyl)-6-fluor-2(3H)-benzothiazolon-5-yl)-8-thia-1,6-diazabicyclo[4.3.0]nonan-7-on (Verbindung Nr. 1.001) mit einem Schmelzpunkt von 132-133°C.

Analog zu vorstehendem Beispiel und den in der Beschreibung genannten Herstellungsverfahren können die Verbindungen der Tabelle 1 hergestellt werden:

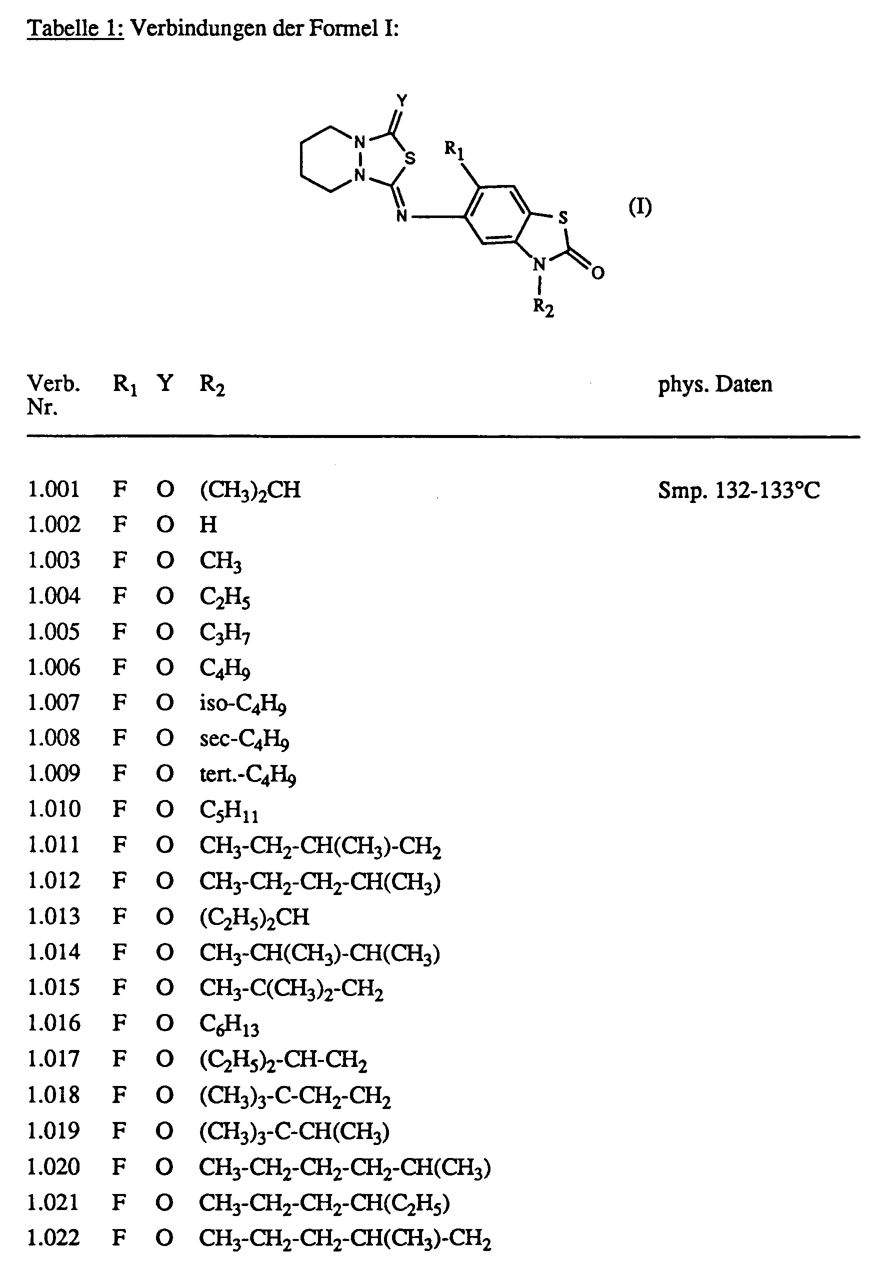

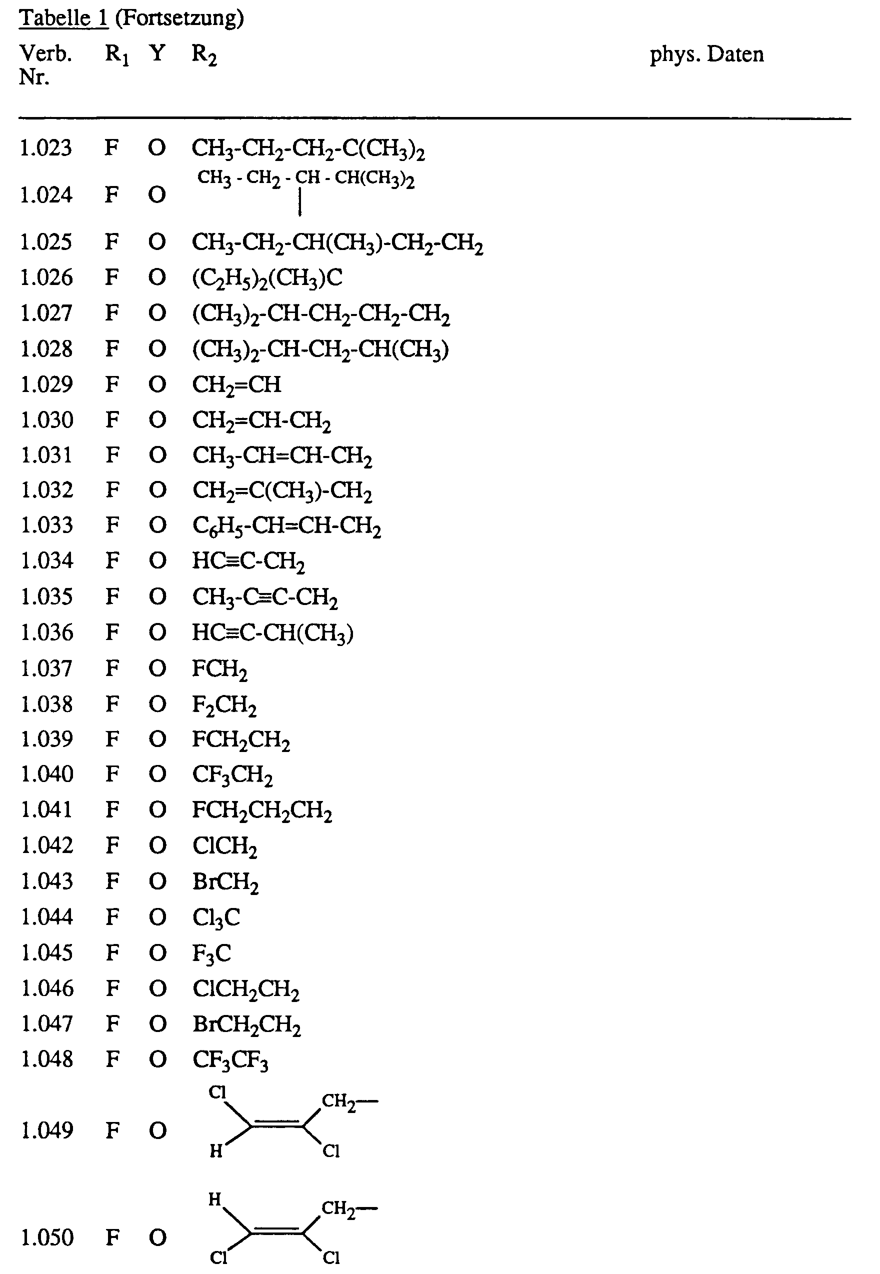

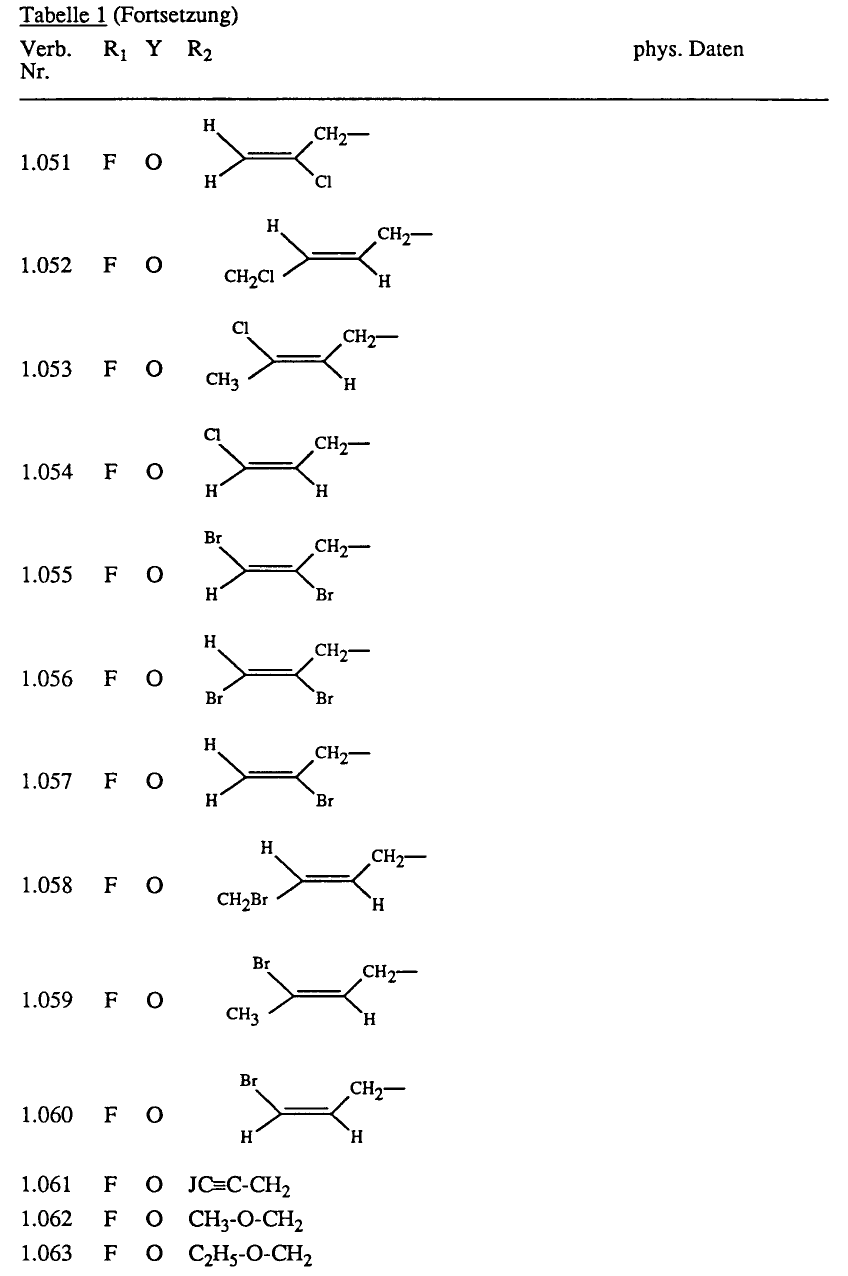

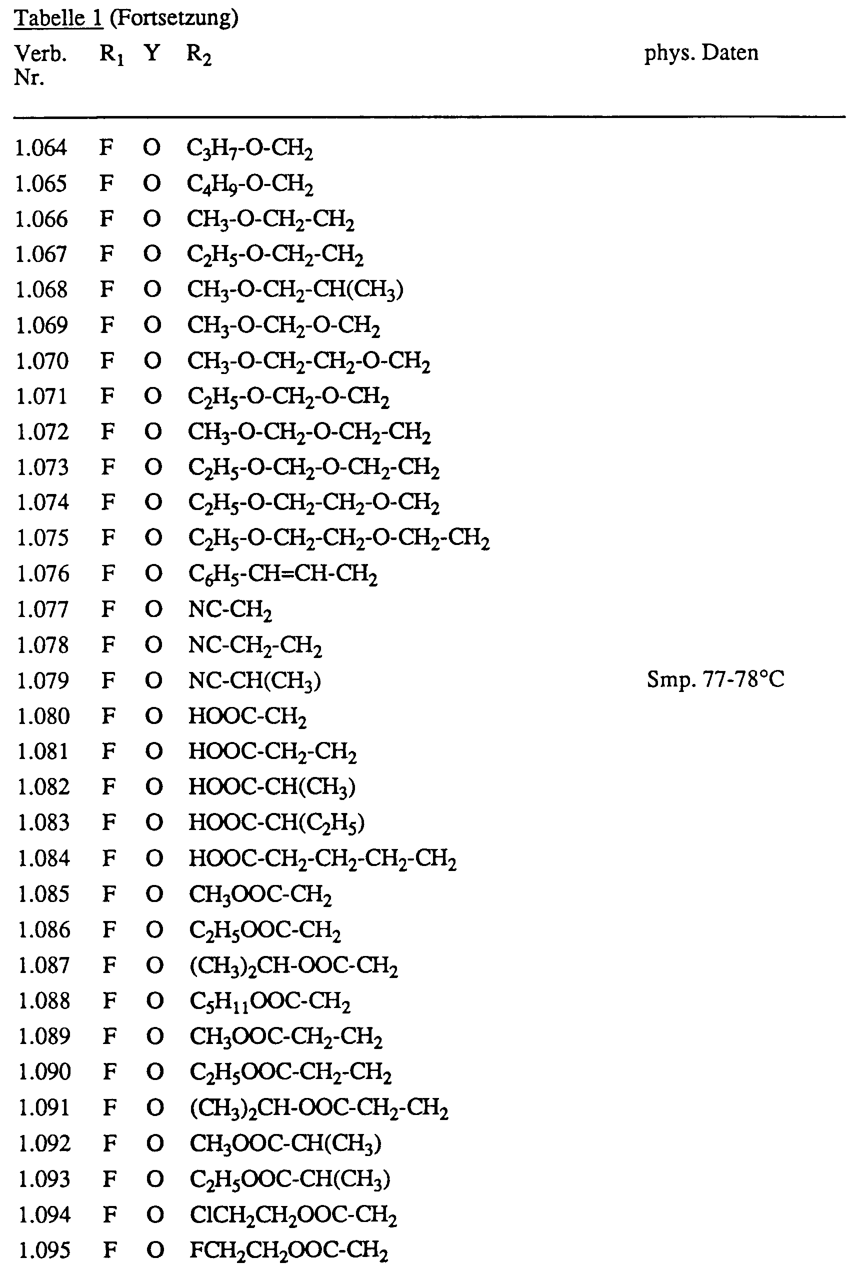

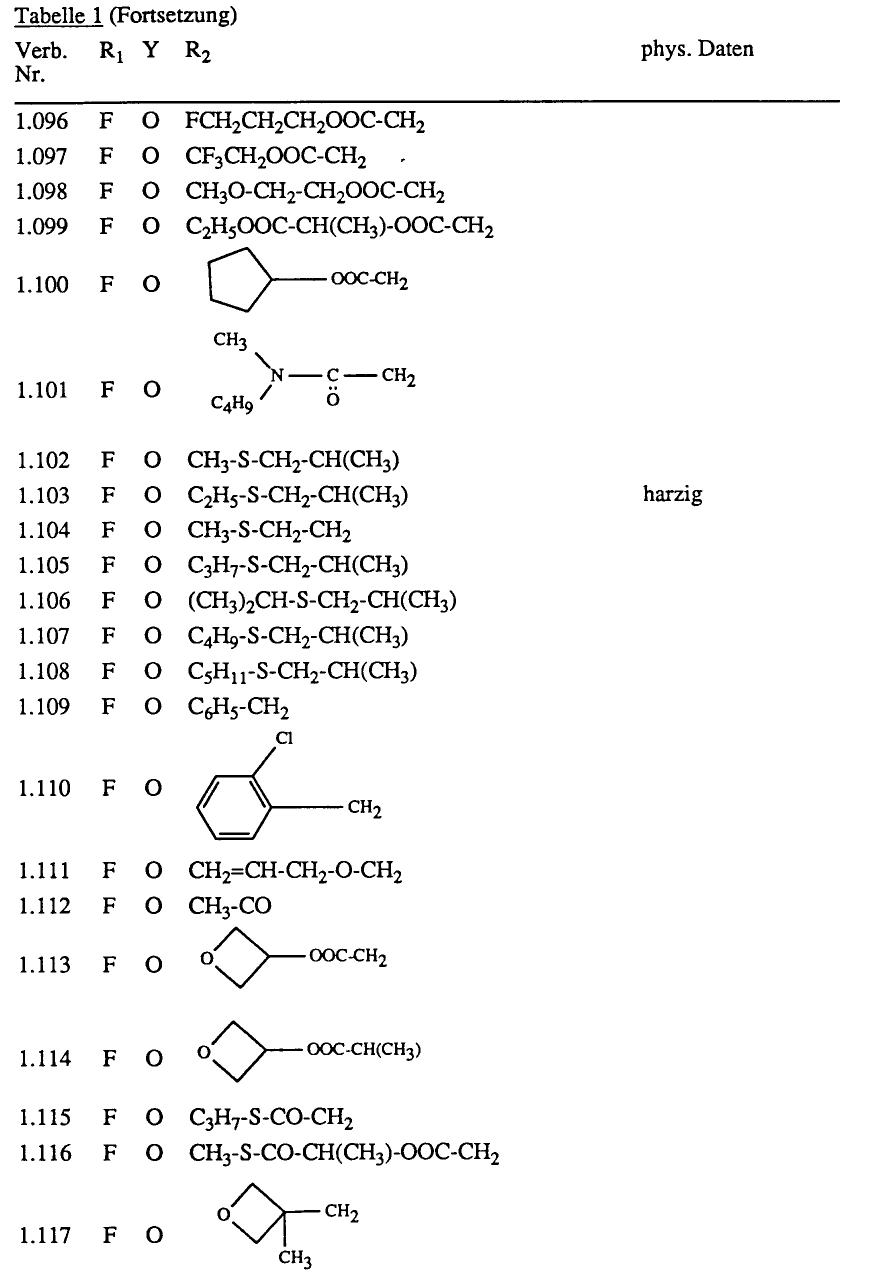

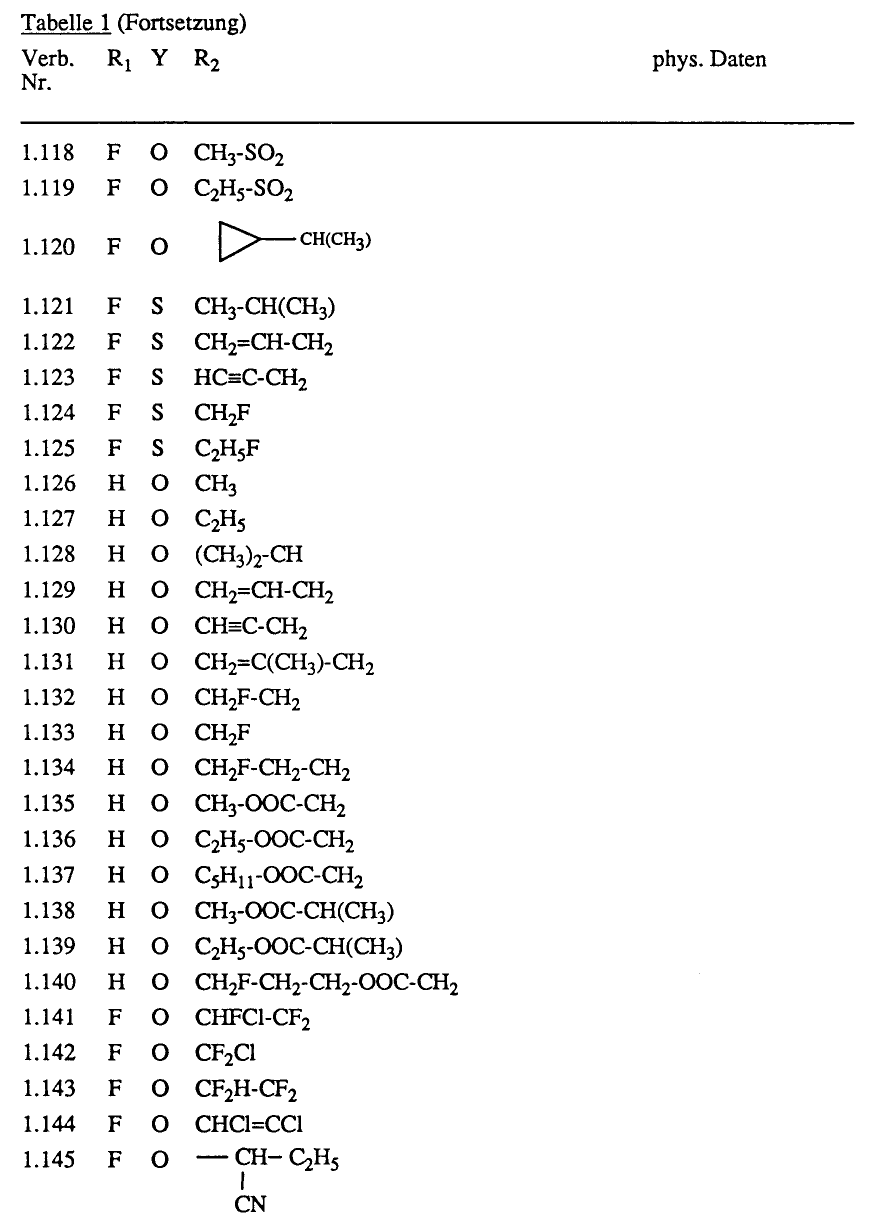

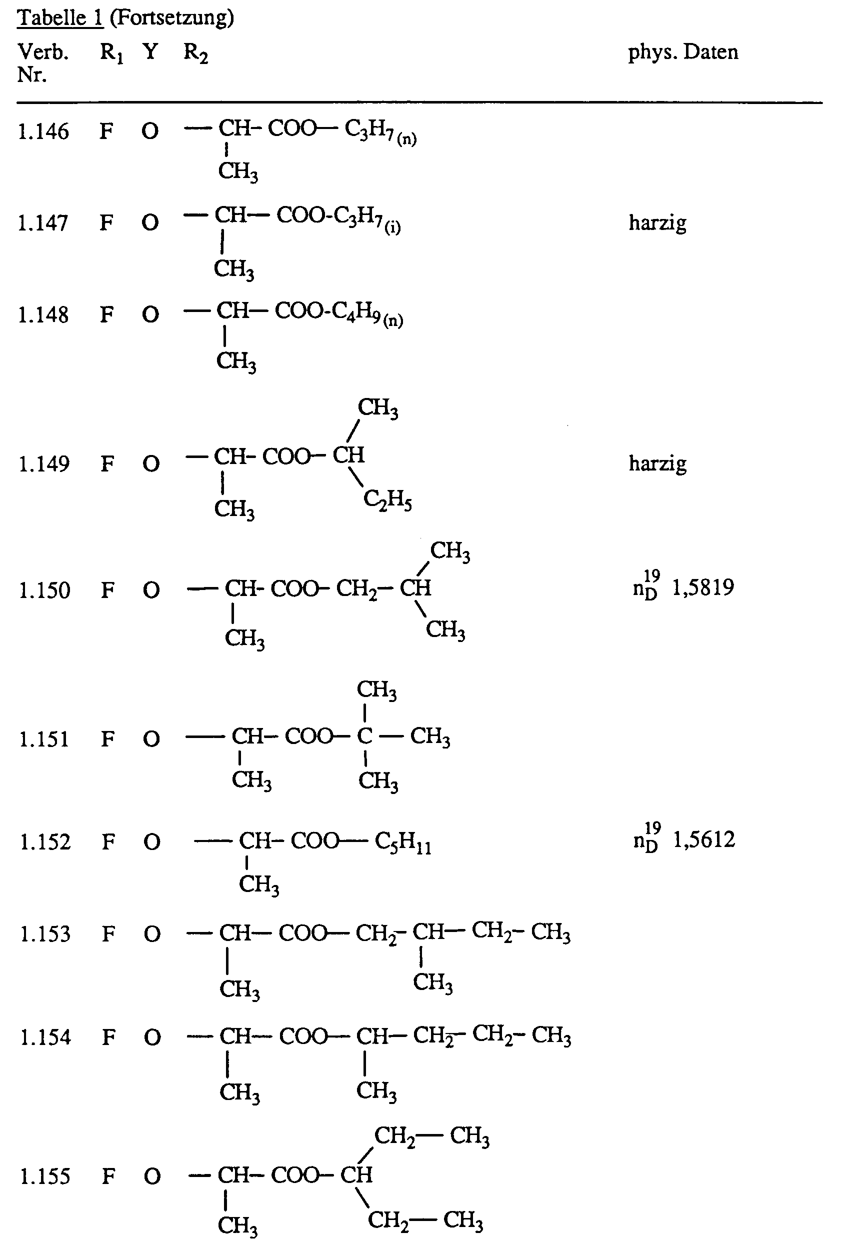

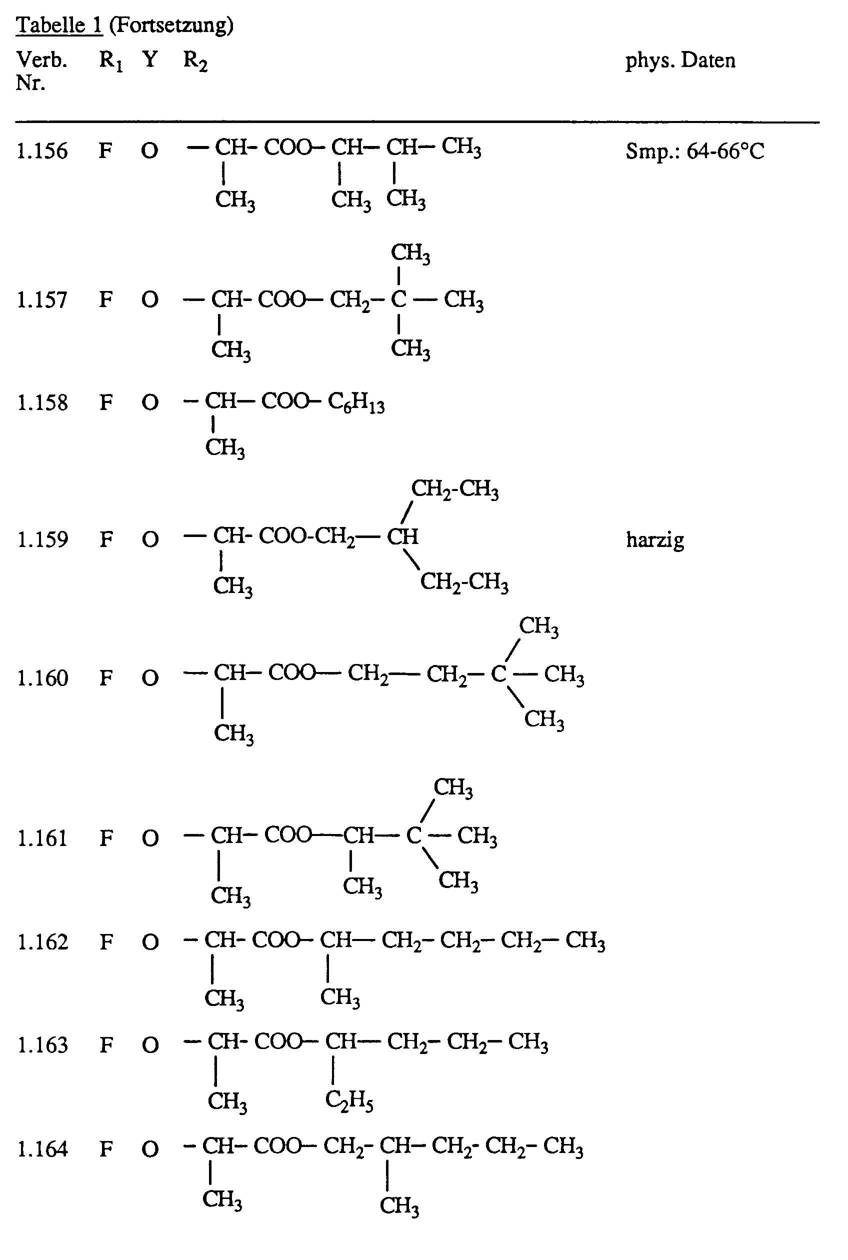

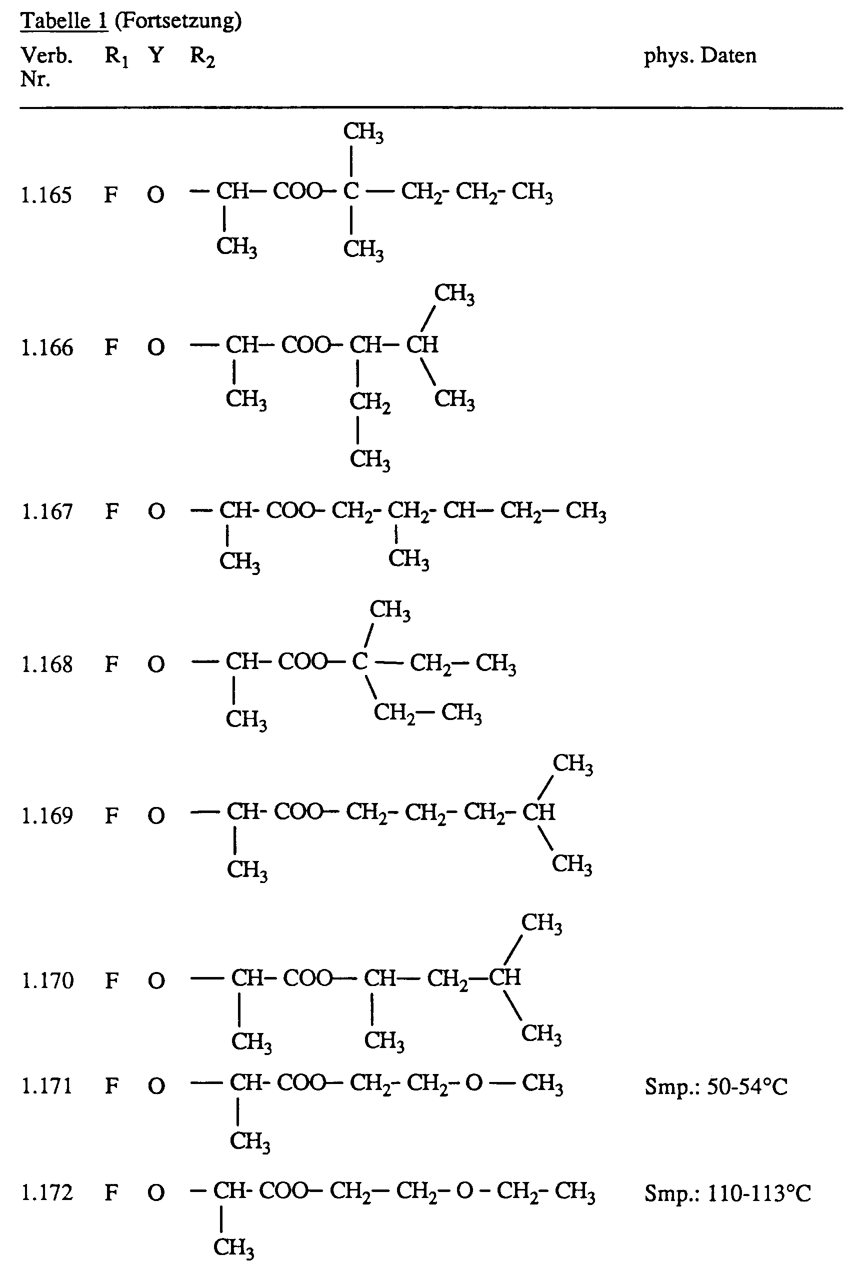

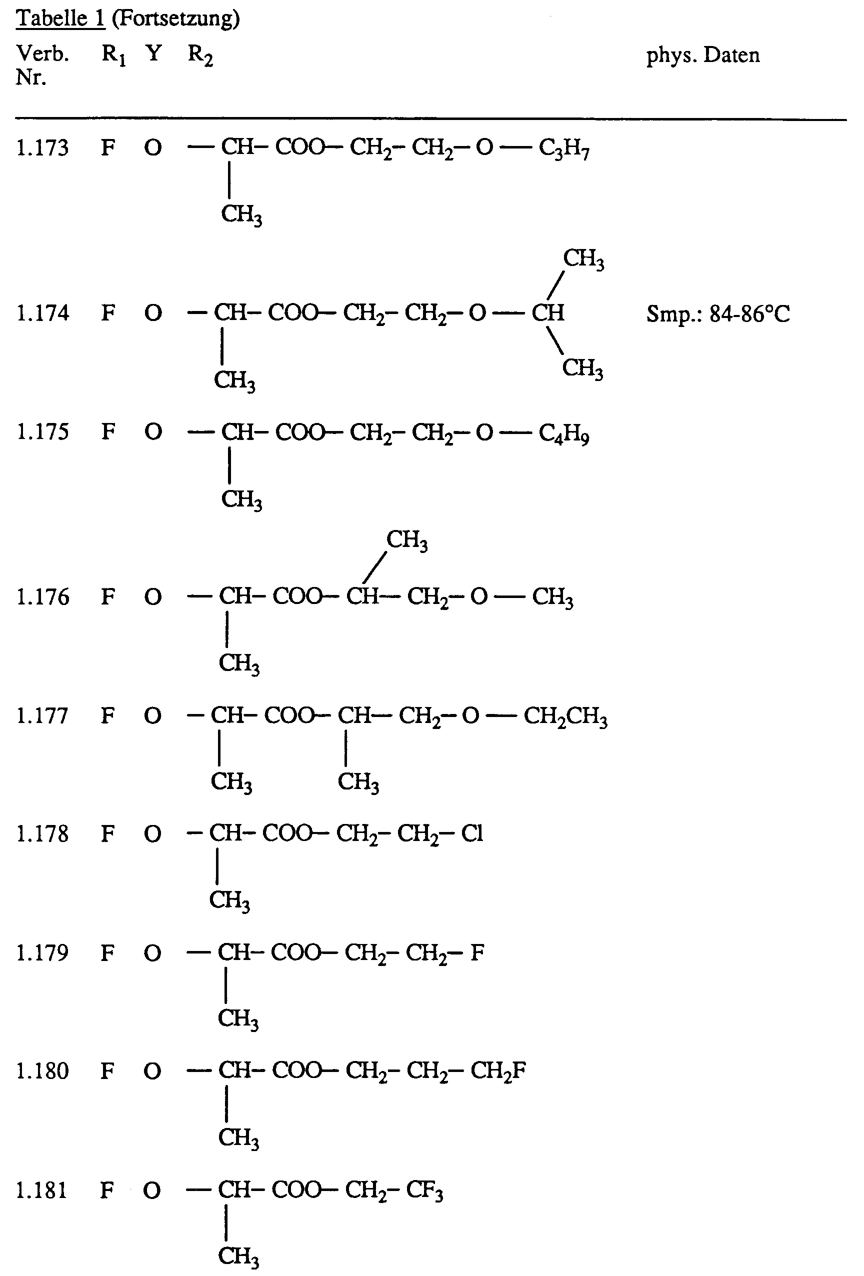

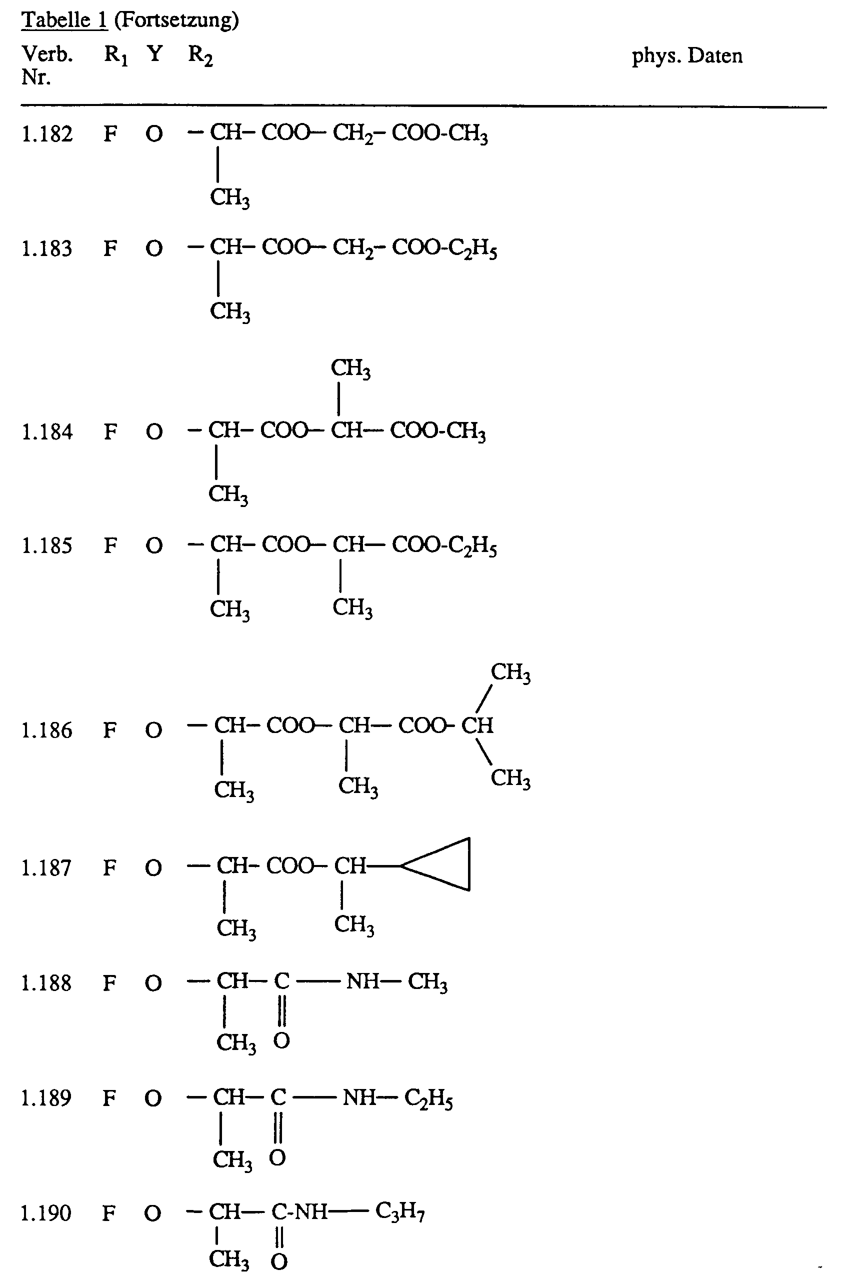

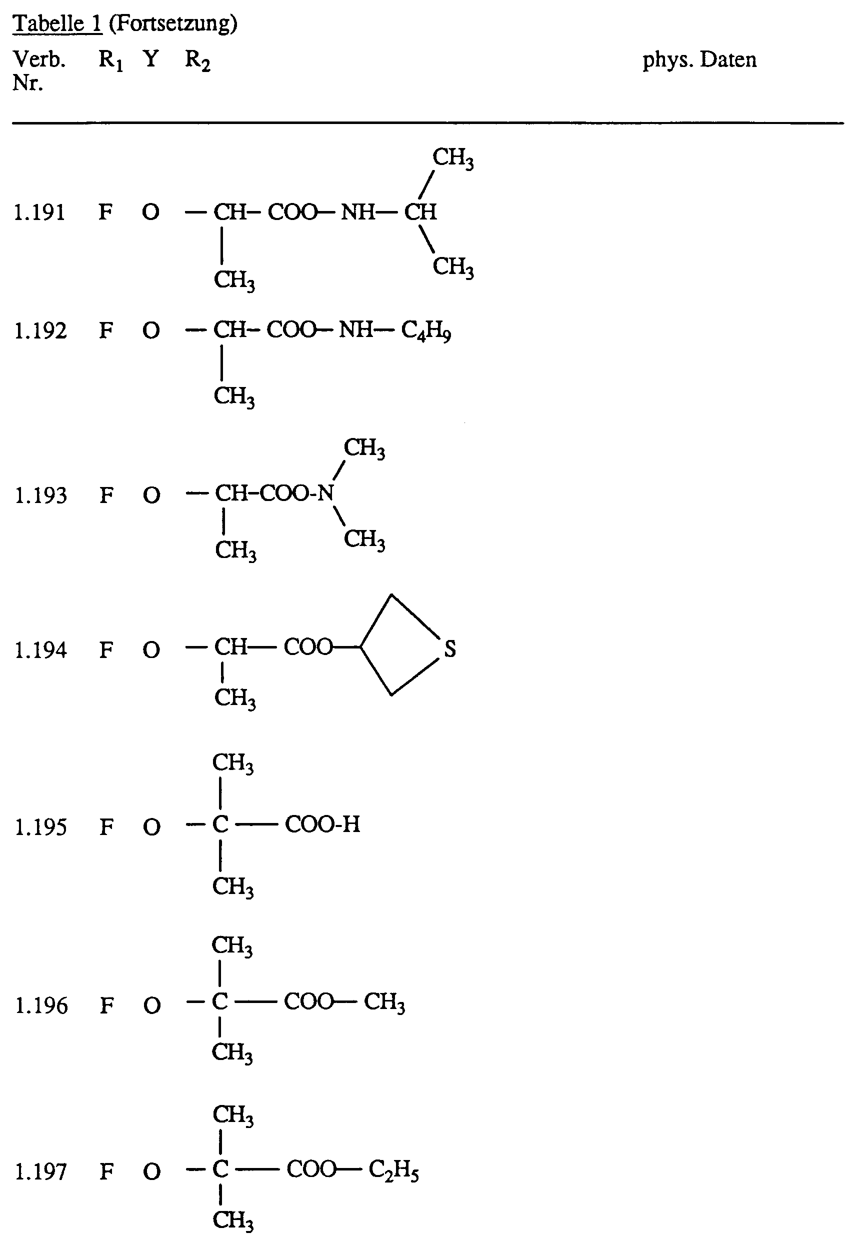

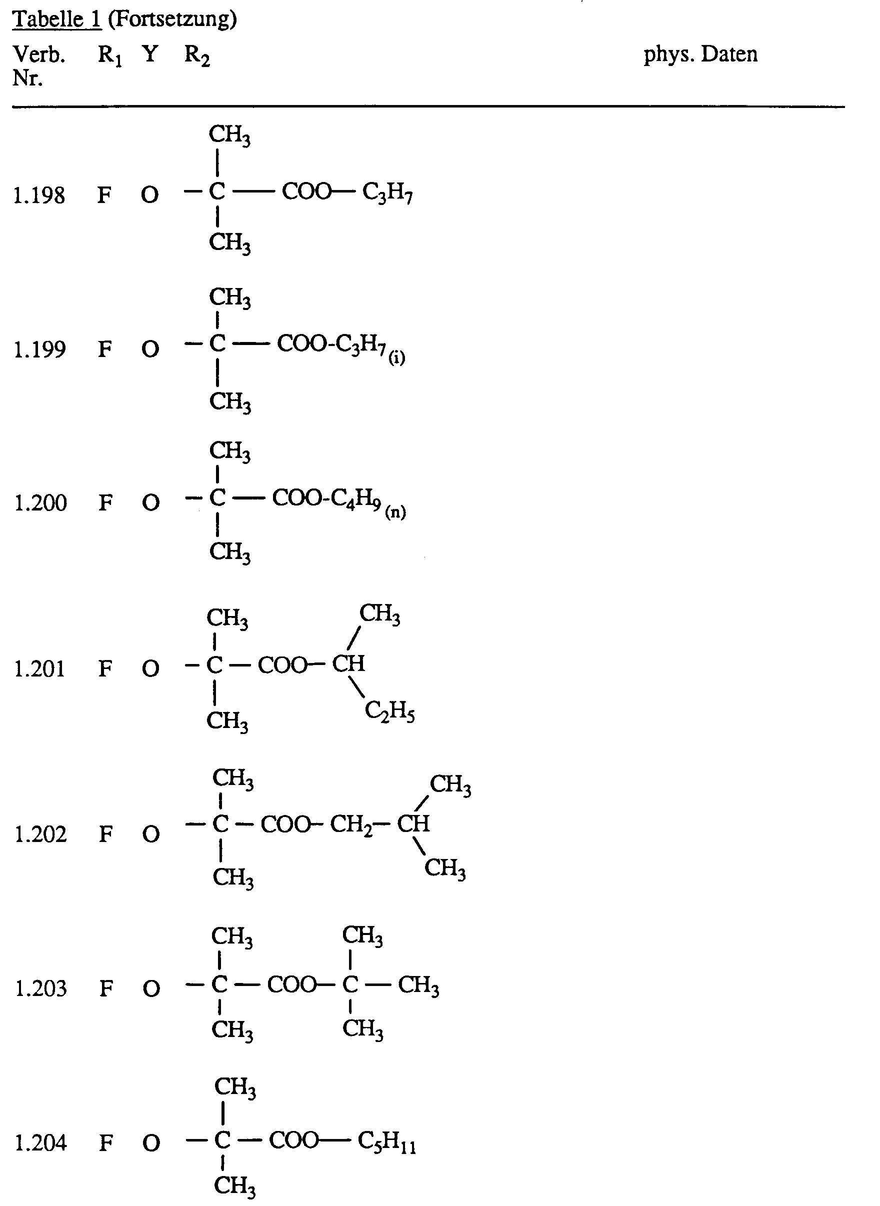

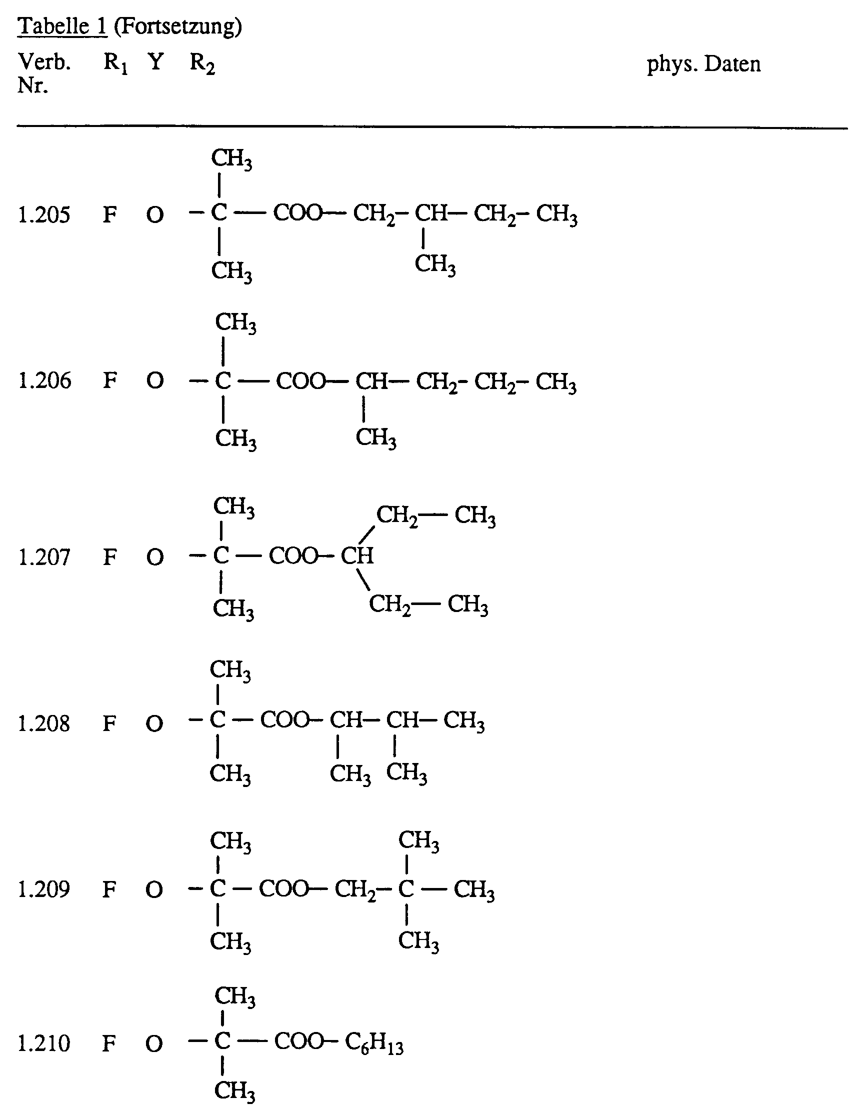

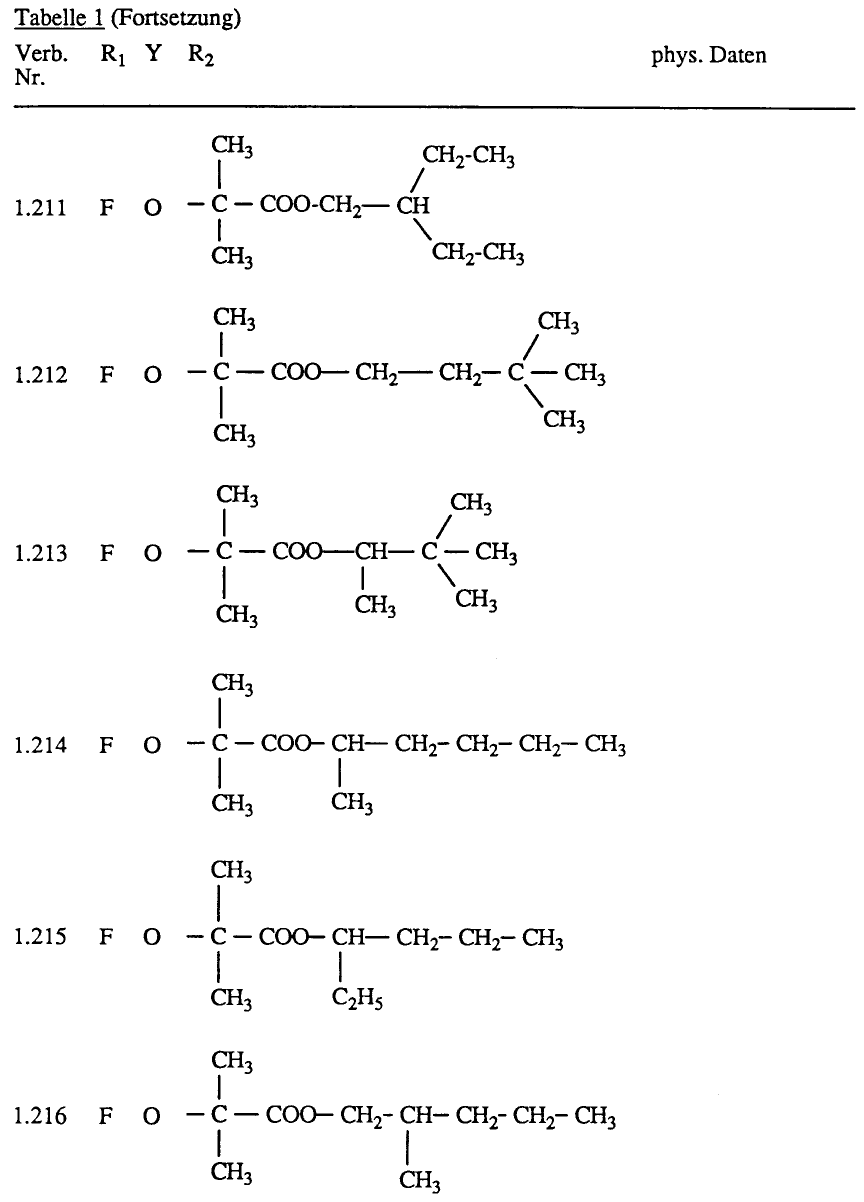

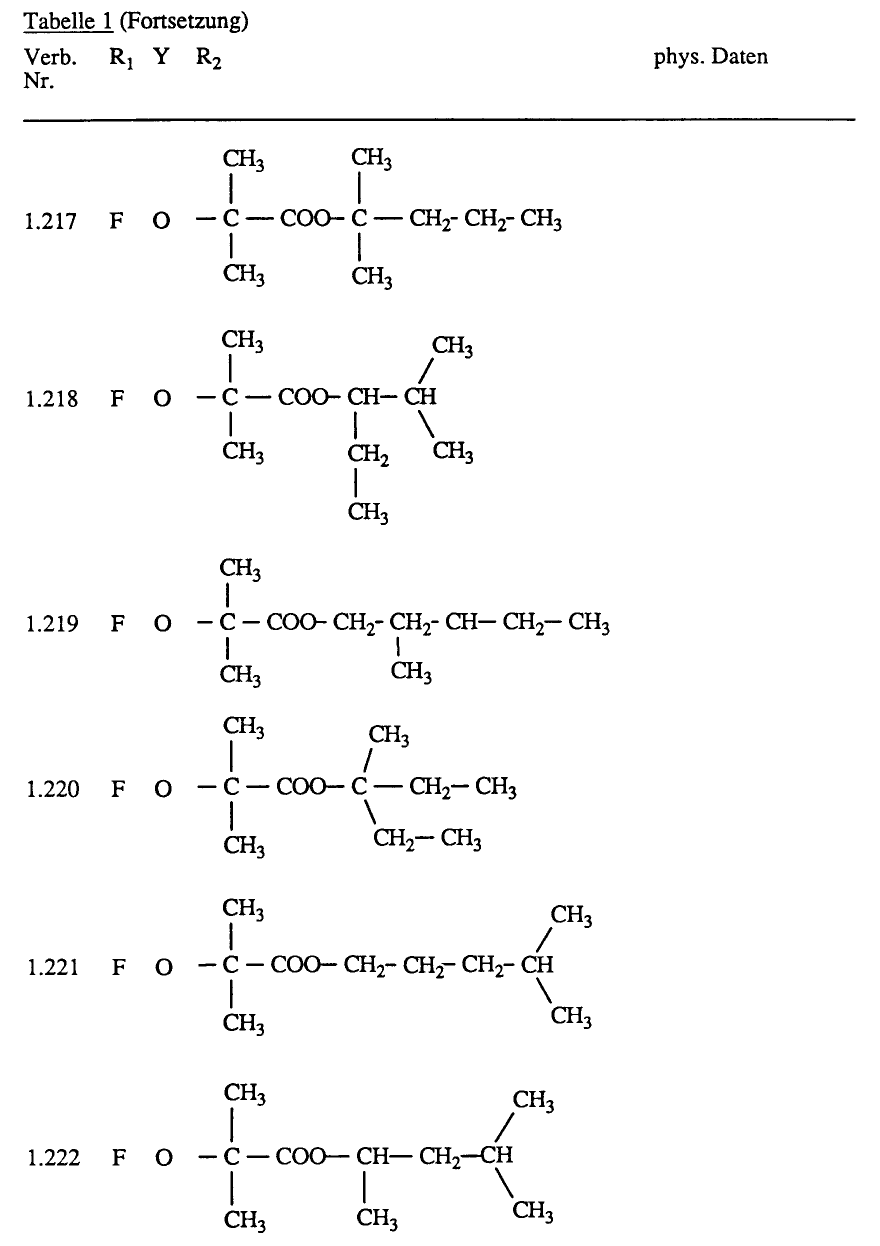

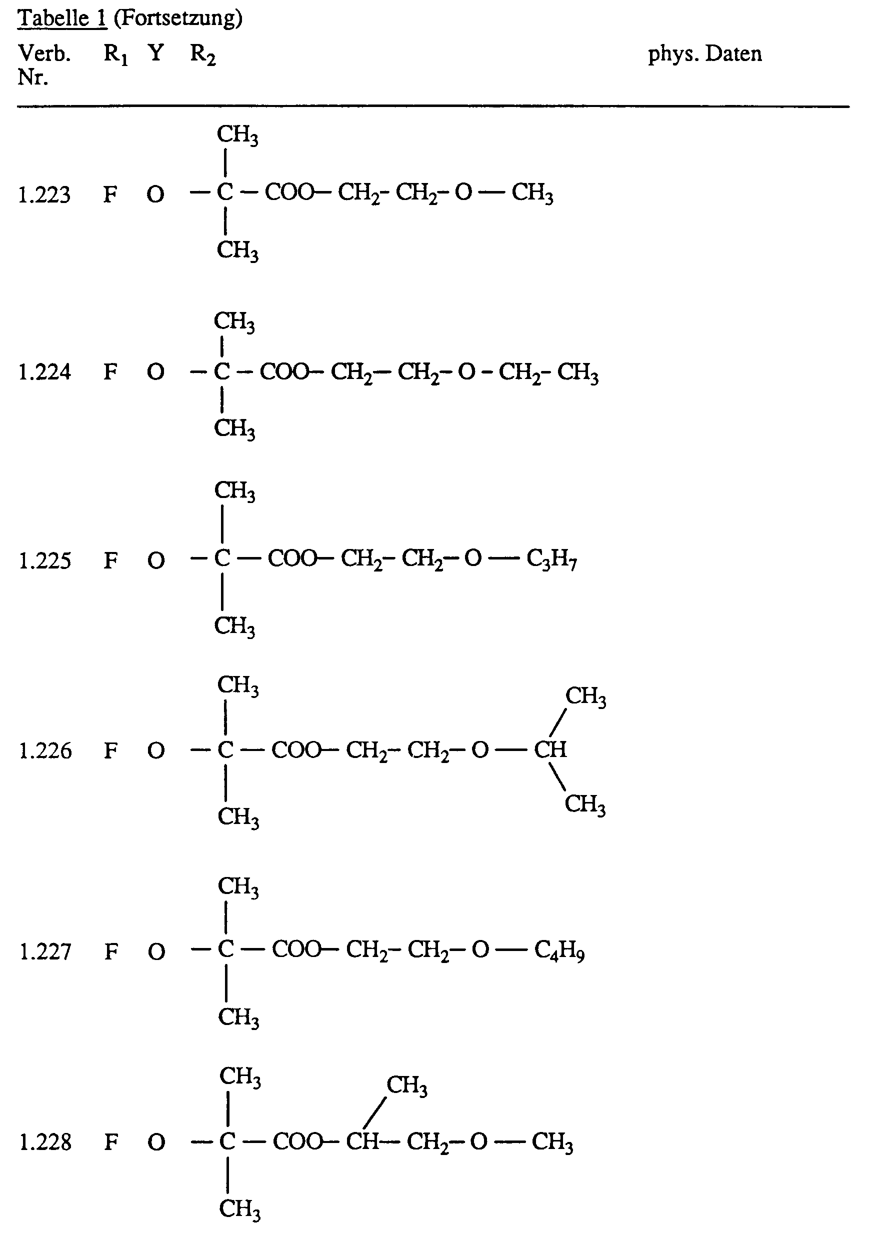

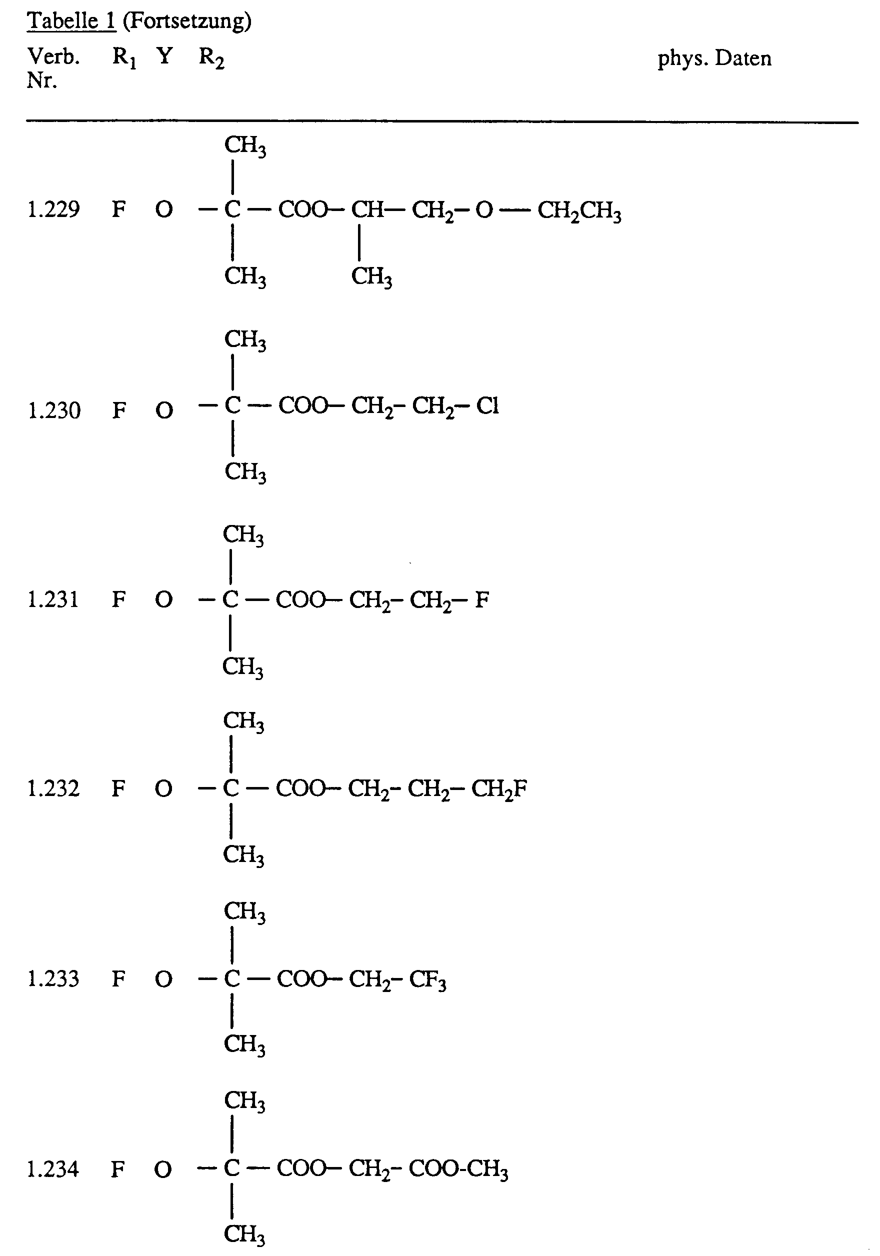

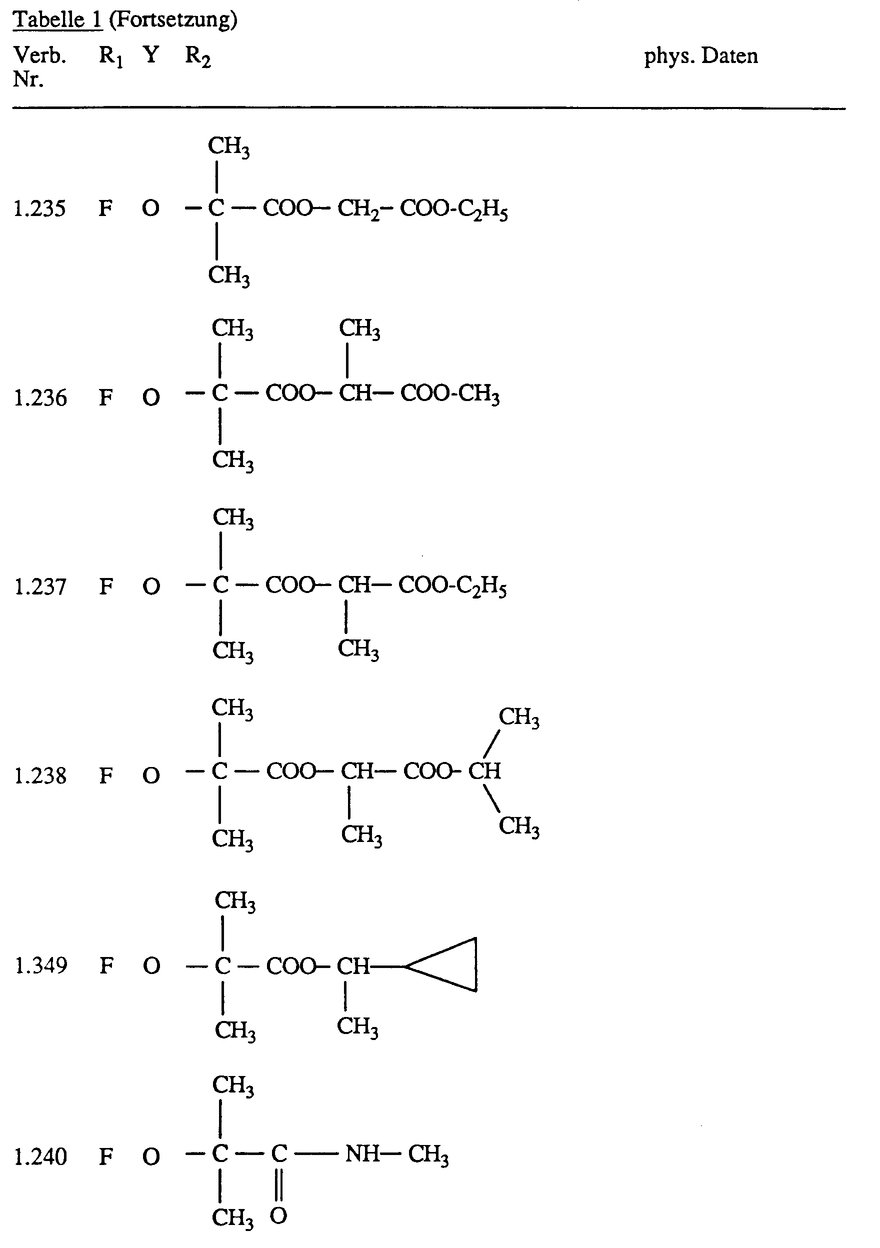

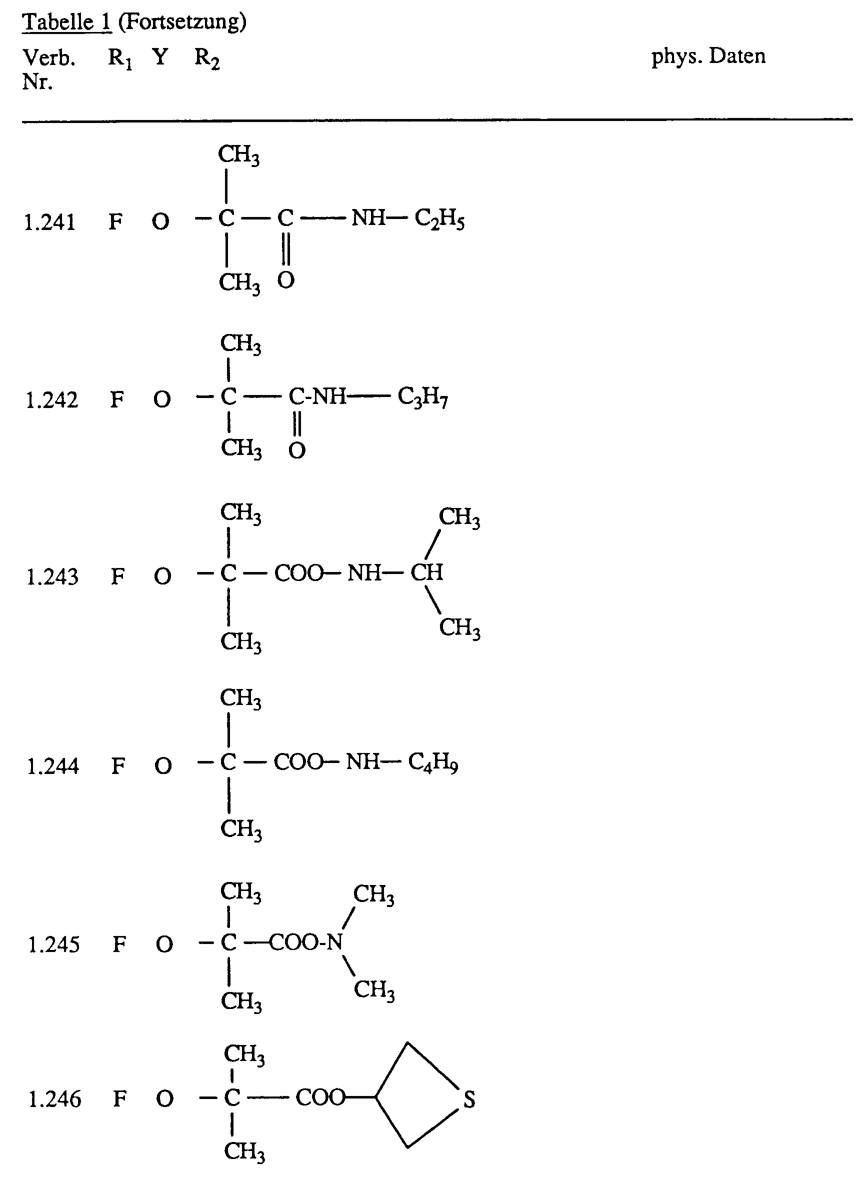

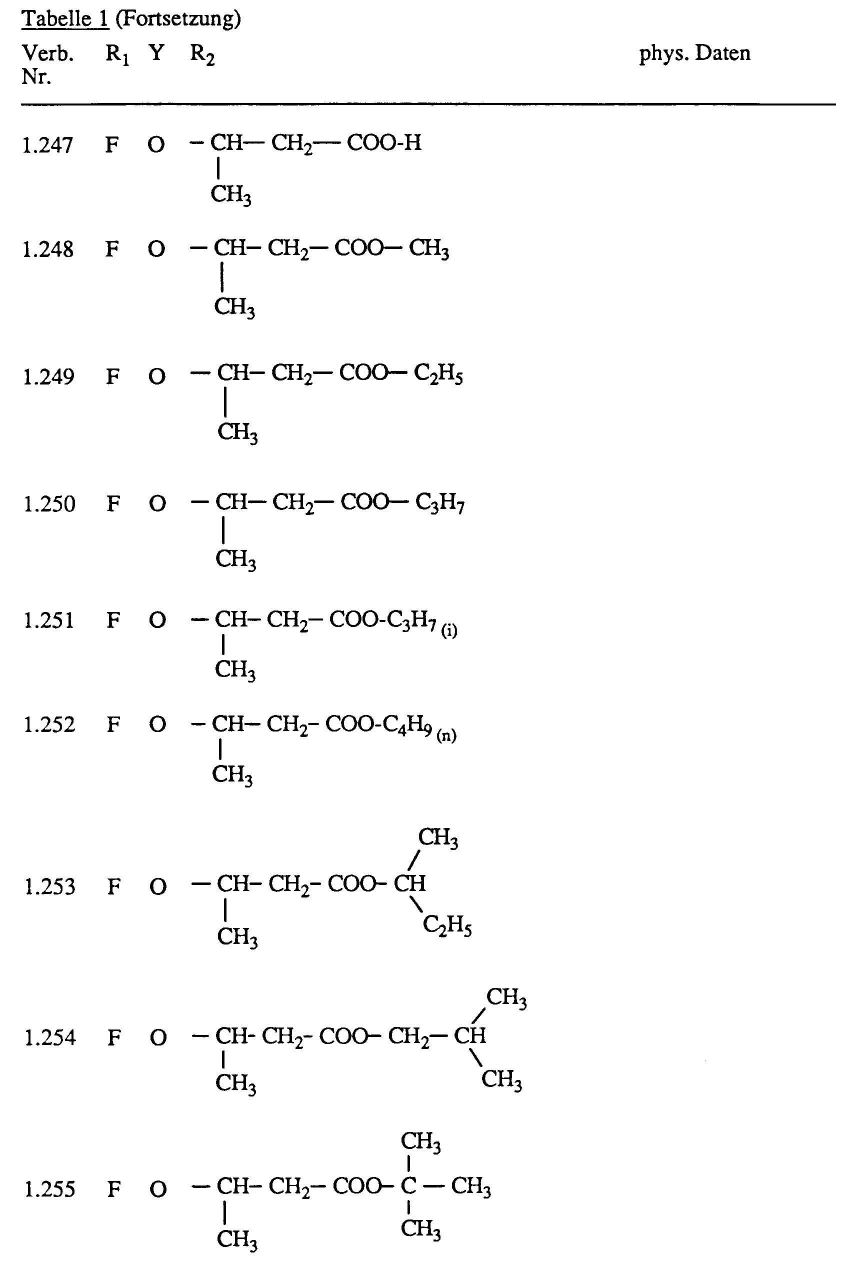

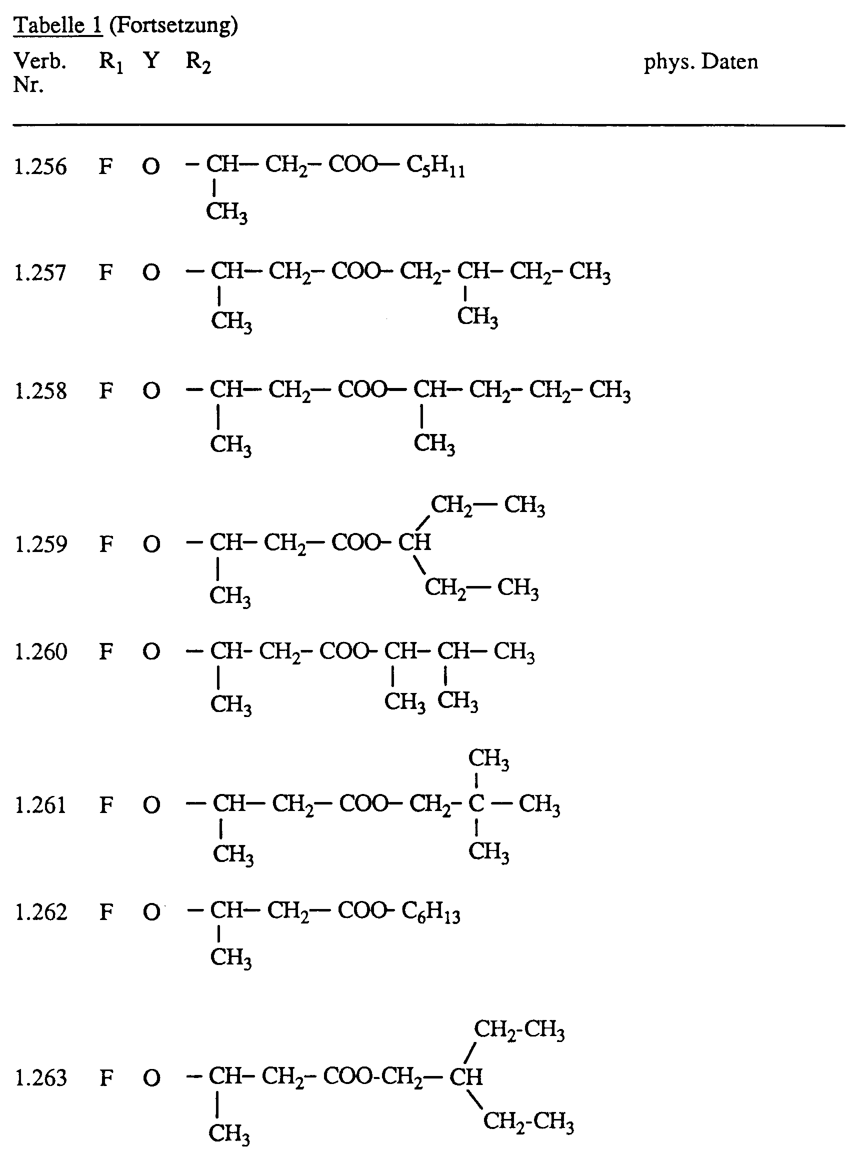

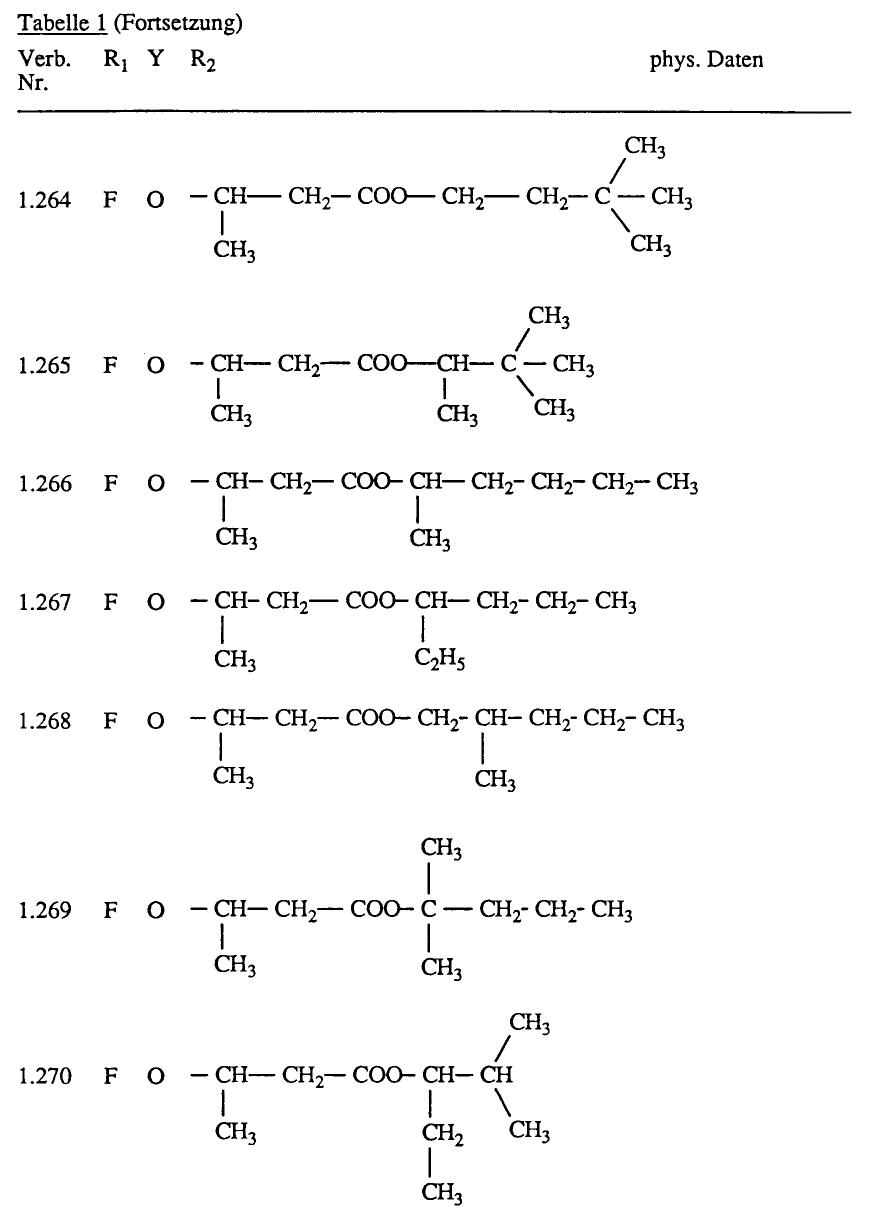

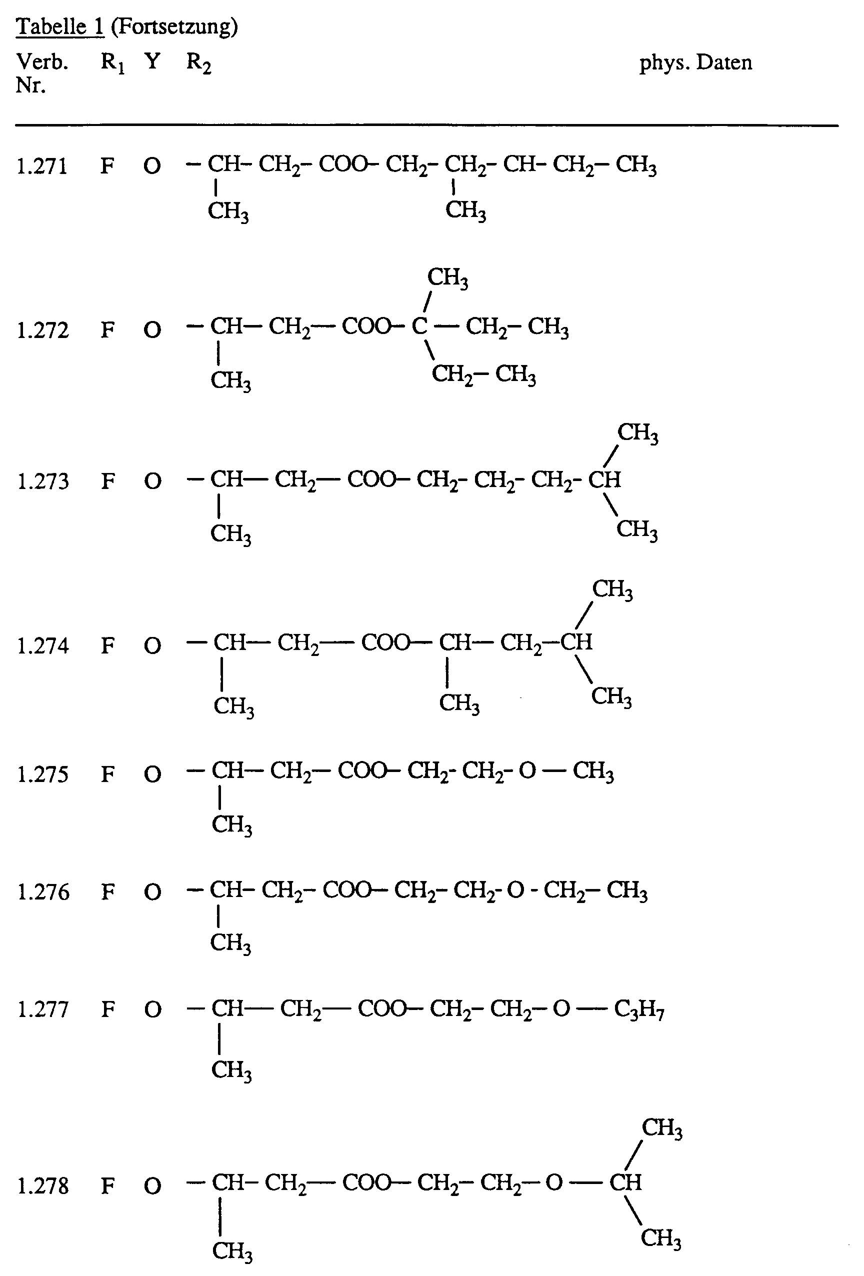

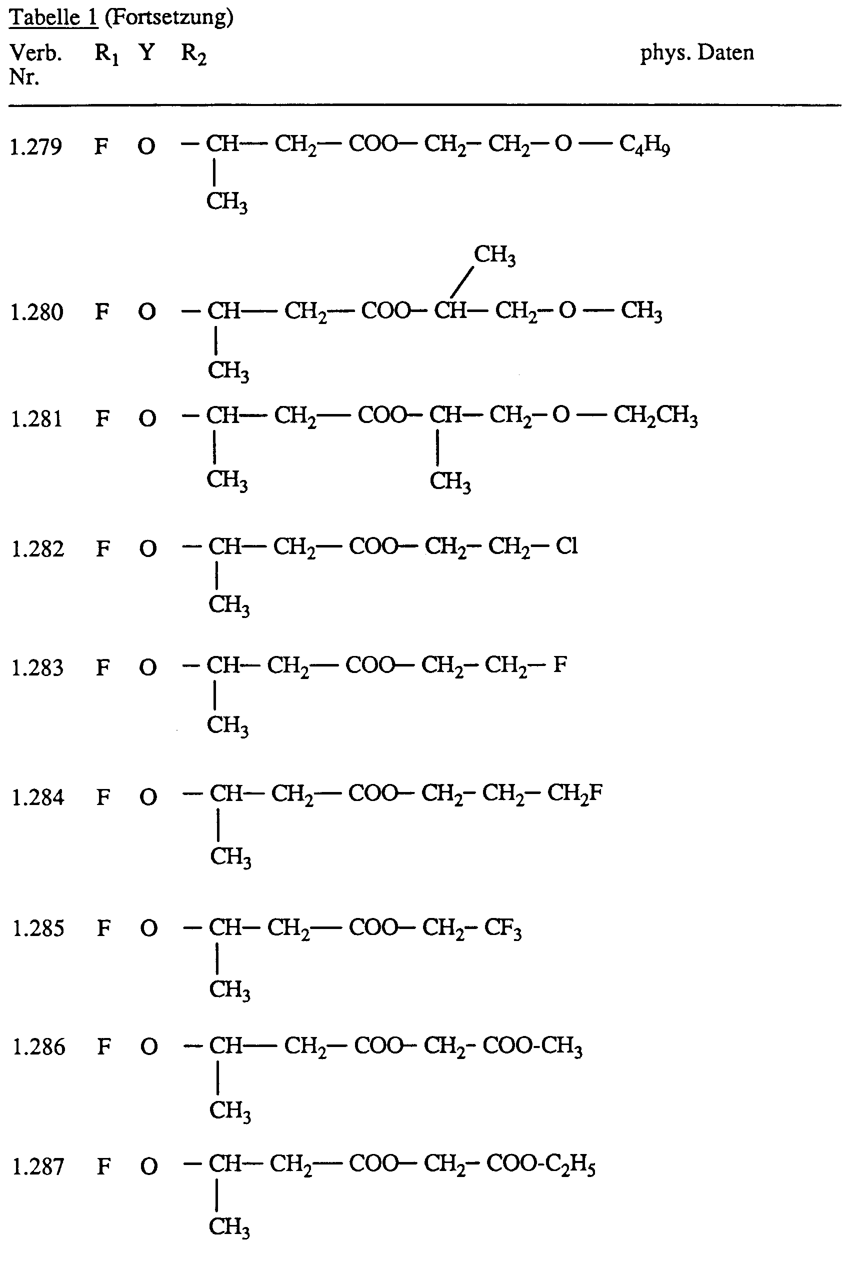

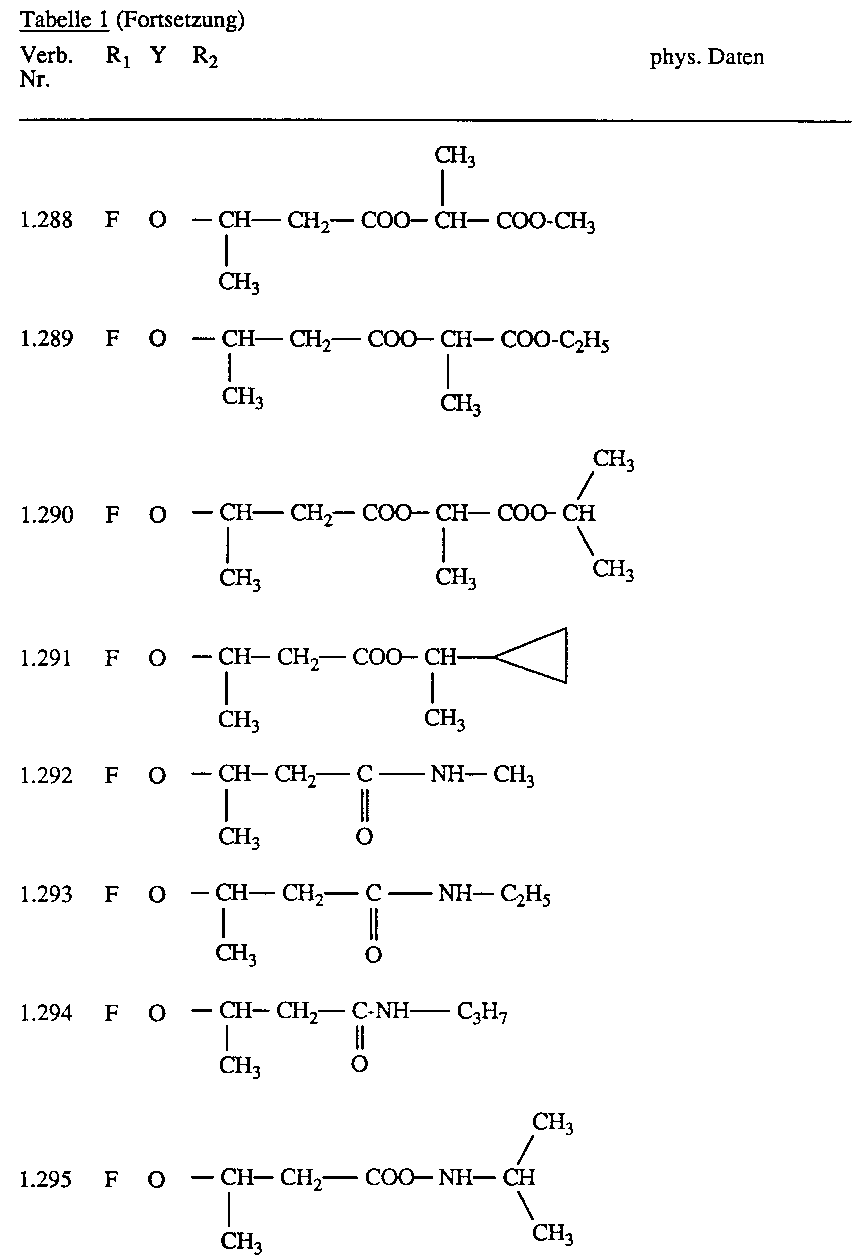

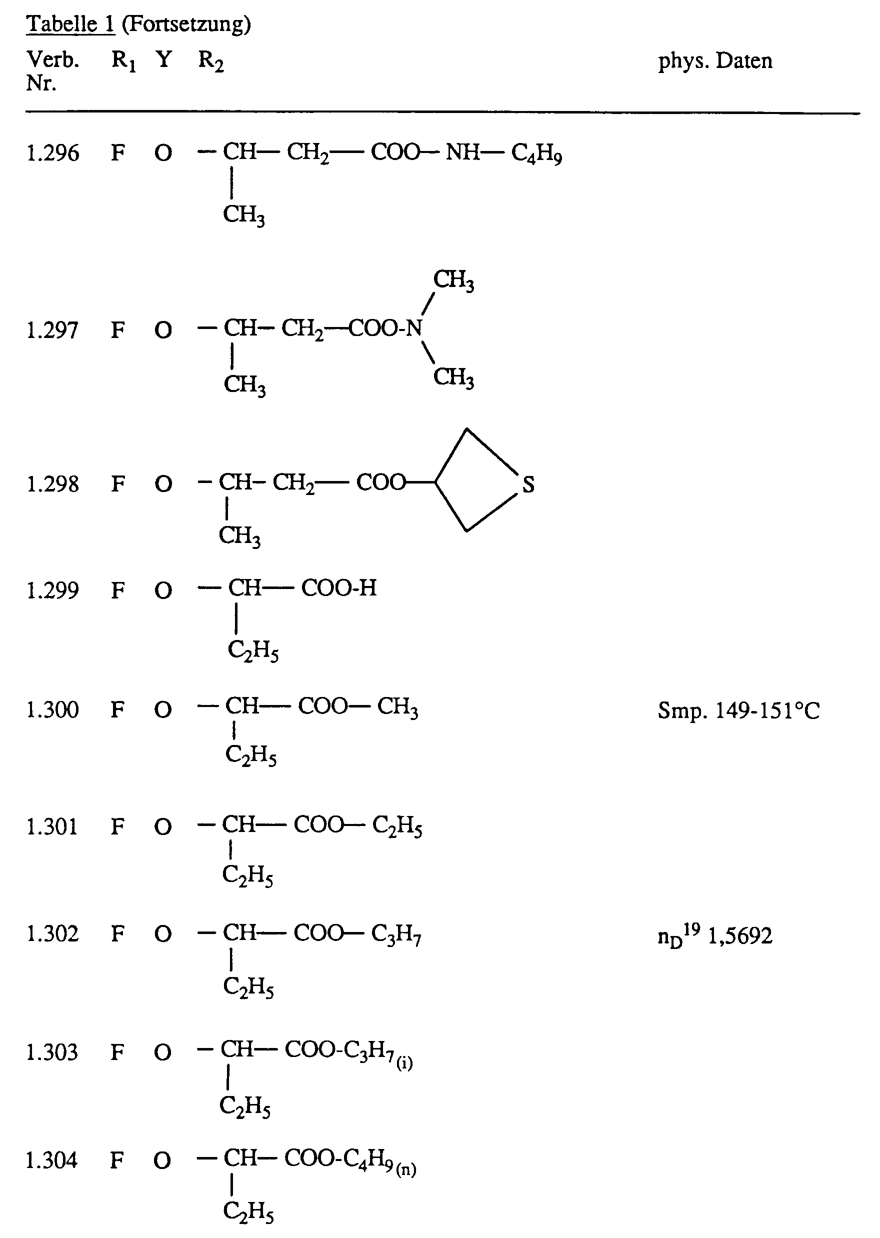

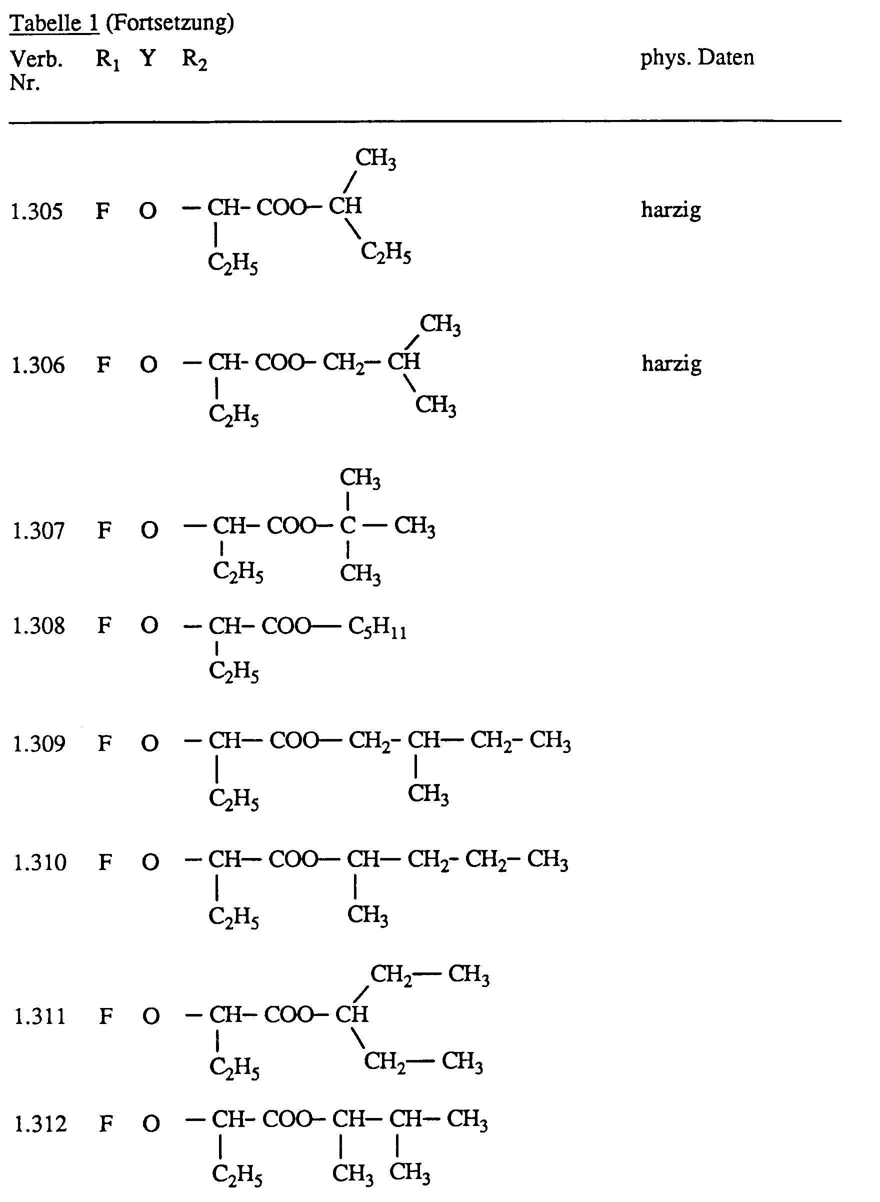

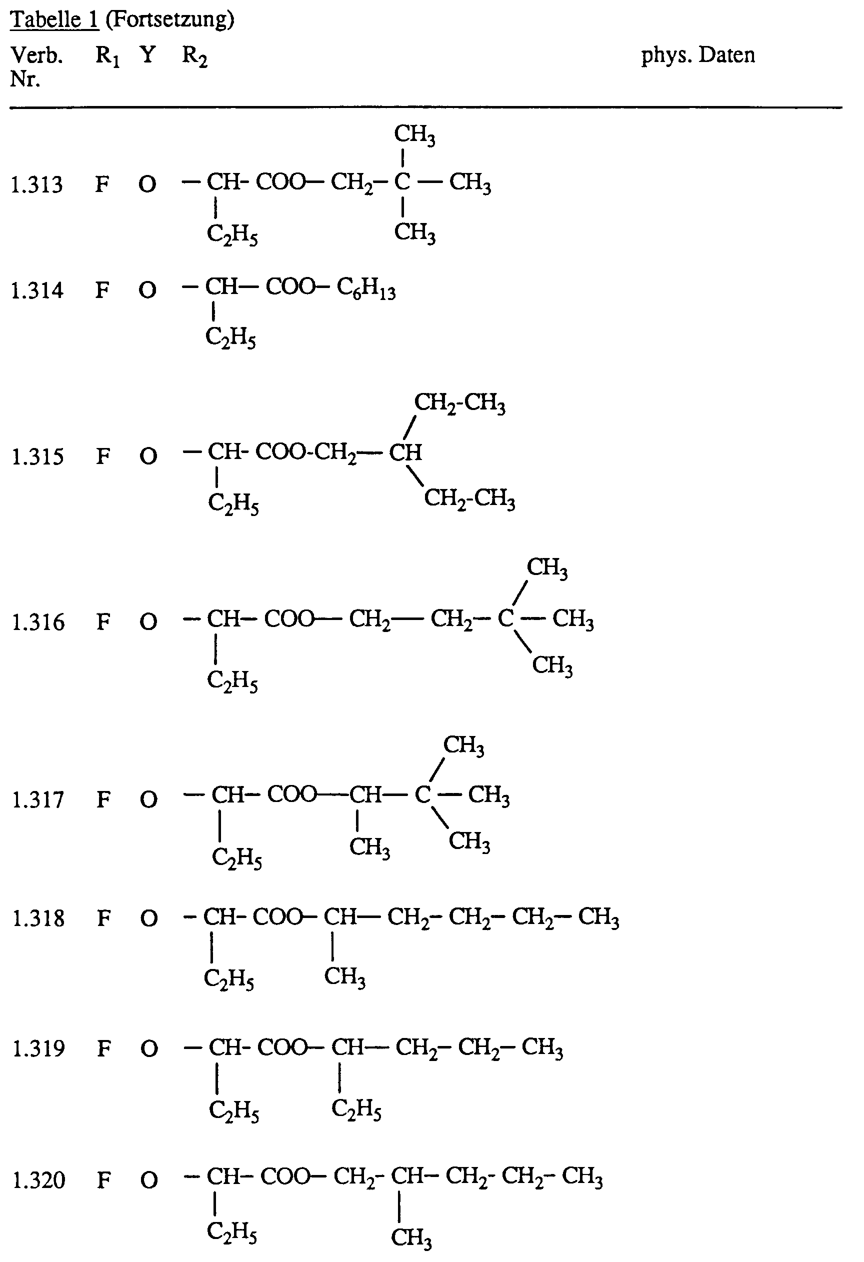

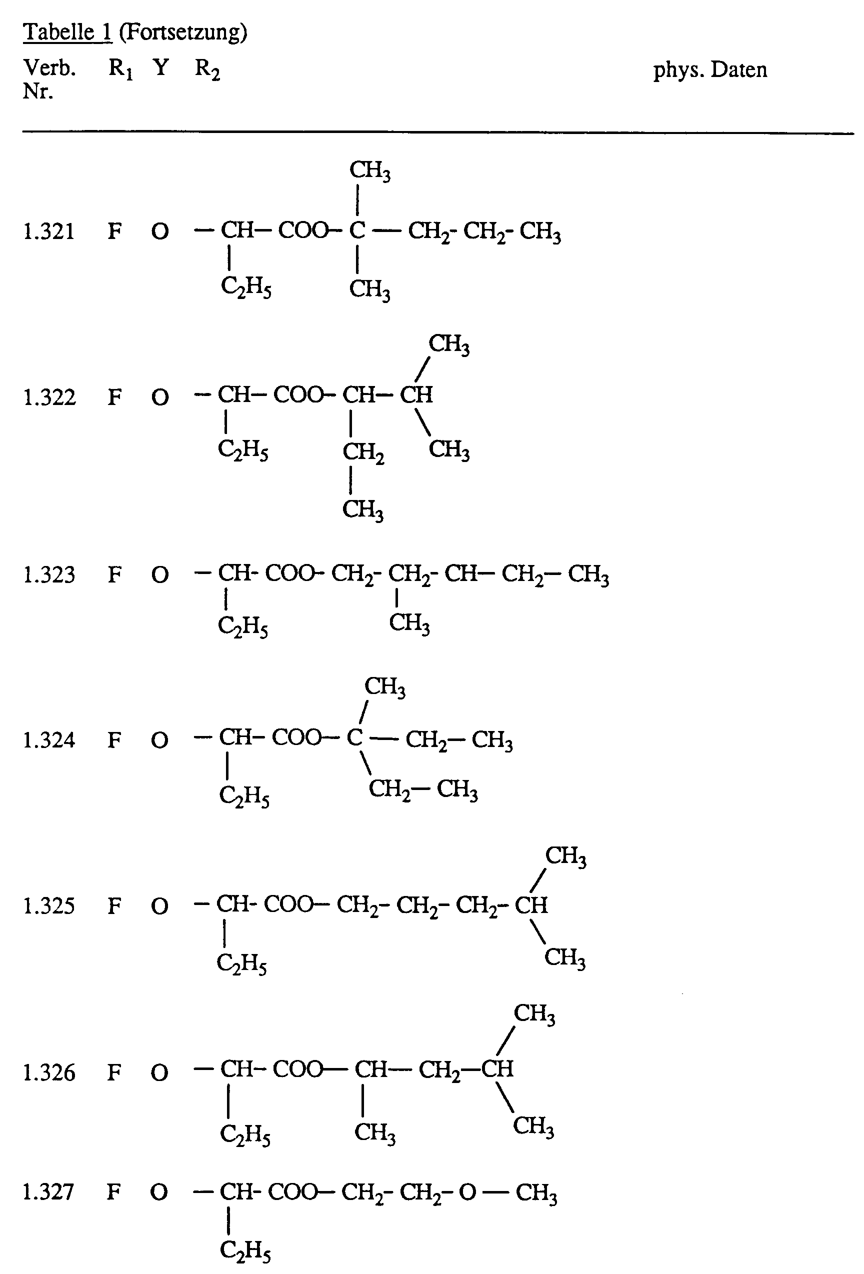

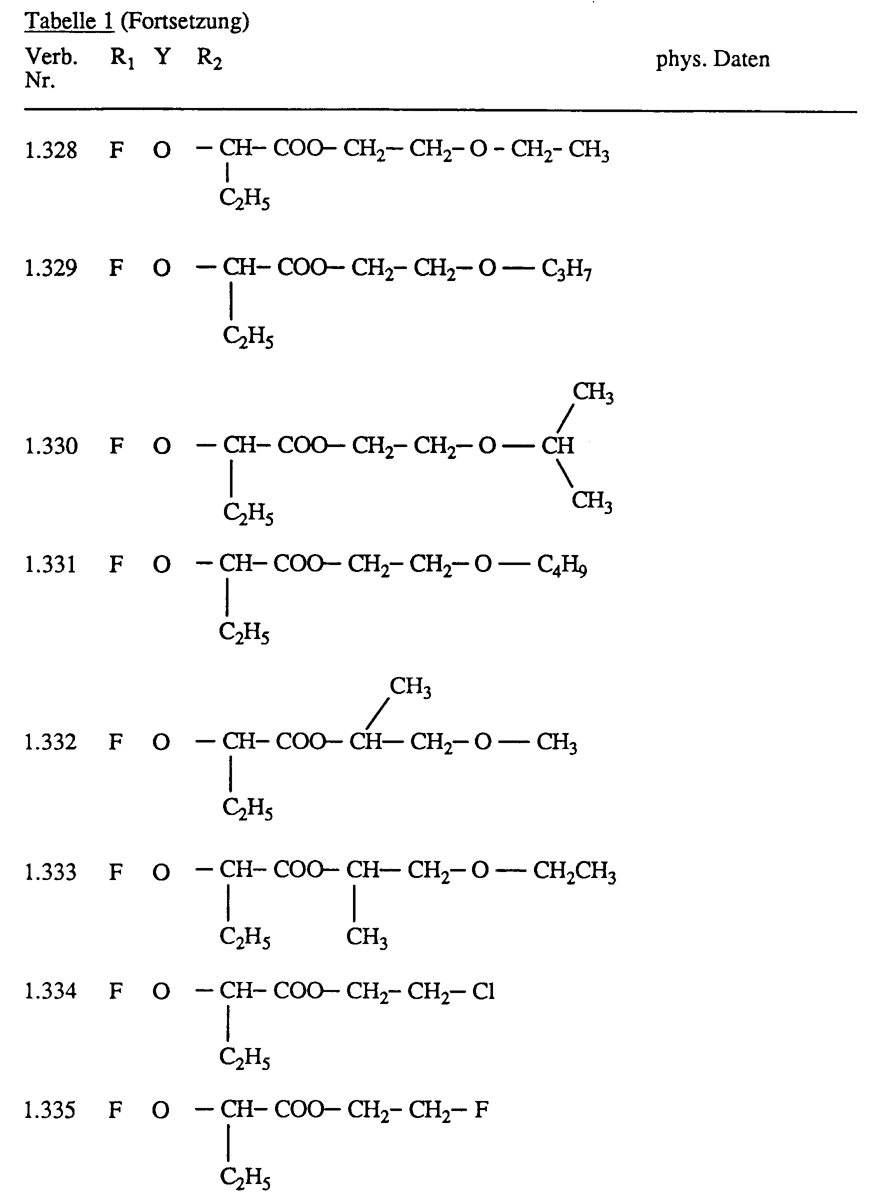

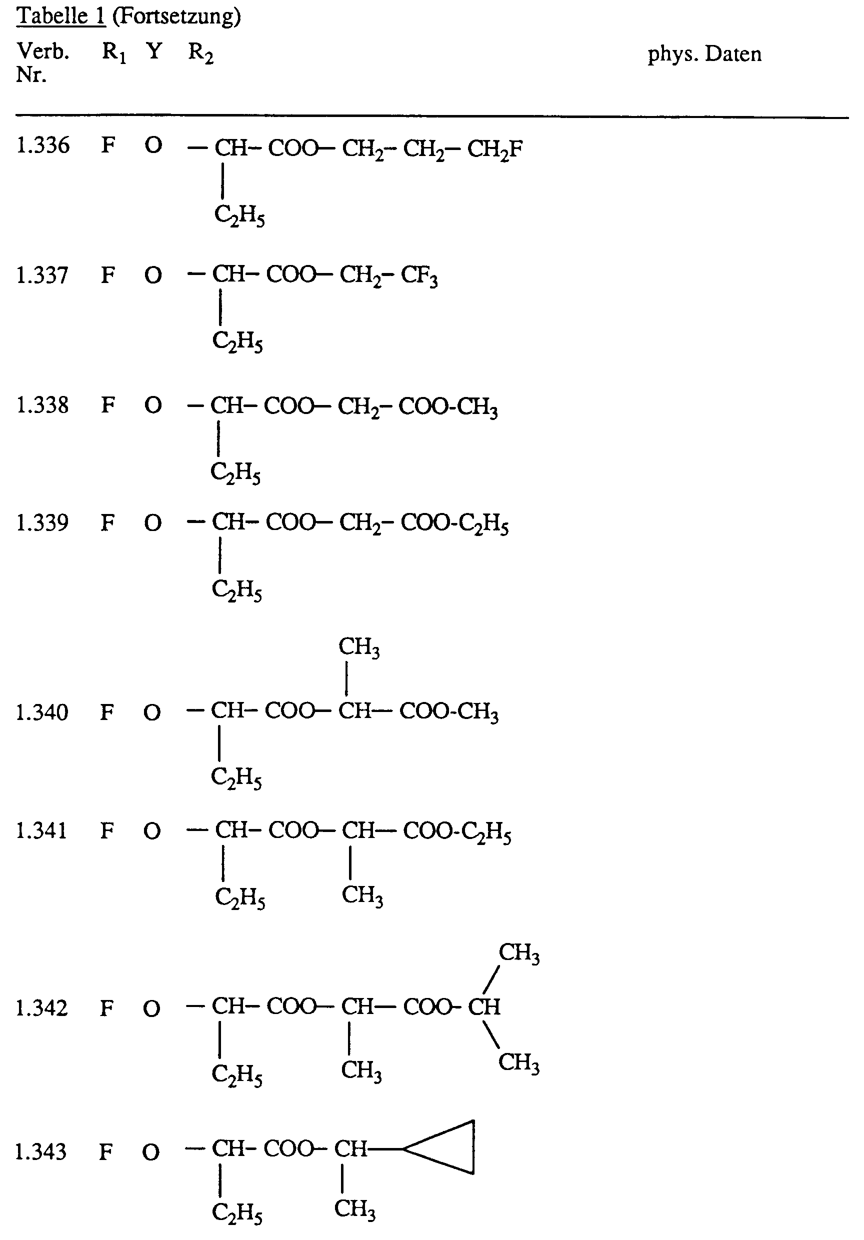

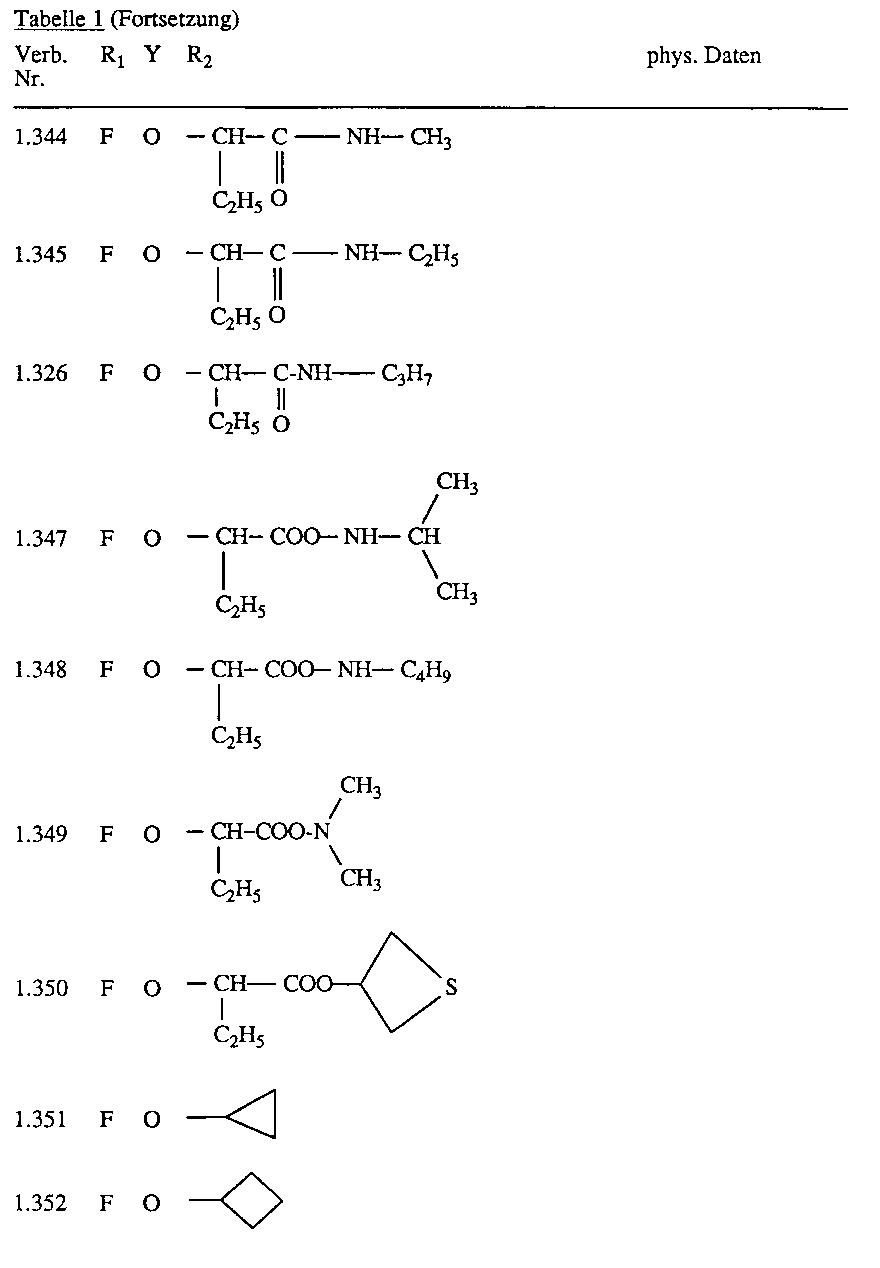

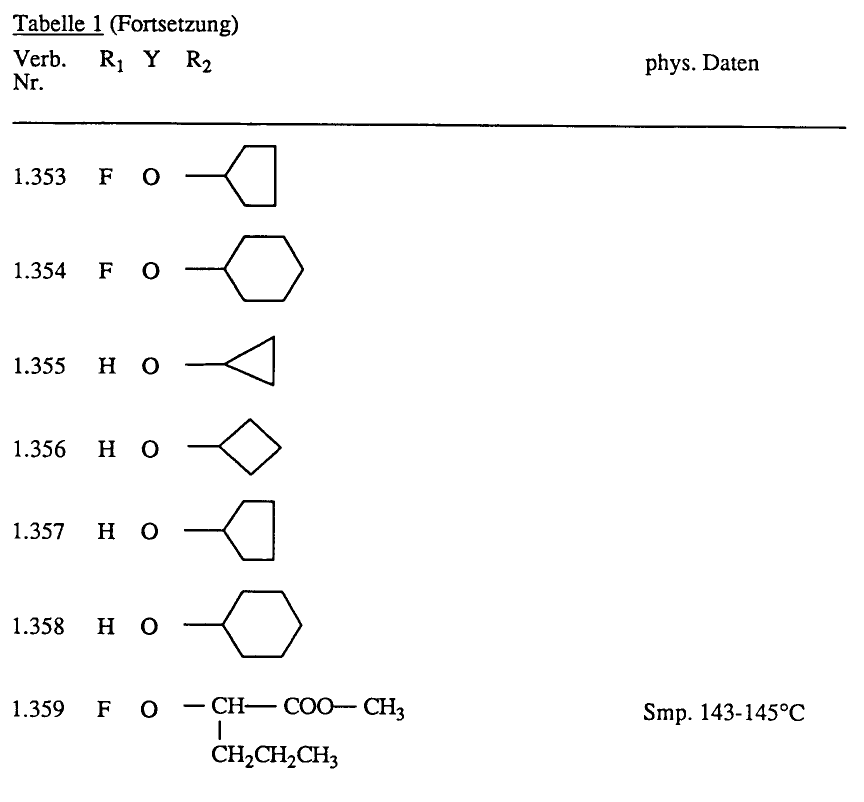

### Formulierungsbeispiele für flüssige Wirkstoffe der Formel I (% = Gewichtsprozent)

### 1. Emulsions-Konzentrate

| | a) | b) | c) |
|---|---|---|---|
| Wirkstoff gemäß Tabelle 1 | 25 % | 40 % | 50 % |
| Ca-Dodecylbenzolsulfonat | 5 % | 8 % | 6 % |
| Ricinusöl-polyethylenglykolether (36 Mol AeO) | 5 % | - | - |
| Tributylphenol-polyethylenglykolether (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

### 2. Lösungen

| | a) | b) | c) | d) |
|---|---|---|---|---|
| Wirkstoff gemäß Tabelle 1 | 80 % | 10 % | 5 % | 95 % |
| Propylenglykol-monomethylether | 20 % | - | - | - |
| Polyethylenglykol MG410 | - | 70 % | - | - |
| N-Methyl-2-pyrrolidon | - | 20 % | - | - |
| Epoxidiertes Kokosnußöl | - | - | 1 % | 5 % |
| Benzin (Siedegrenzen 160-190°C) | - | - | 94 % | - |

Die Lösungen sind zur Anwendung in Form kleinster Tropfen geeignet.

### 3. Granulate

| | a) | b) | c) | d) |
|---|---|---|---|---|
| Wirkstoff gemäß Tabelle 1 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Methylenchlorid gelöst, auf den Träger aufgesprüht und das Lösungsmittel anschließend im Vakuum abgedampft.

### 4. Stäubemittel

| | a) | b) |
|---|---|---|
| Wirkstoff gemäß Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

### Formulierungsbeispiele für feste Wirkstoffe der Formel I (% = Gewichtsprozent)

### 5. Spritzpulver

| | a) | b) | c) |
|---|---|---|---|
| Wirkstoff gemäß Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

### 6. Emulsions-Konzentrat

| | |
|---|---|
| Wirkstoff gemäß Tabelle 1 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

### 7. Stäubemittel

| | a) | b) |
|---|---|---|
| Wirkstoff gemaß Tabelle 1 | 5 % | 8 % |
| Talkum | 95 % | - |
| Kaolin | - | 92 % |

Man erhält anwendungsfertige Stäubemittel, indem der Wirkstoff mit den Trägern vermischt und auf einer geeigneten Mühle vermahlen wird.

### 8. Extruder-Granulat

| | |
|---|---|
| Wirkstoff gemäß Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschließend im Luftstrom getrocknet.

### 9. Umhüllungs-Granulat

| | |
|---|---|
| Wirkstoff gemäß Tabelle 1 | 3 % |
| Polyäthylenglykol (MG200) | 3 % |
| Kaolin | 94 % |

Der fein gemahlene Wirkstoff wird in einem Mischer auf das mit Polyethylenglykol angefeuchtete Kaolin gleichmäßig aufgetragen. Auf diese Weise erhält man staubfreie Umhüllungs-Granulate.

### 10. Suspensions-Konzentrat

| | |
|---|---|
| Wirkstoff gemäß Tabelle 1 | 40 % |
| Propylenglykol | 10 % |
| Nonylphenolpolyethylenglykolether (15 Mol AeO) | 6 % |
| Na-Ligninsulfonat | 10 % |
| Carboxymethylcellulose | 1 % |
| Silikonöl in Form einer 75%-igen wäßrigen Emulsion | 1 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen oder Gießen werden gleich wie die Art der Mittel den angestrebten Zielen und den gegebenen Verhältnissen entsprechend gewählt.

### Biologische Beispiele

### Beispiel B1: Pre-emergente Herbizid-Wirkung

Im Gewächshaus wird unmittelbar nach der Einsaat der Versuchspflanzen in Saatschalen die Erdoberfläche mit einer wäßrigen Spritzbrühe entsprechend einer Aufwandmenge von 4 kg Wirksubstanz/Hektar behandelt. Die Saatschalen werden im Gewächshaus bei 22 - 25°C und 50 - 70 % relativer Luftfeuchtigkeit gehalten.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Tabelle 1 starke Herbizidwirkung. Beispielsweise zeigt die Verbindung 1.001 gegen die getesteten Unkräuter folgende herbizide Wirkung:

**Tabelle B1**

| Preemergente Herbizid-Wirkung: | | | | |
|---|---|---|---|---|
| Testpflanze: | Avena | Sinapis | Setaria | Stellaria |
| Wirkstoff Nr. | | | | |
| 1.001 | 1 | 1 | 1 | 1 |

### Beispiel B2: Pre-emergente Herbizid-Wirkung

Im Gewächshaus wird unmittelbar nach der Einsaat der Versuchspflanzen in Saatschalen die Erdoberfläche mit einer wäßrigen Spritzbrühe entsprechend einer Aufwandmenge von 2 kg Wirksubstanz/Hektar behandelt. Die Saatschalen werden im Gewächshaus bei 22 - 25°C und 50 - 70 % relativer Luftfeuchtigkeit gehalten.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Tabelle 1 starke Herbizidwirkung. Beispiele für die gute herbizide Wirkung sind in Tabelle B2 gegeben:

**Tabelle B2**

| Preemergente Herbizid-Wirkung: | | | | |
|---|---|---|---|---|
| Testpflanze: | Avena | Sinapis | Setaria | Stellaria |
| Wirkstoff Nr. | | | | |
| 1.008 | 1 | 3 | 1 | 1 |
| 1.034 | 1 | 1 | 1 | 1 |
| 1.036 | 1 | 1 | 1 | 1 |
| 1.092 | 1 | 1 | 1 | 1 |
| 1.093 | 2 | 1 | 1 | 1 |
| 1.146 | 3 | 1 | 1 | 1 |
| 1.301 | 2 | 2 | 1 | 2 |
| 1.303 | 3 | 1 | 1 | 1 |

### Beispiel B3: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wäßrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage nach der Behandlung wird die Herbizidwirkung mit einem neunstufigen (1 = vollständige Schädigung, 9 = keine Wirkung) Boniturschema im Vergleich mit einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Tabelle 1 starke Herbizidwirkung. Beispielsweise zeigt die Verbindung 1.001 gegen die getesteten Unkräuter folgende herbizide Wirkung:

**Tabelle B3**

| Post-emergente Herbizid-Wirkung: | | | | | | |
|---|---|---|---|---|---|---|
| Testpflanze: | Avena | Setaria | Lolium | Solanum | Sinapis | Stellaria |
| Wirkstoff Nr. | | | | | | |
| 1.001 | 1 | 2 | 1 | 1 | 1 | 1 |

### Beispiel B4: Post-emergente Herbizid-Wirkung (Kontaktherbizid)

Eine Anzahl Unkräuter, sowohl monokotyle wie dikotyle, wurden nach dem Auflaufen (im 4- bis 6-Blattstadium) mit einer wäßrigen Wirkstoffdispersion in einer Dosierung von 2 kg Wirksubstanz pro Hektar auf die Pflanzen gespritzt und diese bei 24°-26°C und 45-60 % relativer Luftfeuchtigkeit gehalten. 15 Tage nach der Behandlung wird die Herbizidwirkung mit einem neunstufigen (1 = vollständige Schädigung, 9 = keine Wirkung) Boniturschema im Vergleich mit einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Tabelle 1 starke Herbizidwirkung. Beispiele für die gute herbizide Wirkung sind in Tabelle B4 gegeben:

**Tabelle B4**

| Post-emergente Herbizid-Wirkung: | | | | |
|---|---|---|---|---|
| Testpflanze: | Avena | Sinapis | Setaria | Stellaria |
| Wirkstoff Nr. | | | | |
| 1.008 | 2 | 4 | 1 | 1 |
| 1.034 | 1 | 2 | 1 | 1 |
| 1.036 | 1 | 1 | 1 | 1 |
| 1.092 | 1 | 2 | 1 | 1 |
| 1.093 | 1 | 2 | 1 | 1 |
| 1.146 | 2 | 1 | 1 | 1 |
| 1.301 | 2 | 2 | 1 | 1 |
| 1.303 | 3 | 1 | 1 | 2 |

### Beispiel B5: Herbizidwirkung für Wasserreis (paddy)

Die Wasserunkräuter Echinochloa crus galli und Monocharia vag. werden in Plastikbechern (60 cm² Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat durch eine
Spritzung der Prüfsubstanzen auf die Gefäße. Die verwendete Dosis entspricht einer Wirkstoffmenge von 4 kg AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt. Die Verbindungen der Tabelle 1 schädigen dabei die Unkräuter.

## Patentansprüche (Patentansprüche für folgende(n) Vertragsstaat(en): AT, BE, CH, DE, DK, FR, GB, GR, IE, IT, LI, LU, MC, NL, PT, SE)

1. Benzothiazolonderivate der Formel I 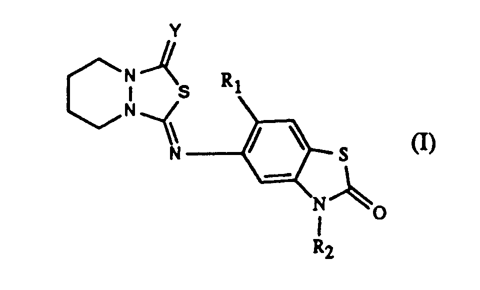 worin
Y Sauerstoff oder Schwefel;
R₁ Wasserstoff oder Fluor und
R₂ Wasserstoff, C₁-C₆-Alkyl, C₂-C₄-Alkenyl, oder C₂-C₆-Alkinyl; durch Halogen substituiertes C₁-C₆-Alkyl, C₂-C₄-Alkyenyl, oder C₃-C₆-Alkinyl; C₁-C₄-Alkoxy-C₁-C₄-alkyl, C₁-C₄-Alkoxy-C₁-C₂-alkoxy-C₁-C₂-alkyl, 1-Phenylpropen-3-yl, durch Cyano oder C₃-C₆-Cycloalkyl substituiertes C₁-C₆-Alkyl; Carboxy-C₁-C₄-alkyl, C₁-C₆-Alkoxycarbonyl-C₁-C₄-alkyl, Halogen-C₁-C₆-alkoxycarbonyl-C₁-C₄-alkyl, C₁-C₄-Alkoxy-C₁-C₂-alkoxycarbonyl-C₁-C₄-alkyl, C₁-C₆-Alkoxycarbonyl-C₁-C₂-alkoxycarbonyl-C₁-C₄-alkyl, C₃-C₆-Cycloalkyl-C₁-C₂-alkoxycarbonyl-C₁-C₄-alkyl, C₁-C₅-Alkylaminocarbonyl-C₁-C₄-alkyl, Di-C₁-C₅-alkylaminocarbonyl-C₁-C₄-alkyl, C₃-C₆-Cycloalkyl, C₁-C₄-Alkylthio-C₁-C₄-alkyl, Benzyl, oder durch Halogen substituiertes Benzyl, C₁-C₄-Alkylsulfonyl, C₃-C₆-Alkenyloxy-C₁-C₄-alkyl, C₁-C₈-Alkylcarbonyl, 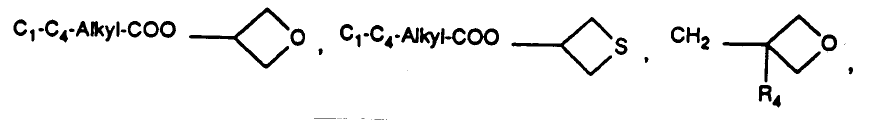 C₁-C₄-Alkylthiocarbonyl-C₁-C₄-alkyl, oder die Gruppe 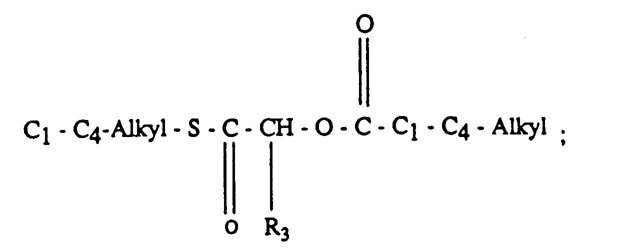
R₃ Wasserstoff oder C₁-C₄-Alkyl; und
R₄ Wasserstoff oder C₁-C₄-Alkyl bedeuten.

2. Verbindungen nach Anspruch 1, dadurch gekennzeichnet, daß
R₁ für Fluor steht.

3. Verbindungen nach Anspruch 1, dadurch gekennzeichnet, daß
R₁ für Fluor und Y für Sauerstoff steht.

4. Verbindungen nach Anspruch 1, dadurch gekennzeichnet daß
R₂ Wasserstoff, C₁-C₄-Alkyl, C₂-C₄-Alkenyl, oder C₂-C₄-Alkinyl; durch Halogen substituiertes C₁-C₄-Alkyl, C₂-C₄-Alkenyl, oder C₃-C₄-Alkinyl; C₁-C₄-Alkoxy-C₁-C₄-alkyl, C₁-C₄-Alkoxy-C₁-C₂-alkoxy-C₁-C₂-alkyl, 1-Phenylpropen-3-yl, durch Cyano oder C₃-C₆-Cycloalkyl substituiertes C₁-C₄-Alkyl, Benzyl, oder durch Halogen substituiertes Benzyl, C₃-C₆-Alkenyloxy-C₁-C₄-alkyl, C₁-C₈-Alkylcarbonyl, 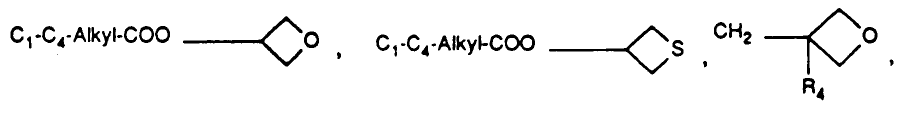 C₁-C₄-Alkylthiocarbonyl-C₁-C₄-alkyl, oder die Gruppe 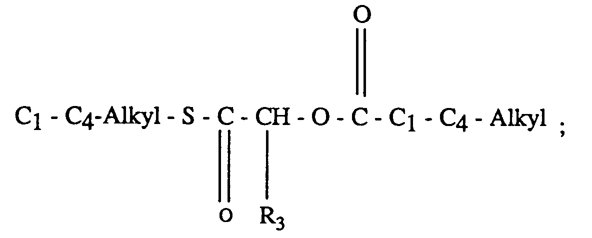 R₃ Wasserstoff oder C₁-C₄-Alkyl; und
R₄ Wasserstoff oder C₁-C₄-Alkyl bedeuten.

5. Verbindungen nach Anspruch 1, dadurch gekennzeichnet, daß
R₂ Wasserstoff, C₁-C₄-Alkyl, C₂-C₄-Alkenyl, oder C₂-C₄-Alkinyl; durch Halogen substituiertes C₁-C₄-Alkyl, C₂-C₄-Alkenyl, oder C₃-C₄-Alkinyl; oder C₁-C₂-Alkoxy-C₁-C₂-alkyl bedeuten.

6. Verbindungen nach Anspruch 1, dadurch gekennzeichnet, daß
R₂ Wasserstoff, C₁-C₄-Alkyl, C₂-C₄-Alkenyl, durch Fluor substituiertes C₁-C₄-Alkyl oder C₂-C₄-Alkenyl bedeuten.

7. Verbindungen nach Anspruch 1, dadurch gekennzeichnet, daß
R₂ C₃-C₆-Cycloalkyl, durch C₃-C₆-Cycloalkyl substituiertes C₁-C₆-Alkyl;C₁-C₄-Alkylthio-C₁-C₄-alkyl, Benzyl, oder durch Halogen substituiertes Benzyl, C₁-C₄-Alkylsulfonyl, C₃-C₆-Alkenyloxy-C₁-C₄-alkyl, C₁-C₈-Alkylcarbonyl, 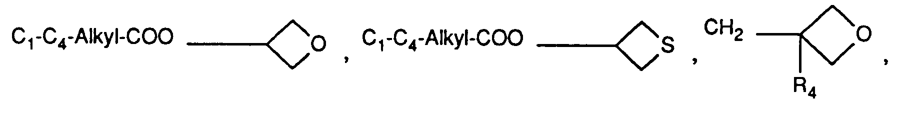 C₁-C₄-Alkylthiocarbonyl-C₁-C₄-alkyl, oder die 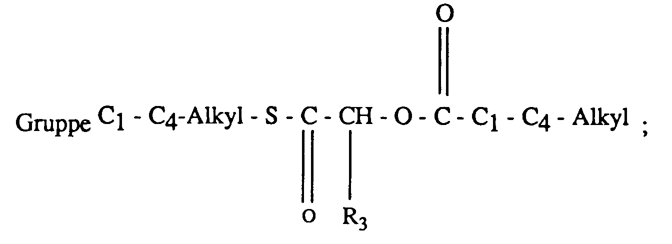 R₃ Wasserstoff oder C₁-C₄-Alkyl; und
R₄ Wasserstoff oder C₁-C₄-Alkyl bedeuten.

8. Verbindungen nach Anspruch 7, dadurch gekennzeichnet, daß
Y Sauerstoff, R₁ Fluor und R₃ Wasserstoff oder Methyl bedeuten.

9. Verbindungen nach Anspruch 1, dadurch gekennzeichnet, daß
Y für Sauerstoff, R₁ für Fluor und R₂ für C₁-C₃-Alkyl, CH₂F, CH₂CH₂F, CH₂CH₂CH₂F, CHF₂, Allyl, Propargyl, 1-Methyl-3-propinyl, Methoxycarbonylmethyl, Ethoxycarbonylmethyl, Isopropoxycarbonylmethyl, 1-(Methoxycarbonyl)-ethyl, 1-(Ethoxycarbonyl)-ethyl oder 1-(Isopropoxycarbonyl)-ethyl steht.

10. 9-[6-Fluor-3-isopropyl-2(3H)-benzothiazolon-5-yl]imino-8-thia-1,6-diazabicyclo[4.3.0]nonan-7-on gemäss Anspruch 1.

11. Verbindungen nach Anspruch 1, dadurch gekennzeichnet, daß
R₂ durch Halogen substituiertes C₁-C₆-Alkyl, C₂-C₄-Alkenyl, oder C₃-C₆-Alkinyl; C₁-C₄-Alkoxy-C₁-C₄-alkyl, C₁-C₄-Alkoxy-C₁-C₂-alkoxy-C₁-C₂-alkyl, durch Cyano oder
C₃-C₆-Cycloalkyl substituiertes C₁-C₆-Alkyl; Carboxy-C₁-C₄-alkyl, C₁-C₆-Alkoxycarbonyl-C₁-C₄-alkyl, C₁-C₄-Alkoxy-C₁-C₂-alkoxycarbonyl-C₁-C₄-alkyl, C₁-C₆-Alkoxycarbonyl-C₁-C₂-alkoxycarbonyl-C₁-C₄-alkyl, C₃-C₆-Cycloalkyl-C₁-C₂-alkoxycarbonyl-C₁-C₄-alkyl, C₁-C₅-Alkylaminocarbonyl-C₁-C₄-alkyl, Di-C₁-C₅-alkylaminocarbonyl-C₁-C₄-alkyl, C₃-C₆-Cycloalkyl, C₁-C₄-Alkylthio-C₁-C₄-alkyl, C₃-C₆-Alkenyloxy-C₁-C₄-alkyl, 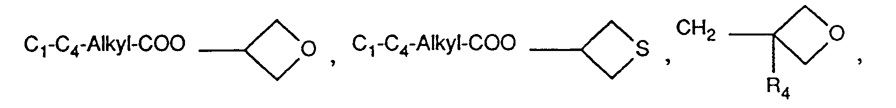 C₁-C₄-Alkylthiocarbonyl-C₁-C₄-alkyl, oder die Gruppe 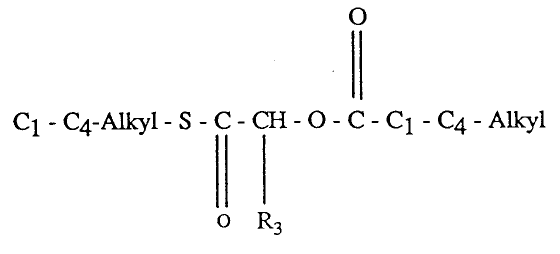 bedeutet.

12. Verbindungen nach Anspruch 11, dadurch gekennzeichnet, daß
R₂ C₁-C₄-Alkoxy-C₁-C₄-alkyl, C₁-C₄-Alkoxy-C₁-C₂-alkoxy-C₁-C₂-alkyl, durch Cyano substituiertes C₁-C₆-Alkyl; Carboxy-C₁-C₄-alkyl, C₁-C₆-Alkoxycarbonyl-C₁-C₄-alkyl, C₁-C₄-Alkoxy-C₁-C₂-alkoxycarbonyl-C₁-C₄-alkyl, C₁-C₆-Alkoxycarbonyl-C₁-C₂-alkoxycarbonyl-C₁-C₄-alkyl, C₃-C₆-Cycloalkyl-C₁-C₂-alkoxycarbonyl-C₁-C₄-alkyl, C₁-C₅-Alkylaminocarbonyl-C₁-C₄-alkyl, Di-C₁-C₅-alkylaminocarbonyl-C₁-C₄-alkyl oder C₃-C₆-Cycloalkyl bedeutet.

13. Verbindungen nach Anspruch 12, dadurch gekennzeichnet, daß
R₂ C₁-C₆-Alkoxycarbonyl-C₁-C₄-alkyl oder C₁-C₄-Alkoxy-C₁-C₂-alkoxycarbonyl-C₁-C₄-alkyl, bedeutet.

14. Verbindungen nach Anspruch 11, dadurch gekennzeichnet, daß R₁ für Fluor steht und Y Sauerstoff bedeutet.

15. Verfahren zur Herstellung der Verbindungen der Formel I 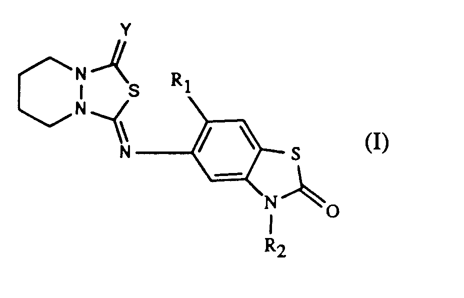 worin
Y Sauerstoff oder Schwefel;
R₁ Wasserstoff oder Fluor und
R₂ Wasserstoff, C₁-C₆-Alkyl, C₂-C₄-Alkenyl, oder C₂-C₆-Alkinyl; durch Halogen substituiertes C₁-C₆-Alkyl, C₂-C₄-Alkenyl, oder C₃-C₆-Alkinyl; C₁-C₄-Alkoxy-C₁-C₄-alkyl, C₁-C₄-Alkoxy-C₁-C₂-alkoxy-C₁-C₂-alkyl, 1-Phenylpropen-3-yl, durch Cyano oder C₃-C₆-Cycloalkyl substituiertes C₁-C₆-Alkyl; Carboxy-C₁-C₄-alkyl, C₁-C₆-Alkoxycarbonyl-C₁-C₄-alkyl, Halogen-C₁-C₆-alkoxycarbonyl-C₁-C₄-alkyl, C₁-C₄-Alkoxy-C₁-C₂-alkoxycarbonyl-C₁-C₄-alkyl, C₁-C₆-Alkoxycarbonyl-C₁-C₂-alkoxycarbonyl-C₁-C₄-alkyl, C₃-C₆-Cycloalkyl-C₁-C₂-alkoxycarbonyl-C₁-C₄-alkyl, C₁-C₅-Alkylaminocarbonyl-C₁-C₄-alkyl, Di-C₁-C₅-alkylaminocarbonyl-C₁-C₄-alkyl, C₃-C₆-Cycloalkyl, C₁-C₄-Alkylthio-C₁-C₄-alkyl, Benzyl, oder durch Halogen substituiertes Benzyl, C₁-C₄-Alkylsulfonyl, C₃-C₆-Alkenyloxy-C₁-C₄-alkyl, C₁-C₈-Alkylcarbonyl, 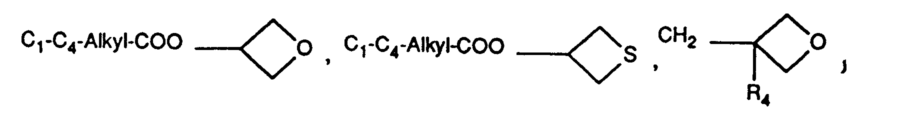 C₁-C₄-Alkylthiocarbonyl-C₁-C₄-alkyl, oder die Gruppe 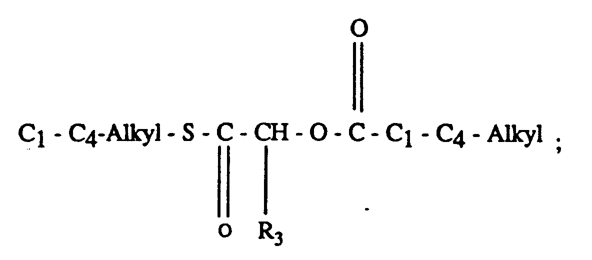
R₃ Wasserstoff oder C₁-C₄-Alkyl; und
R₄ Wasserstoff oder C₁-C₄-Alkyl bedeuten, dadurch gekennzeichnet, daß man ein Isothiocyanat der Formel II 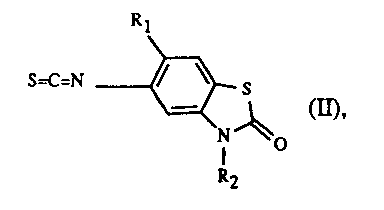 worin die Substituenten R₁ und R₂ die unter Formel I angegebene Bedeutung haben, mit einem Hexahydropyridazin der Formel III 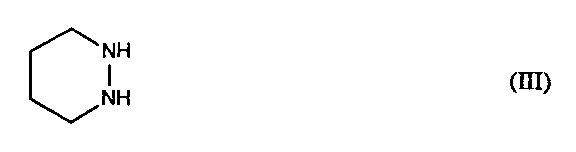 in die Verbindung der Formel IV 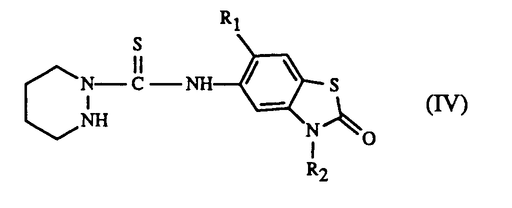 überführt und diese anschließend mit einer Verbindung der Formel V
CYCl₂ (V),
worin Y für Sauerstoff oder Schwefel steht, in Gegenwart einer Base umsetzt.

16. Ein herbizides und den Pflanzenwuchs hemmendes Mittel, gekennzeichnet durch ein Gehalt an einem oder mehreren Benzothiazolonderivaten der Formel I, gemäß Anspruch 1.

17. Mittel gemäß Anspruch 16, dadurch gekennzeichnet, daß es zwischen 0,1 % und 95 % Wirkstoff der Formel I gemäß Anspruch 1 enthält.

18. Verfahren zur Bekämpfung unerwünschten pflanzenwachstums, dadurch gekennzeichnet, daß man einen Wirkstoff der Formel I, gemäß Anspruch 1, oder ein diesen Wirkstoff enthaltendes Mittel gemäß Anspruch 16 in einer wirksamen Menge auf die Pflanzen oder deren Lebensraum appliziert.

19. Verfahren gemäß Anspruch 18, dadurch gekennzeichnet, daß man eine Wirkstoffmenge zwischen 0,001 und 4 kg pro Hektar appliziert.

20. Verfahren gemäß Anspruch 18, zur selektiven pre- oder post-emergenten Bekämfpung von Unkräutern in Nutzpflanzenkulturen.

21. Verfahren zur Hemmung des Pflanzenwachstums, dadurch gekennzeichnet, daß man einen Wirkstoff der Formel I, gemäß Anspruch 1, oder ein diesen Wirkstoff enthaltendes Mittel gemäß Anspruch 16 in einer wirksamen Menge auf die Pflanzen oder deren Lebensraum appliziert.

## Patentansprüche (Patentansprüche für folgende(n) Vertragsstaat(en): ES)

1. Verfahren zur Herstellung der Verbindungen der Formel I 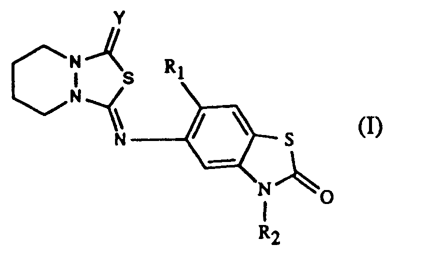 worin
Y Sauerstoff oder Schwefel;
R₁ Wasserstoff oder Fluor und
R₂ Wasserstoff, C₁-C₆-Alkyl, C₂-C₄-Alkenyl, oder C₂-C₆-Alkinyl; durch Halogen substituiertes C₁-C₆-Alkyl, C₂-C₄-Alkenyl, oder C₃-C₆-Alkinyl; C₁-C₄-Alkoxy-C₁-C₄-alkyl, C₁-C₄-Alkoxy-C₁-C₂-alkoxy-C₁-C₂-alkyl, 1-Phenylpropen-3-yl, durch Cyano oder C₃-C₆-Cycloalkyl substituiertes C₁-C₆-Alkyl; Carboxy-C₁-C₄-alkyl, C₁-C₆-Alkoxycarbonyl-C₁-C₄-alkyl, Halogen-C₁-C₆-alkoxycarbonyl-C₁-C₄-alkyl, C₁-C₄-Alkoxy-C₁-C₂-alkoxycarbonyl-C₁-C₄-alkyl, C₁-C₆-Alkoxycarbonyl-C₁-C₂-alkoxycarbonyl-C₁-C₄-alkyl, C₃-C₆-Cycloalkyl-C₁-C₂-alkoxycarbonyl-C₁-C₄-alkyl, C₁-C₅-Alkylaminocarbonyl-C₁-C₄-alkyl, Di-C₁-C₅-alkylaminocarbonyl-C₁-C₄-alkyl, C₃-C₆-Cycloalkyl, C₁-C₄-Alkylthio-C₁-C₄-alkyl, Benzyl, oder durch Halogen substituiertes Benzyl, C₁-C₄-Alkylsulfonyl, C₃-C₆-Alkenyloxy-C₁-C₄-alkyl, C₁-C₈-Alkylcarbonyl, 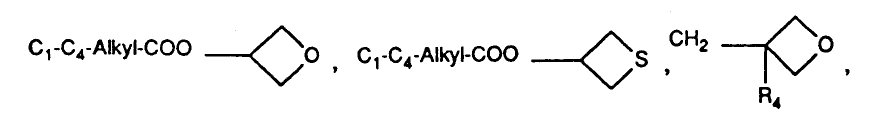 C₁-C₄-Alkylthiocarbonyl-C₁-C₄-alkyl, oder die Gruppe 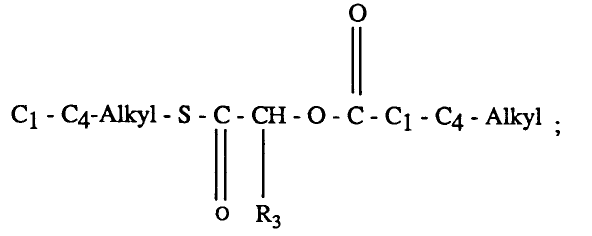
R₃ Wasserstoff oder C₁-C₄-Alkyl; und
R₄ Wasserstoff oder C₁-C₄-Alkyl bedeuten, dadurch gekennzeichnet, daß man ein Isothiocyanat der Formel II 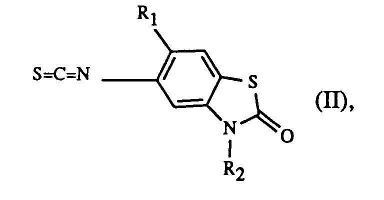 worin die Substituenten R₁ und R₂ die unter Formel I angegebene Bedeutung haben, mit einem Hexahydropyridazin der Formel III 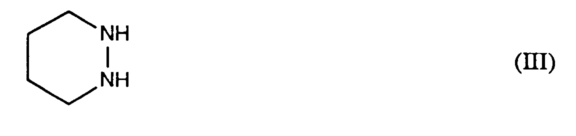 in die Verbindung der Formel IV 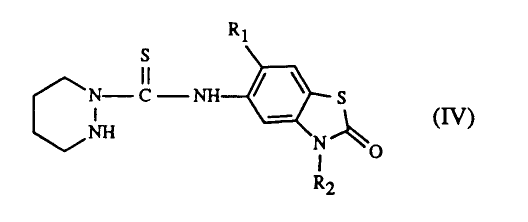 überführt und diese anschließend mit einer Verbindung der Formel V
CYCl₂ (V),
worin Y für Sauerstoff oder Schwefel steht, in Gegenwart einer Base umsetzt.

2. Verfahren nach Anspruch 1 zur Herstellung von Verbindungen der Formel I, Worin R₁ für Fluor steht.

3. Verfahren nach Anspruch 1 zur Herstellung von Verbindungen der Formel I, worin R₁ für Fluor und Y für Sauerstoff steht.

4. Verfahren nach Anspruch I zur Herstellung von Verbindungen der Formel I, worin R₂ Wasserstoff, C₁-C₄-Alkyl, C₂-C₄-Alkenyl, oder C₂-C₄-Alkinyl; durch Halogen substituiertes C₁-C₄-Alkyl, C₂-C₄-Alkenyl, oder C₃-C₄-Alkinyl; C₁-C₄-Alkoxy-C₁-C₄-alkyl, C₁-C₄-Alkoxy-C₁-C₂-alkoxy-C₁-C₂-alkyl, 1-Phenylpropen-3-yl, durch Cyano oder C₃-C₆-Cycloalkyl substituiertes C₁-C₄-Alkyl, Benzyl, oder durch Halogen substituiertes Benzyl, C₃-C₆-Alkenyloxy-C₁-C₄-alkyl, C₁-C₈-Alkylcarbonyl, 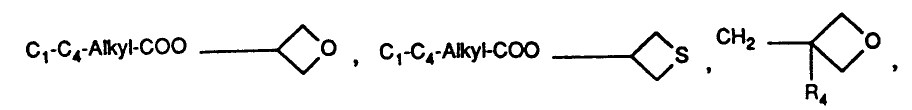 C₁-C₄-Alkylthiocarbonyl-C₁-C₄-alkyl, oder die Gruppe 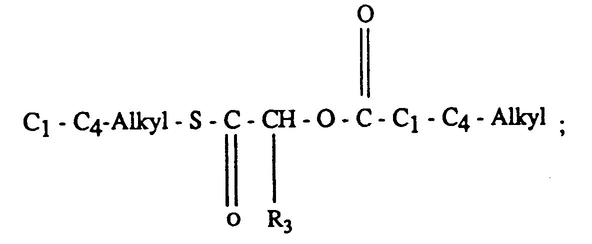 R₃ Wasserstoff oder C₁-C₄-Alkyl; und
R₄ Wasserstoff oder C₁-C₄-Alkyl bedeuten.

5. Verfahren nach Anspruch 1 zur Herstellung von Verbindungen der Formel I, worin R₂ Wasserstoff, C₁-C₄-Alkyl, C₂-C₄-Alkenyl, oder C₂-C₄-Alkinyl; durch Halogen substituiertes C₁-C₄-Alkyl, C₂-C₄-Alkenyl, oder C₃-C₄-Alkinyl; oder C₁-C₂-Alkoxy-C₁-C₂-alkyl bedeuten.

6. Verfahren nach Anspruch 1 zur Herstellung von Verbindungen der Formel I, worin R₂ Wasserstoff, C₁-C₄-Alkyl, C₂-C₄-Alkenyl, durch Fluor substituiertes C₁-C₄-Alkyl oder C₂-C₄-Alkenyl bedeuten.

7. Verfahren nach Anspruch 1 zur Herstellung von Verbindungen der Formel I, worin R₂ C₃-C₆-Cycloalkyl, durch C₃-C₆-Cycloalkyl substituiertes C₁-C₆-Alkyl;C₁-C₄-Alkylthio-C₁-C₄-alkyl, Benzyl, oder durch Halogen substituiertes Benzyl, C₁-C₄-Alkylsulfonyl, C₃-C₆-Alkenyloxy-C₁-C₄-alkyl, C₁-C₈-Alkylcarbonyl, 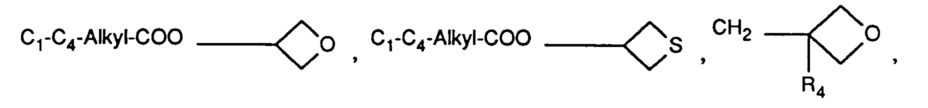 C₁-C₄-Alkylthiocarbonyl-C₁-C₄-alkyl, oder die Gruppe 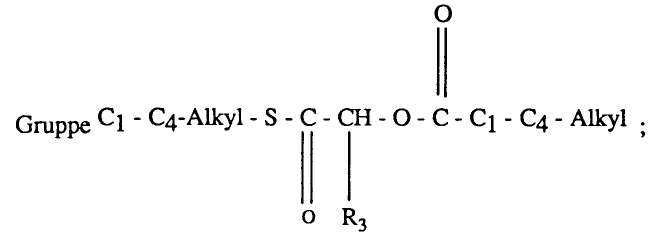 R₃ Wasserstoff oder C₁-C₄-Alkyl; und
R₄ Wasserstoff oder C₁-C₄-Alkyl bedeuten.

8. Verfahren nach Anspruch 7 zur Herstellung von Verbindungen der Formel I, worin Y Sauerstoff, R₁ Fluor und R₃ Wasserstoff oder Methyl bedeuten.

9. Verfahren nach Anspruch 1 zur Herstellung von Verbindungen der Formel I, worin Y für Sauerstoff, R₁ für Fluor und R₂ für C₁-C₃-Alkyl, CH₂F, CH₂CH₂F, CH₂CH₂CH₂F, CHF₂, Allyl, Propargyl, 1-Methyl-3-propinyl, Methoxycarbonylmethyl, Ethoxycarbonylmethyl, Isopropoxycarbonylmethyl, 1-(Methoxycarbonyl)-ethyl, 1-(Ethoxycarbonyl)-ethyl oder 1-(Isopropoxycarbonyl)-ethyl steht.

10. Verfahren nach Anspruch 1 zur Herstellung von 9-[3-(1-Methylethyl)-6-fluor-2(3H)-benzothiazolon-5-yl)-8-thia-1,6-diazabicyclo[4.3.0]nonan-7-on.

11. Verfahren nach Anspruch 1 zur Herstellung von Verbindungen der Formel I, worin R₂ durch Halogen substituiertes C₁-C₆-Alkyl, C₂-C₄-Alkenyl, oder C₃-C₆-Alkinyl; C₁-C₄-Alkoxy-C₁-C₄-alkyl, C₁-C₄-Alkoxy-C₁-C₂-alkoxy-C₁-C₂-alkyl, durch Cyano oder C₃-C₆-Cycloalkyl substituiertes C₁-C₆-Alkyl; Carboxy-C₁-C₄-alkyl, C₁-C₆-Alkoxycarbonyl-C₁-C₄-alkyl, C₁-C₄-Alkoxy-C₁-C₂-alkoxycarbonyl-C₁-C₄-alkyl, C₁-C₆-Alkoxycarbonyl-C₁-C₂-alkoxycarbonyl-C₁-C₄-alkyl, C₃-C₆-Cycloalkyl-C₁-C₂-alkoxycarbonyl-C₁-C₄-alkyl, C₁-C₅-Alkylaminocarbonyl-C₁-C₄-alkyl, Di-C₁-C₅-Alkylaminocarbonyl-C₁-C₄-alkyl, C₃-C₆-Cycloalkyl, C₁-C₄-Alkylthio-C₁-C₄-alkyl, C₃-C₆-Alkenyloxy-C₁-C₄-alkyl, 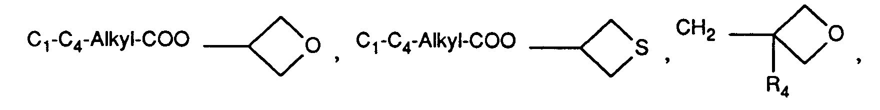 C₁-C₄-Alkylthiocarbonyl-C₁-C₄-alkyl, oder die Gruppe 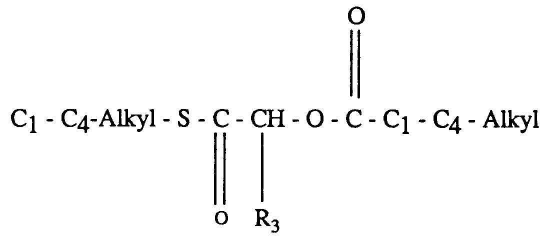 beudetet.

12. Verfahren nach Anspruch 11 zur Herstellung von Verbindungen der Formel I, worin R₂ C₁-C₄-Alkoxy-C₁-C₄-alkyl, C₁-C₄-Alkoxy-C₁-C₂-alkoxy-C₁-C₂-alkyl, durch Cyano substituiertes C₁-C₆-Alkyl; Carboxy-C₁-C₄-alkyl, C₁-C₆-Alkoxycarbonyl-C₁-C₄-alkyl, C₁-C₄-Alkoxy-C₁-C₂-alkoxycarbonyl-C₁-C₄-alkyl, C₁-C₆-Alkoxycarbonyl-C₁-C₂-alkoxycarbonyl-C₁-C₄-alkyl, C₃-C₆-Cycloalkyl-C₁-C₂-alkoxycarbonyl-C₁-C₄-alkyl, C₁-C₅-Alkylaminocarbonyl-C₁-C₄-alkyl, Di-C₁-C₅-alkylaminocarbonyl-C₁-C₄-alkyl oder C₃-C₆-Cycloalkyl bedeutet.

13. Verfahren nach Anspruch 12 zur Herstellung von Verbindungen der Formel I, worin R₂ C₁-C₆-Alkoxycarbonyl-C₁-C₄-alkyl oder C₁-C₄-Alkoxy-C₁-C₂-alkoxycarbonyl-C₁-C₄-alkyl, bedeutet.

14. Verfahren nach Anspruch 11 zur Herstellung von Verbindungen der Formel I, worin R₁ für Fluor steht und Y Sauerstoff bedeutet.

15. Ein herbizides und den Pflanzenwuchs hemmendes Mittel, gekennzeichnet durch ein Gehalt an einem oder mehreren Benzothiazolonderivaten der Formel I, gemäß Anspruch 1.

16. Mittel gemäß Anspruch 15, dadurch gekennzeichnet, daß es zwischen 0,1 % und 95 % Wirkstoff der Formel I gemäß Anspruch 1 enthält.

17. Verfahren zur Bekämpfung unerwünschten Pflanzenwachstums, dadurch gekennzeichnet, daß man einen Wirkstoff der Formel I, gemäß Anspruch 1, oder ein diesen Wirkstoff enthaltendes Mittel gemäß Anspruch 15 in einer wirksamen Menge auf die Pflanzen oder deren Lebensraum appliziert.

18. Verfahren gemäß Anspruch 17, dadurch gekennzeichnet, daß man eine Wirkstoffmenge zwischen 0,001 und 4 kg pro Hektar appliziert.

19. Verfahren gemäß Anspruch 17, zur selektiven pre- oder post-emergenten Bekämpfung von Unkräutern in Nutzpflanzenkulturen.

20. Verfahren zur Hemmung des Pflanzenwachstums, dadurch gekennzeichnet, daß man einen Wirkstoff der Formel I, gemäß Anspruch 1, oder ein diesen Wirkstoff enthaltendes Mittel gemäß Anspruch 15 in einer wirksamen Menge auf die Pflanzen oder deren Lebensraum appliziert.

## Claims (Claims for the following Contracting State(s): AT, BE, CH, DE, DK, FR, GB, GR, IE, IT, LI, LU, MC, NL, PT, SE)

1. A benzothiazolone derivative of formula I 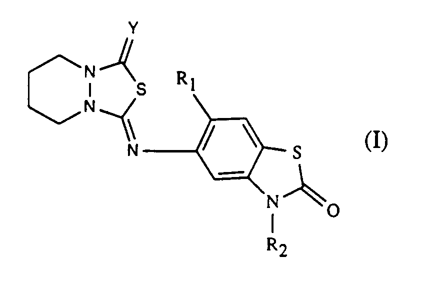 wherein
Y is oxygen or sulfur;
R₁ is hydrogen or fluorine; and
R₂ is hydrogen, C₁-C₆alkyl, C₂-C₄alkenyl, or C₂-C₆alkynyl; halo-substituted C₁-C₆alkyl, C₂-C₄alkenyl, or C₃-C₆alkynyl; C₁-C₄alkoxy-C₁-C₄alkyl, C₁-C₄alkoxy-C₁-C₂alkoxy-C₁-C₂alkyl, 1-phenylpropen-3-yl, cyano- or C₃-C₆cycloalkyl-substituted C₁-C₆alkyl; carboxy-C₁-C₄alkyl, C₁-C₆alkoxycarbonyl-C₁-C₄alkyl, halo-C₁-C₆alkoxycarbonyl-C₁-C₄alkyl, C₁-C₄alkoxy-C₁-C₂alkoxycarbonyl-C₁-C₄alkyl, C₁-C₆alkoxycarbonyl-C₁-C₂alkoxycarbonyl-C₁-C₄alkyl, C₃-C₆cycloalkyl-C₁-C₂alkoxycarbonyl-C₁-C₄alkyl, C₁-C₅alkylaminocarbonyl-C₁-C₄alkyl, di-C₁-C₅alkylaminocarbonyl-C₁-C₄alkyl, C₃-C₆cycloalkyl, C₁-C₄alkylthio-C₁-C₄alkyl, benzyl, or halo-substituted benzyl, C₁-C₄alkylsulfonyl, C₃-C₆-alkenyloxy-C₁-C₄alkyl, C₁-C₈alkylcarbonyl, 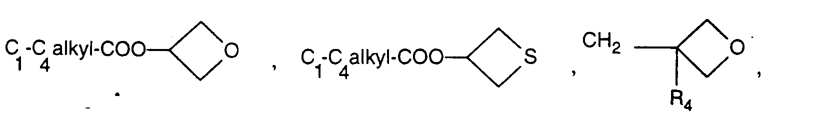 C₁-C₄alkylthiocarbonyl-C₁-C₄alkyl, or the group 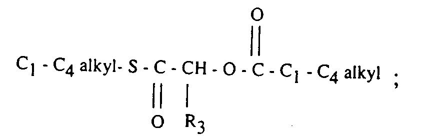
R₃ is hydrogen or C₁-C₄alkyl; and
R₄ is hydrogen or C₁-C₄alkyl.

2. A compound according to claim 1 wherein R₁ is fluorine.

3. A compound according to claim 1 wherein R₁ is fluorine and Y is oxygen.

4. A compound according to claim 1 wherein R₂ is hydrogen, C₁-C₄alkyl, C₂-C₄alkenyl, or C₂-C₄alkynyl; halo-substituted C₁-C₄alkyl, C₂-C₄alkenyl, or C₃-C₄alkynyl; C₁-C₄alkoxy-C₁-C₄alkyl, C₁-C₄alkoxy-C₁-C₂alkoxy-C₁-C₂alkyl, 1-phenylpropen-3-yl, cyano- or C₃-C₆cycloalkyl-substituted C₁-C₄alkyl, benzyl, or halo-substituted benzyl, C₃-C₆alkenyloxy-C₁-C₄alkyl, C₁-C₈alkylcarbonyl, 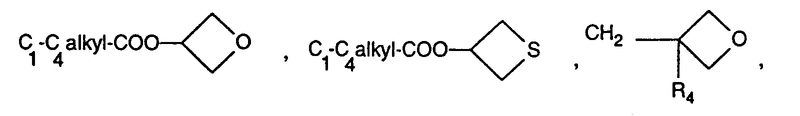 C₁-C₄alkylthiocarbonyl-C₁-C₄alkyl, or the group 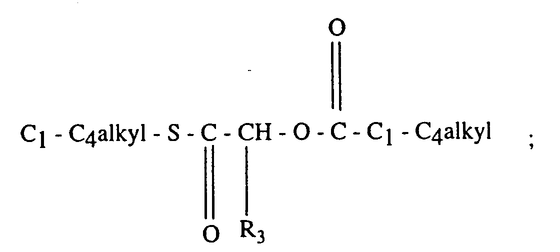 R₃ is hydrogen or C₁-C₄alkyl; and
R₄ is hydrogen or C₁-C₄alkyl.

5. A compound according to claim 1 wherein R₂ is hydrogen, C₁-C₄alkyl, C₂-C₄alkenyl, or C₂-C₄alkynyl; halo-substituted C₁-C₄alkyl, C₂-C₄alkenyl, or C₃-C₄alkynyl; or C₁-C₂alkoxy-C₁-C₂alkyl.

6. A compound according to claim 1 wherein R₂ is hydrogen, C₁-C₄alkyl, C₂-C₄alkenyl, fluoro-substituted C₁-C₄alkyl or C₂-C₄alkenyl.

7. A compound according to claim 1 wherein R₂ is C₃-C₆cycloalkyl, C₃-C₆cycloalkyl-substituted C₁-C₆alkyl; C₁-C₄alkylthio-C₁-C₄alkyl, benzyl, or halo-substituted benzyl, C₁-C₄alkylsulfonyl, C₃-C₆alkenyloxy-C₁-C₄alkyl, C₁-C₈alkylcarbonyl, 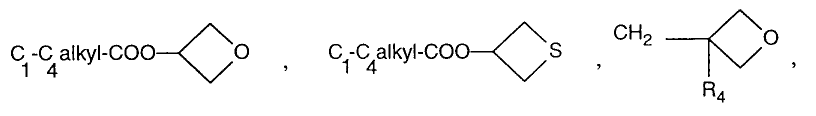 C₁-C₄-alkylthiocarbonyl-C₁-C₄alkyl, or the group 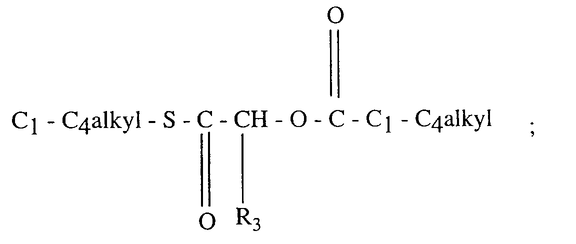 R₃ is hydrogen or C₁-C₄alkyl; and
R₄ is hydrogen or C₁-C₄alkyl.

8. A compound according to claim 7 wherein Y is oxygen, R₁ is fluorine and R₃ is hydrogen or methyl.

9. A compound according to claim 1 wherein Y is oxygen, R₁ is fluorine and R₂ is C₁-C₃alkyl, CH₂F, CH₂CH₂F, CH₂CH₂CH₂F, CHF₂, allyl, propargyl, 1-methyl-3-propynyl, methoxycarbonylmethyl, ethoxycarbonylmethyl, isopropoxycarbonylmethyl, 1-(methoxycarbonyl)-ethyl, 1-(ethoxycarbonyl)-ethyl or 1-(isopropoxycarbonyl)-ethyl.

10. 9-[6-Fluoro-3-isopropyl-2(3H)-benzothiazolon-5-yl]imino-8-thia-1,6-diazabicyclo[4.3.0]nonan-7-one according to claim 1.

11. A compound according to claim 1 wherein R₂ is halo-substituted C₁-C₆alkyl, C₂-C₄alkenyl, or C₃-C₆alkynyl; C₁-C₄alkoxy-C₁-C₄alkyl, C₁-C₄alkoxy-C₁-C₂alkoxy-C₁-C₂alkyl, cyano- or C₃-C₆cycloalkyl-substituted C₁-C₆alkyl; carboxy-C₁-C₄alkyl, C₁-C₆alkoxycarbonyl-C₁-C₄alkyl, C₁-C₄alkoxy-C₁-C₂alkoxycarbonyl-C₁-C₄alkyl, C₁-C₆alkoxycarbonyl-C₁-C₂alkoxycarbonyl-C₁-C₄alkyl, C₃-C₆cycloalkyl-C₁-C₂alkoxycarbonyl-C₁-C₄alkyl, C₁-C₅alkylaminocarbonyl-C₁-C₄alkyl, di-C₁-C₅alkylaminocarbonyl-C₁-C₄alkyl, C₃-C₆cycloalkyl, C₁-C₄alkylthio-C₁-C₄alkyl, C₃-C₆alkenyloxy-C₁-C₄alkyl, 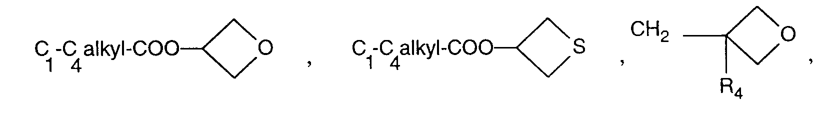 C₁-C₄alkylthiocarbonyl-C₁-C₄alkyl, or the group

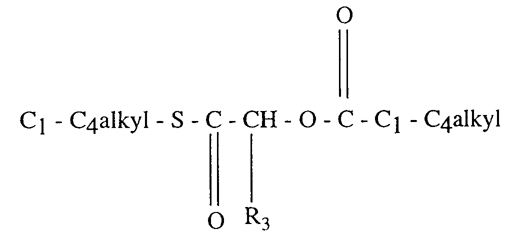

12. A compound according to claim 11 wherein R₂ is C₁-C₄alkoxy-C₁-C₄alkyl, C₁-C₄-alkoxy-C₁-C₂alkoxy-C₁-C₂alkyl, cyano-substituted C₁-C₆alkyl; carboxy-C₁-C₄alkyl, C₁-C₆alkoxycarbonyl-C₁-C₄alkyl, C₁-C₄alkoxy-C₁-C₂alkoxycarbonyl-C₁-C₄alkyl, C₁-C₆alkoxycarbonyl-C₁-C₂alkoxycarbonyl-C₁-C₄alkyl, C₃-C₆cycloalkyl-C₁-C₂alkoxycarbonyl-C₁-C₄alkyl, C₁-C₅alkylaminocarbonyl-C₁-C₄alkyl, di-C₁-C₅alkylaminocarbonyl-C₁-C₄alkyl or C₃-C₆cycloalkyl.

13. A compound according to claim 12 wherein R₂ is C₁-C₆alkoxycarbonyl-C₁-C₄alkyl or C₁-C₄alkoxy-C₁-C₂alkoxycarbonyl-C₁-C₄alkyl.

14. A compound according to claim 11 wherein R₁ is fluorine and Y is oxygen.

15. A process for the preparation of a compound of formula I 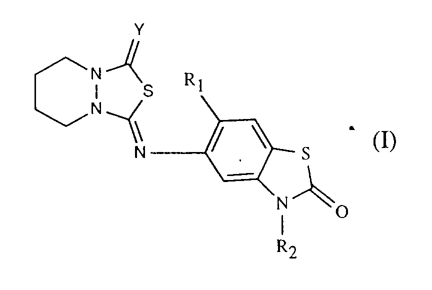 wherein
Y is oxygen or sulfur;
R₁ is hydrogen or fluorine; and
R₂ is hydrogen, C₁-C₆alkyl, C₂-C₄alkenyl, or C₂-C₆alkynyl; halo-substituted C₁-C₆alkyl, C₂-C₄alkenyl, or C₃-C₆alkynyl; C₁-C₄alkoxy-C₁-C₄alkyl, C₁-C₄alkoxy-C₁-C₂alkoxy-C₁-C₂alkyl, 1-phenylpropen-3-yl, cyano- or C₃-C₆cycloalkyl-substituted C₁-C₆alkyl; carboxy-C₁-C₄alkyl, C₁-C₆alkoxycarbonyl-C₁-C₄alkyl, halo-C₁-C₆alkoxycarbonyl-C₁-C₄alkyl, C₁-C₄alkoxy-C₁-C₂alkoxycarbonyl-C₁-C₄alkyl, C₁-C₆alkoxycarbonyl-C₁-C₂alkoxycarbonyl-C₁-C₄alkyl, C₃-C₆cycloalkyl-C₁-C₂alkoxycarbonyl-C₁-C₄alkyl, C₁-C₅alkylaminocarbonyl-C₁-C₄alkyl, di-C₁-C₅alkylaminocarbonyl-C₁-C₄alkyl, C₃-C₆cycloalkyl, C₁-C₄alkylthio-C₁-C₄alkyl, benzyl, or halo-substituted benzyl, C₁-C₄alkylsulfonyl, C₃-C₆alkenyloxy-C₁-C₄alkyl, C₁-C₈alkylcarbonyl, 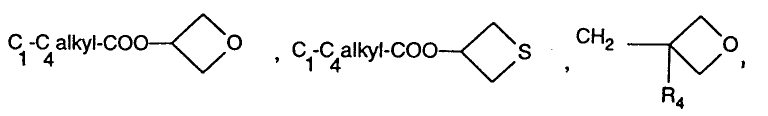 , C₁-C₄alkylthiocarbonyl-C₁-C₄alkyl, or the group 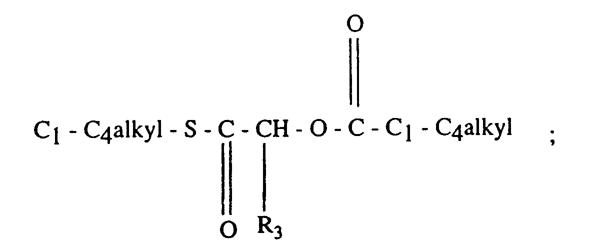
R₃ is hydrogen or C₁-C₄alkyl; and
R₄ is hydrogen or C₁-C₄alkyl; which process comprises converting an isothiocyanate of formula II 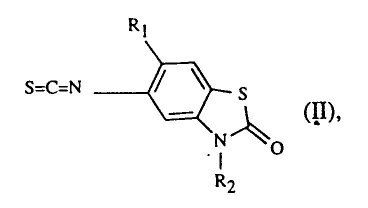 wherein the substituents R₁ and R₂ are as defined for formula I, with a hexahydropyridazine of formula III 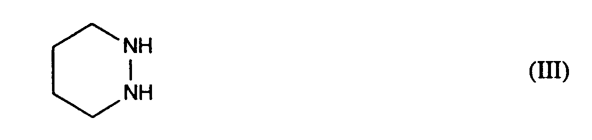 into a compound of formula IV 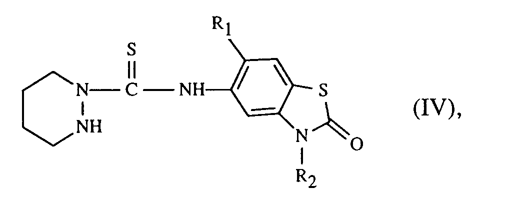 which is then reacted with a compound of formula V
CYCl₂ (V),
wherein Y is oxygen or sulfur, in the presence of a base.

16. A herbicidal and plant growth regulating composition comprising one or more benzothiazolone derivatives of formula I according to claim 1.

17. A composition according to claim 16 comprising from 0.1 % to 95 % of a compound of formula I according to claim 1.

18. A method of controlling undesirable plant growth, which comprises applying a compound of formula I according to claim 1, or a composition comprising such a compound according to claim 16, in an effective amount to the plants or to the locus thereof.

19. A method according to claim 18, which comprises applying the active ingredient in an amount of from 0.001 to 4 kg per hectare.

20. A method according to claim 18 for the selective pre- or post-emergence control of weeds in crops of useful plants.

21. A method of inhibiting plant growth, which comprises applying a compound of formula I according to claim 1, or a composition comprising such a compound according to claim 16, in an effective amount to the plants or to the locus thereof.

## Claims (Claims for the following Contracting State(s): ES)

1. A process for the preparation of a compound of formula I 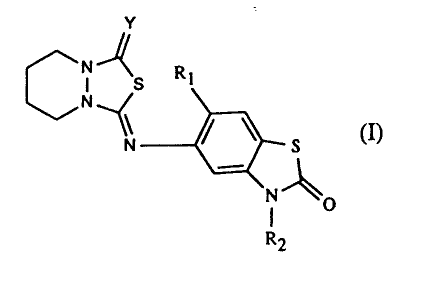 wherein
Y is oxygen or sulfur;
R₁ is hydrogen or fluorine; and
R₂ is hydrogen, C₁-C₆alkyl, C₂-C₄alkenyl, or C₂-C₆alkynyl; halo-substituted C₁-C₆alkyl, C₂-C₄alkenyl, or C₃-C₆alkynyl; C₁-C₄alkoxy-C₁-C₄alkyl, C₁-C₄alkoxy-C₁-C₂alkoxy-C₁-C₂alkyl, 1-phenylpropen-3-yl, cyano- or C₃-C₆cycloalkyl-substituted C₁-C₆alkyl; carboxy-C₁-C₄alkyl, C₁-C₆alkoxycarbonyl-C₁-C₄alkyl, halo-C₁-C₆alkoxycarbonyl-C₁-C₄alkyl, C₁-C₄alkoxy-C₁-C₂alkoxycarbonyl-C₁-C₄alkyl, C₁-C₆alkoxycarbonyl-C₁-C₂alkoxycarbonyl-C₁-C₄alkyl, C₃-C₆cycloalkyl-C₁-C₂alkoxycarbonyl-C₁-C₄alkyl, C₁-C₅alkylaminocarbonyl-C₁-C₄alkyl, di-C₁-C₅alkylaminocarbonyl-C₁-C₄alkyl, C₃-C₆cycloalkyl, C₁-C₄alkylthio-C₁-C₄alkyl, benzyl, or halo-substituted benzyl, C₁-C₄alkylsulfonyl, C₃-C₆alkenyloxy-C₁-C₄alkyl, C₁-C₈alkylcarbonyl, 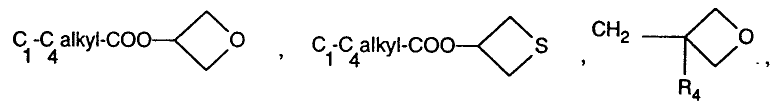 C₁-C₄alkylthiocarbonyl-C₁-C₄alkyl, or the group 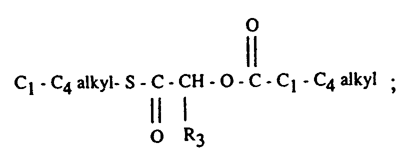
R₃ is hydrogen or C₁-C₄alkyl; and
R₄ is hydrogen or C₁-C₄alkyl; which process comprises converting an isothiocyanate of formula II 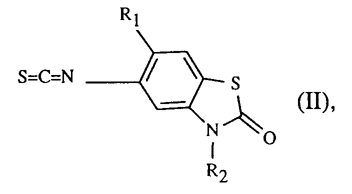 wherein the substituents R₁ and R₂ are as defined for formula I, with a hexahydropyridazine of formula III 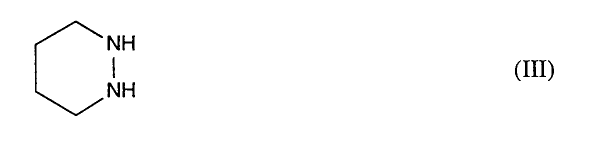 into a compound of formula IV 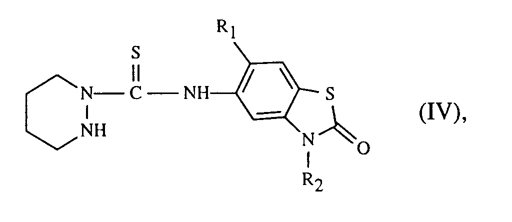 which is then reacted with a compound of formula V
CYCl₂ (V),
wherein Y is oxygen or sulfur, in the presence of a base.

2. A process according to claim 1 for the preparation of a compound of formula I wherein R₁ is fluorine.

3. A process according to claim 1 for the preparation of a compound of formula I wherein R₁ is fluorine and Y is oxygen.

4. A process according to claim 1 for the preparation of a compound of formula I wherein R₂ is hydrogen, C₁-C₄alkyl, C₂-C₄alkenyl, or C₂-C₄alkynyl; halo-substituted C₁-C₄alkyl, C₂-C₄alkenyl, or C₃-C₄alkynyl; C₁-C₄alkoxy-C₁-C₄alkyl, C₁-C₄alkoxy-C₁-C₂alkoxy-C₁-C₂alkyl, 1-phenylpropen-3-yl, cyano- or C₃-C₆cycloalkyl-substituted C₁-C₄alkyl, benzyl, or halo-substituted benzyl, C₃-C₆alkenyloxy-C₁-C₄alkyl, C₁-C₈alkylcarbonyl, 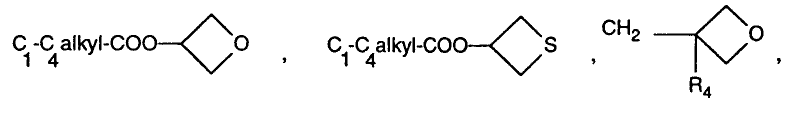 C₁-C₄alkylthiocarbonyl-C₁-C₄alkyl, or the group 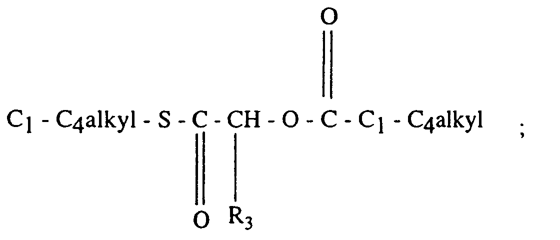 R₃ is hydrogen or C₁-C₄alkyl; and
R₄ is hydrogen or C₁-C₄alkyl.

5. A process according to claim 1 for the preparation of a compound of formula I wherein R₂ is hydrogen, C₁-C₄alkyl, C₂-C₄alkenyl, or C₂-C₄alkynyl; halo-substituted C₁-C₄alkyl, C₂-C₄alkenyl, or C₃-C₄alkynyl; or C₁-C₂alkoxy-C₁-C₂alkyl.

6. A process according to claim 1 for the preparation of a compound of formula I wherein R₂ is hydrogen, C₁-C₄alkyl, C₂-C₄alkenyl, fluoro-substituted C₁-C₄alkyl or C₂-C₄alkenyl.

7. A process according to claim 1 for the preparation of a compound of formula I wherein R₂ is C₃-C₆cycloalkyl, C₃-C₆cycloalkyl-substituted C₁-C₆alkyl; C₁-C₄alkylthio-C₁-C₄alkyl, benzyl, or halo-substituted benzyl, C₁-C₄alkylsulfonyl, C₃-C₆alkenyloxy-C₁-C₄alkyl, C₁-C₈alkylcarbonyl, 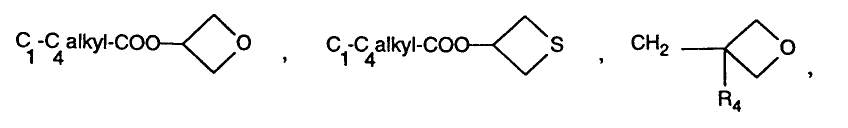 C₁-C₄alkylthiocarbonyl-C₁-C₄alkyl, or the group 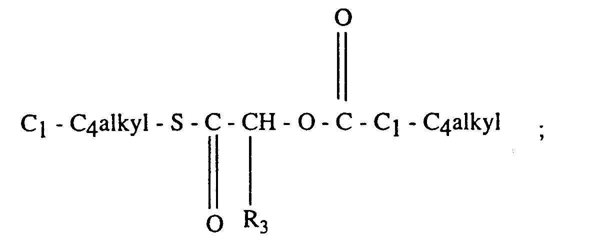 R₃ is hydrogen or C₁-C₄alkyl; and
R₄ is hydrogen or C₁-C₄alkyl.

8. A process according to claim 7 for the preparation of a compound of formula I wherein Y is oxygen, R₁ is fluorine and R₃ is hydrogen or methyl.

9. A process according to claim 1 for the preparation of a compound of formula I wherein Y is oxygen, R₁ is fluorine and R₂ is C₁-C₃alkyl, CH₂F, CH₂CH₂F, CH₂CH₂CH₂F, CHF₂, allyl, propargyl, 1-methyl-3-propynyl, methoxycarbonylmethyl, ethoxycarbonylmethyl, isopropoxycarbonylmethyl, 1-(methoxycarbonyl)-ethyl, 1-(ethoxycarbonyl)-ethyl or 1-(isopropoxycarbonyl)-ethyl.

10. A process according to claim 1 for the preparation of 9-[3-(1-methylethyl)-6-fluoro-2(3H)-benzothiazolon-5-yl)-8-thia-1,6-diazabicyclo[4.3.0]nonan-7-one.

11. A process according to claim 1 for the preparation of a compound of formula I wherein R₂ is halo-substituted C₁-C₆alkyl, C₂-C₄alkenyl, or C₃-C₆alkynyl; C₁-C₄alkoxy-C₁-C₄alkyl, C₁-C₄alkoxy-C₁-C₂alkoxy-C₁-C₂alkyl, cyano- or C₃-C₆cycloalkyl-substituted C₁-C₆alkyl; carboxy-C₁-C₄alkyl, C₁-C₆alkoxycarbonyl-C₁-C₄alkyl, C₁-C₄alkoxy-C₁-C₂alkoxycarbonyl-C₁-C₄alkyl, C₁-C₆alkoxycarbonyl-C₁-C₂alkoxycarbonyl-C₁-C₄alkyl, C₃-C₆cycloalkyl-C₁-C₂alkoxycarbonyl-C₁-C₄alkyl, C₁-C₅alkylaminocarbonyl-C₁-C₄alkyl, di-C₁-C₅alkylaminocarbonyl-C₁-C₄alkyl, C₃-C₆cycloalkyl, C₁-C₄alkylthio-C₁-C₄alkyl, C₃-C₆alkenyloxy-C₁-C₄alkyl, 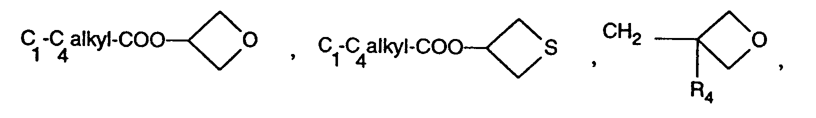 C₁-C₄alkylthiocarbonyl-C₁-C₄alkyl, or the group

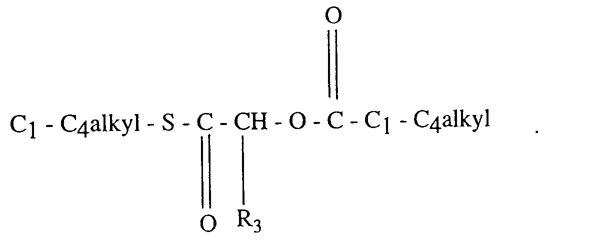

12. A process according to claim 11 for the preparation of a compound of formula I wherein R₂ is C₁-C₄alkoxy-C₁-C₄alkyl, C₁-C₄alkoxy-C₁-C₂alkoxy-C₁-C₂alkyl, cyano-substituted C₁-C₆alkyl; carboxy-C₁-C₄alkyl, C₁-C₆alkoxycarbonyl-C₁-C₄alkyl, C₁-C₄alkoxy-C₁-C₂alkoxycarbonyl-C₁-C₄alkyl, C₁-C₆alkoxycarbonyl-C₁-C₂alkoxycarbonyl-C₁-C₄alkyl, C₃-C₆cycloalkyl-C₁-C₂alkoxycarbonyl-C₁-C₄alkyl, C₁-C₅alkylaminocarbonyl-C₁-C₄alkyl, di-C₁-C₅alkylaminocarbonyl-C₁-C₄alkyl or C₃-C₆cycloalkyl.

13. A process according to claim 12 for the preparation of a compound of formula I wherein R₂ is C₁-C₆alkoxycarbonyl-C₁-C₄alkyl or C₁-C₄alkoxy-C₁-C₂alkoxycarbonyl-C₁-C₄alkyl.

14. A process according to claim 11 for the preparation of a compound of formula I wherein R₁ is fluorine and Y is oxygen.

15. A herbicidal and plant growth regulating composition comprising one or more benzothiazolone derivatives of formula I according to claim 1.

16. A composition according to claim 15 comprising from 0.1 % to 95 % of a compound of formula I according to claim 1.

17. A method of controlling undesirable plant growth, which comprises applying a compound of formula I according to claim 1, or a composition comprising such a compound according to claim 15, in an effective amount to the plants or to the locus thereof.

18. A method according to claim 17, which comprises applying the active ingredient in an amount of from 0.001 to 4 kg per hectare.

19. A method according to claim 17 for the selective pre- or post-emergence control of weeds in crops of useful plants.

20. A method of inhibiting plant growth, which comprises applying a compound of formula I according to claim 1, or a composition comprising such a compound according to claim 15, in an effective amount to the plants or to the locus thereof.

## Revendications (Revendications pour l'(les) Etat(s) contractant(s) suivant(s): AT, BE, CH, DE, DK, FR, GB, GR, IE, IT, LI, LU, MC, NL, PT, SE)

1. Dérivés de la benzothiazolone répondant à la formule I 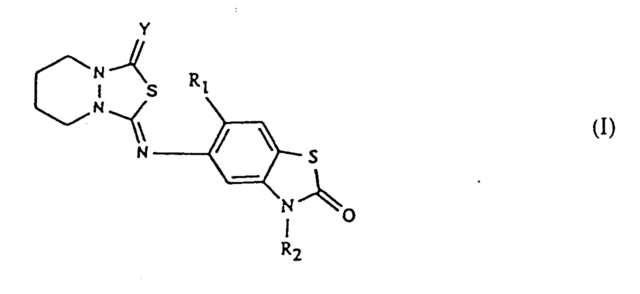 dans laquelle
Y représente l'oxygène ou le soufre;
R₁ représente l'hydrogène ou le fluor et
R₂ représente l'hydrogène un groupe alkyle en C₁-C₆, alcényle en C₂-C₄ ou alcynyle en C₂-C₆ ; un groupe alkyle en C₁-C₆, alcényle en C₂-C₄ ou alcynyle en C₃-C₆ substitué par des halogènes ; un groupe (alcoxy en C₁-C₄)-alkyle, en C₁-C₄, (alcoxy en C₁-C₄)-(alcoxy en C₁-C₂)-alkyle en C₁-C₂, 1-phénylpropén-3-yle, alkyle en C₁-C₆ portant un substituant cyano ou cycloalkyle en C₃-C₆ ; carboxyalkyle en C₁-C₄, (alcoxy en C₁-C₆)-carbonylalkyle C₁-C₄, halogéno-(alcoxy en C₁-C₆)-carbonylalkyle en C₁-C₄, (alcoxy en C₁-C₄)-(alcoxy en C₁-C₂)-carbonylalkyle en C₁-C₄, (alcoxy en C₁-C₆)-carbonyl-(alcoxy en C₁-C₂)-carbonylalkyle en C₁-C₄, (cycloalkyle en C₃-C₆)-(alcoxy en C₁-C2)-carbonylalkyle en C₁-C₄, (alkyle en C₁-C₅)-aminocarbonylalkyle en C₁-C₄, di-(alkyle en C₁-C₅)-aminocarbonylalkyle en C₁-C₄, cycloalkyle en C₃-C₆, (alkylthio en C₁-C₄)-alkyle en C₁-C₄, benzyle ou benzyle substitué par des halogènes, alkyl-sulfonyle en C₁-C₄, (alcényle en C₃-C₆)-oxyalkyle en C₁-C₄, (alkyle en C₁-C₈)-carbonyle, 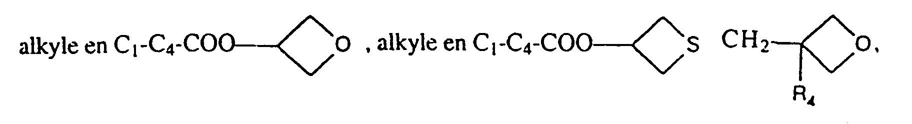 (alkyle en C₁-C₄)-thiocarbonylalkyle en C₁-C₄, ou le groupe 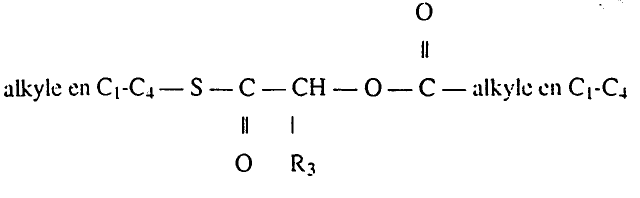
R₃ représente l'hydrogène ou un groupe alkyle en C₁-C₄ et
R₄ représente l'hydrogène ou un groupe alkyle en C₁-C₄.

2. Composés selon la revendication 1, caractérisés en ce que R₁ représente le fluor.

3. Composés selon la revendication 1, caractérisés en ce que R₁ représente le fluor et Y l'oxygène.

4. Composés selon la revendication 1, caractérisés en ce que
R₂ représente l'hydrogène, un groupe alkyle en C₁-C₆, alcényle en C₂-C₄ ou alcynyle en C₂-C₆ ; un groupe alkyle en C₁-C₆, alcényle en C₂-C₄ ou alcynyle en C₃-C₆ substitué par des halogènes ; un groupe (alcoxy en C₁-C₄)-alkyle en C₁-C₄, (alcoxy en C₁-C₄)-(alcoxy en C₁-C₂)-alkyle en C₁-C₂, 1-phénylpropén-3-yle, alkyle en C₁-C₆ portant un substituant cyano ou cycloalkyle en C₃-C₆ ; carboxyalkyle en C₁-C₄, (alcoxy en C₁-C₆)-carbonylalkyle en C₁-C₄, halogéno-(alcoxy en C₁-C₆)-carbonylalkyle en C₁-C₄, (alcoxy en C₁-C₄)-(alcoxy en C₁-C₂)-carbonylalkyle en C₁-C₄, (alcoxy en C₁-C₆)-carbonyl-(alcoxy en C₁-C₂)-carbonylalkyle en C₁-C₄, (cycloalkyle en C₃-C₆)-(alcoxy en C₁-C2)-carbonylalkyle en C₁-C₄, (alkyle en C₁-C₅)-aminocarbonylalkyle en C₁-C₄, di-(alkyle en C₁-C₅)-aminocarbonylalkyle en C₁-C₄, cycloalkyle en C₃-C₆, (alkylthio en C₁-C₄)-alkyle en C₁-C₄, benzyle ou benzyle substitué par des halogènes, alkyl-sulfonyle en C₁-C₄, (alcényle en C₃-C₆)-oxyalkyle en C₁-C₄, (alkyle en C₁-C₈)-carbonyle, 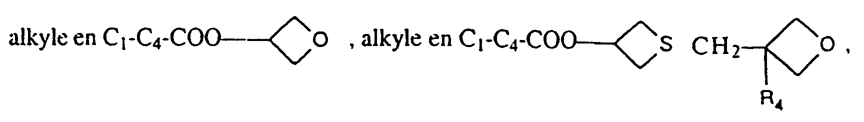 (alkyle en C₁-C₄)-thiocarbonylalkyle en C₁-C₄, ou le groupe 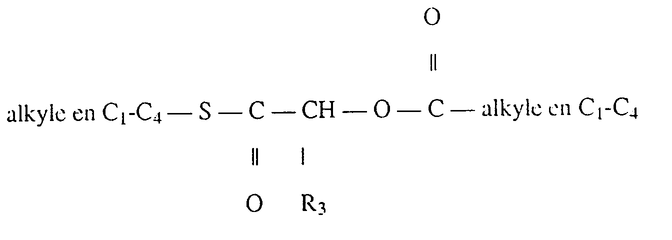 R₃ représente l'hydrogène ou un groupe alkyle en C₁-C₄ et
R₄ représente l'hydrogène ou un groupe alkyle en C₁-C₄.

5. Composés selon la revendication 1, caractérisés en ce que R₂ représente l'hydrogène, un groupe alkyle en C₁-C₄ alcényle en C₂-C₄ ou alcynyle en C₂-C₄ ; un groupe alkyle en C₁-C₄, alcényle en C₂-C₄ ou alcynyle en C₃-C₄ substitué par des halogènes ; ou un groupe (alcoxy en C₁-C₂)alkyle en C₁-C₂.

6. Composés selon la revendication 1, caractérisés en ce que R₂ représente l'hydrogène, un groupe alkyle en C₁-C₄, alcényle en C₂-C₄, ou un groupe alkyle en C₁-C₄ ou alcényle en C₂-C₄ substitué par le fluor.

7. Composés selon la revendication 1, caractérisés en ce que R₂ représente un groupe cycloalkyle en C₃-C₆, alkyle en C₁-C₆ substitué par un groupe cycloalkyle en C₃-C₆ ; un groupe (alkyle en C₁-C₄)-thioalkyle en C₁-C₄, un groupe benzyle éventuellement substitué par des halogènes un groupe alkylsulfonyle en C₁-C₄, (alcényle en C₃-C₆)-oxyalkyle en C₁-C₄, (alkyle en C₁-C₈)-carbonyle, 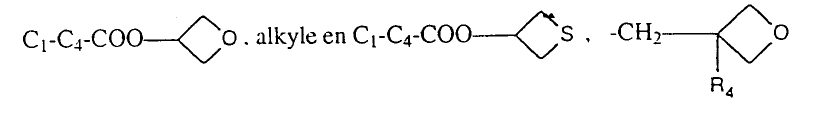 un groupe (alkyle en C₁-C₄)-thiocarbonylalkyle en C₁-C₄ ou le groupe 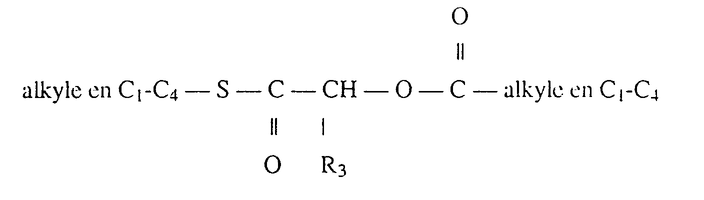 R₃ représente l'hydrogène ou un groupe alkyle en C₁-C₄
R₄ représente l'hydrogène ou un groupe alkyle en C₁-C₄,

8. Composés selon la revendication 7, caractérisés en ce que Y représente l'oxygène, R₁ le fluor et R₃ l'hydrogène ou un groupe méthyle.

9. Composés selon la revendication 1, caractérisés en ce que Y représente l'oxygène, R₁ le fluor et R₂ un groupe alkyle en C₁-C₃, CH₂F, CH₂CH₂F, CH₂CH₂CH₂F, CHF₂, un groupe allyle, propargyle, 1-méthyl-3-propynyle, méthoxycarbonylméthyle, éthoxycarbonylméthyle, isopropoxycarbonylméthyle, 1-(méthoxycarbonyl)-éthyle, 1-(éthoxycarbonyl)éthyle ou 1-(isopropoxycarbonyl)-éthyle.

10. La 9-[6-fluoro-3-isopropyl-2(3H)-benzothiazolone-5-yl]-imino-8-thia-1,6-diazabicyclo[4.3.0]nonane-7-one selon revendication 1.

11. Composés selon la revendication 1, caractérisés en ce que R₂ représente un groupe alkyle en C₁-C₆, alcényle en C₂-C₄ ou alcynyle en C₃-C₆ substitué par des halogènes ; un groupe (alcoxy en C₁-C₄)-alkyle en C₁-C₄, un groupe (alcoxy en C₁-C₄)-(alcoxy en C₁-C₂)-alkyle en C₁-C₂, un groupe alkyle en C₁-C₆ substitué par un groupe cyano ou cycloalkyle en C₃-C₆ ; un groupe carboxyalkyle en C₁-C₄, (alcoxy en C₁-C₆)-carbonylalkyle en C₁-C₄, un groupe (alcoxy en C₁-C₄)-(alcoxy en C₁-C₂)-carbonylalkyle en C₁-C₄, (alcoxy en C₁-C₆)-carbonyl-(alcoxy en C₁-C₂)-carbonylalkyle en C₁-C₄, (cycloalkyle en C₃-C₆)-(alcoxy en C₁-C₂)-carbonylalkyle en C₁-C₄, (alkyle en C₁-C₅)-aminocarbonyl-alkyle en C₁-C₄ di-(alkyle en C₁-C₅)-aminocarbonylalkyle en C₁-C₄, cycloalkyle en C₃-C₆, (alkylthio en C₁-C₄)-alkyle en C₁-C₄, (alcényle en C₃-C₆)-oxyalkyle en C₁-C₄, 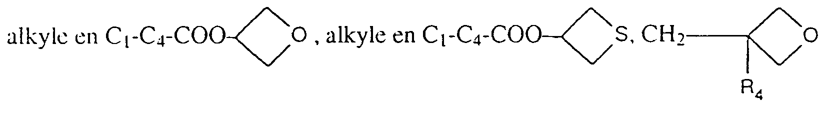 (alkyle en C₁-C₄)-thiocarbonyl-alkyle en C₁ - C₄ ou le groupe

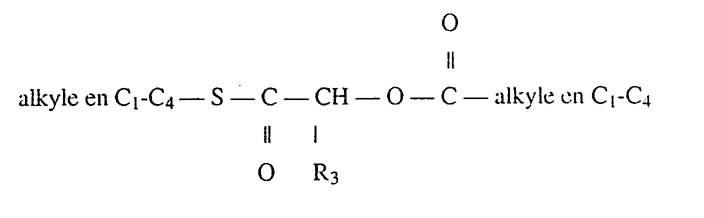

12. Composés selon la revendication 11, caractérisés en ce que R₂ représente un groupe (alcoxy en C₁-C₄)-alkyle en C₁-C₄, (alcoxy en C₁-C₄)-(alcoxy en C₁-C₂)-alkyle en C₁-C₂, alkyle en C₁-C₆ substitué par un groupe cyano ; carboxyalkyle en C₁-C₄, (alcoxy en C₁-C₆)-carbonylalkyle en C₁-C₄, (alcoxy en C₁-C₄)-(alcoxy en C₁-C₂)-carbonylalkyle en C₁-C₄, (alcoxy en C₁-C₆)-carbonyl-(alcoxy en C₁-C₂)-carbonylalkyle en C₁-C₄, (cycloalkyle en C₃-C₆)-(alcoxy en C₁-C₂)-carbonylalkyle en C₁-C₄, (alkyle en C₁-C₅)-aminocarbonylalkyle en C₁-C₄, di-(alkyle en C₁-C₅)-aminocarbonylalkyle en C₁-C₄ ou cycloalkyle en C₃-C₆.

13. Composés selon la revendication 12, caractérisés en ce que R₂ représente un groupe (alcoxy en C₁-C₆)-carbonylalkyle en C₁-C₄ ou (alcoxy en C₁-C₄)-(alcoxy en C₁-C₂)-carbonylalkyle en C₁-C₄.

14. Composés selon la revendication 11, caractérisés en ce que R₁ représente le fluor, et Y l'oxygène.

15. Procédé de préparation des composés de formule I 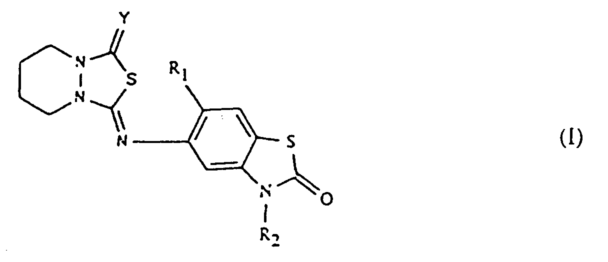 dans laquelle
Y représente l'oxygène ou le soufre ;
R₁ représente l'hydrogène ou le fluor et
R₂ représente l'hydrogène, un groupe alkyle en C₁-C₆, alcényle en C₂-C₄ ou alcynyle en C₂-C₆ ; un groupe alkyle en C₁-C₆, alcényle en C₂-C₄ ou alcynyle en C₃-C₆ substitué par des halogènes ; un groupe (alcoxy en C₁-C₄)-alkyle en C₁-C₄, (alcoxy en C₁-C₄)-(alcoxy en C₁-C₂)-alkyle en C₁-C₂, 1-phénylpropén-3-yle, alkyle en C₁-C₆ portant un substituant cyano ou cycloalkyle en C₃-C₆ ; carboxyalkyle en C₁-C₄, (alcoxy en C₁-C₆)-carbonylalkyle en C₁-C₄, halogéno-(alcoxy en C₁-C₆)-carbonylalkyle en C₁-C₄, (alcoxy en C₁-C₄)-(alcoxy en C₁-C₂)-carbonylalkyle en C₁-C₄, (alcoxy en C₁-C₆)-carbonyl-(alcoxy en C₁-C₂)-carbonylalkyle en C₁-C₄, (cycloalkyle en C₃-C₆)-(alcoxy en C₁-C2)-carbonylalkyle en C₁-C₄, (alkyle en C₁-C₅)-aminocarbonylalkyle en C₁-C₄, di-(alkyle en C₁-C₅)-aminocarbonylalkyle en C₁-C₄, cycloalkyle en C₃-C₆, (alkylthio en C₁-C₄)-alkyle en C₁-C₄, benzyle ou benzyle substitué par des halogènes, alkyl-sulfonyle en C₁-C₄, (alcényle en C₃-C₆)-oxyalkyle en C₁-C₄, (alkyle en C₁-C₈)-carbonyle, 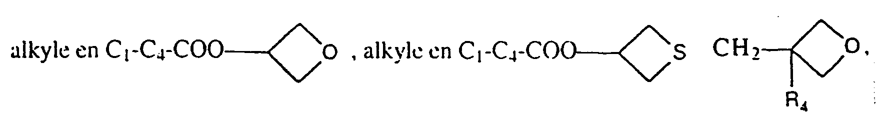 (alkyle en C₁-C₄)-thiocarbonylalkyle en C₁-C₄, ou le groupe 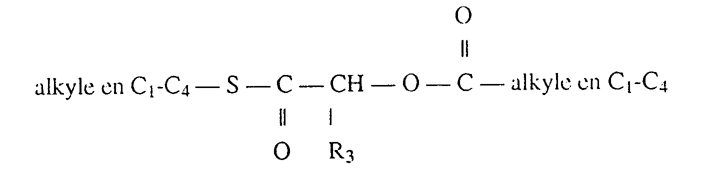
R₃ représente l'hydrogène ou un groupe alkyle en C₁-C₄ et
R₄ représente l'hydrogène ou un groupe alkyle en C₁-C₄,
caractérisé en ce que l'on convertit un isothiocyanate de formule II 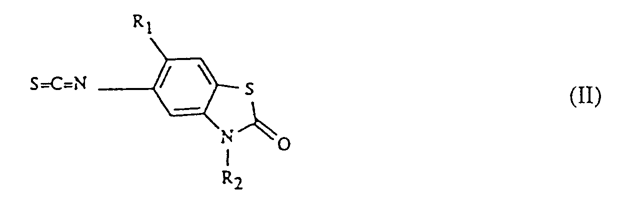 dans laquelle R₁ et R₂ ont les significations indiquées en référence à la formule I, à l'aide d'une hexahydropyridazine de formule III 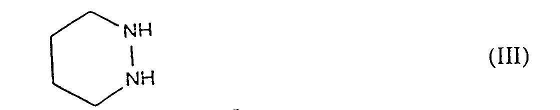 en le composé de formule IV 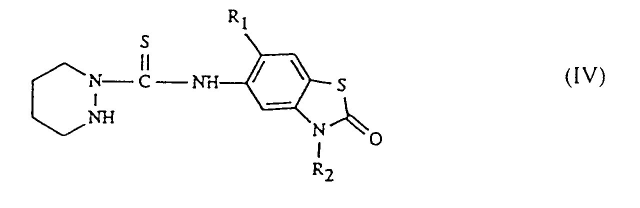 qu'on fait ensuite réagir avec un composé de formule V
CYCl₂ (V)
dans laquelle Y représente l'oxygène ou le soufre.

16. Un produit herbicide et inhibiteur de la croissance des végétaux, caractérisé, en ce qu'il contient un ou plusieurs dérivés de la benzothiazolone répondant à la formule I selon la revendication 1.

17. Produit selon la revendication 16, caractérisé en ce qu'il contient de 0,1 à 95 % d'une substance active de formule I selon la revendication 1.

18. Procédé pour combattre les croissances de végétaux indésirables, caractérisé en ce que l'on applique sur les végétaux ou leur habitat une quantité efficace d' une substance active de formule I selon la revendication 1 ou d'un produit contenant une telle substance active selon la revendication 16.

19. Procédé selon revendication 18, caractérisé en ce que l'on applique une quantité de substance active de 0,001 à 4 kg par ha.

20. Procédé selon revendication 18 pour la lutte sélective en pré-levée ou en post-levée contre les végétaux adventices dans les cultures de végétaux utiles.

21. Procédé pour inhiber la croissance des végétaux, caractérisé en ce que l'on applique sur les végétaux ou leur habitat une quantité efficace d'une substance active de formule I selon la revendication 1 ou d'un produit contenant une telle substance active selon la revendication 16.

## Revendications (Revendications pour l'(les) Etat(s) contractant(s) suivant(s): ES)

1. Procédé de préparation des composés de formule I 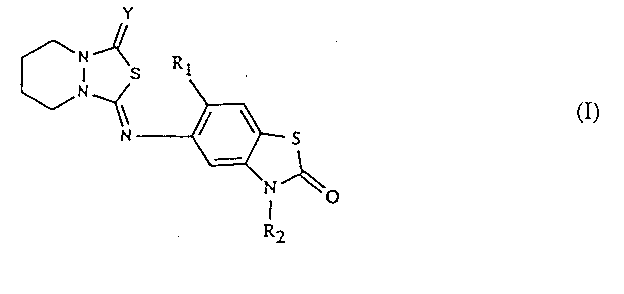 dans laquelle
Y représente l'oxygène ou le soufre ;
R₁ représente l'hydrogène ou le fluor et
R₂ représente l'hydrogène, un groupe alkyle en C₁-C₆, alcényle en C₂-C₄ ou alcynyle en C₂-C₆ ; un groupe alkyle en C₁-C₆, alcényle en C₂-C₄ ou alcynyle en C₃-C₆ substitué par des halogènes ; un groupe (alcoxy en C₁-C₄)-alkyle en C₁-C₄, (alcoxy en C₁-C₄)-(alcoxy en C₁-C₂)-alkyle en C₁-C₂, 1-phénylpropén-3-yle, alkyle en C₁-C₆ portant un substituant cyano ou cycloalkyle en C₃-C₆ ; carboxyalkyle en C₁-C₄, (alcoxy en C₁-C₆)-carbonylalkyle en C₁-C₄, halogéno-(alcoxy en C₁-C₆)-carbonylalkyle en C₁-C₄, (alcoxy en C₁-C₄)-(alcoxy en C₁-C₂)-carbonylalkyle en C₁-C₄, (alcoxy en C₁-C₆)-carbonyl-(alcoxy en C₁-C₂)-carbonylalkyle en C₁-C₄, (cycloalkyle en C₃-C₆)-(alcoxy en C₁-C2)-carbonylalkyle en C₁-C₄, (alkyle en C₁-C₅)-aminocarbonylalkyle en C₁-C₄, di-(alkyle en C₁-C₅)-aminocarbonylalkyle en C₁-C₄, cycloalkyle en C₃-C₆, (alkylthio en C₁-C₄)-alkyle en C₁-C₄, benzyle ou benzyle substitué par des halogènes, alkyl-sulfonyle en C₁-C₄, (alcényle en C₃-C₆)-oxyalkyle en C₁-C₄, (alkyle en C₁-C₈)-carbonyle, 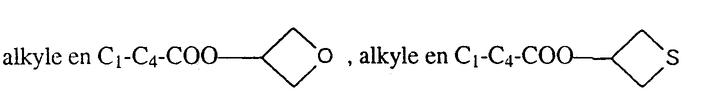 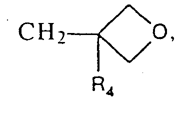 (alkyle en C₁-C₄)-thiocarbonylalkyle en C₁-C₄, ou le groupe 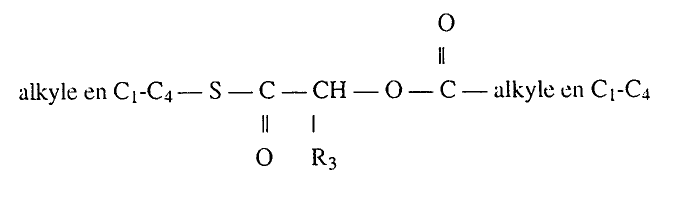
R₃ représente l'hydrogène ou un groupe alkyle en C₁-C₄ et
R₄ représente l'hydrogène ou un groupe alkyle en C₁-C₄
caractérisé en ce que l'on convertit un isothiocyanate de formule II 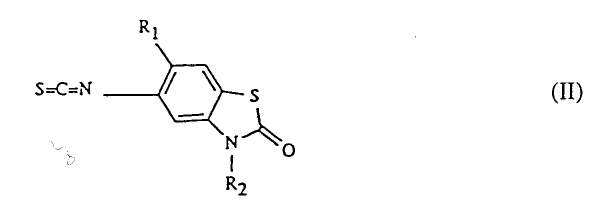 dans laquelle R1 et R₂ ont les significations indiquées en référence à la formule I, à l'aide d'une hexahydropyridazine de formule III 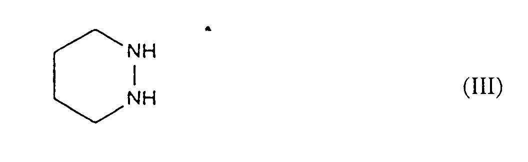 en le composé de formule IV 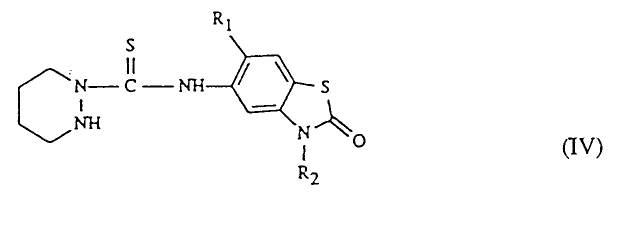 qu'on fait ensuite réagir avec un composé de formule V
CYCl₂ (V)
dans laquelle Y représente l'oxygène ou le soufre.

2. Procédé selon la revendication 1, pour la préparation des composés de formule I dans laquelle R₁ représente le fluor.

3. Procédé selon la revendication 1, pour la préparation des composés de formule I dans laquelle R₁ représente le fluor et Y l'oxygène.

4. Procédé selon la revendication 1, pour la préparation des composés de formule I dans laquelle R₂ représente l'hydrogène, un groupe alkyle en C₁-C₆, alcényle en C₂-C₄ ou alcynyle en C₂-C₆ ; un groupe alkyle en C₁-C₆, alcényle en C₂-C₄ ou alcynyle en C₃-C₆ substitué par des halogènes ; un groupe (alcoxy en C₁-C₄)-alkyle en C₁-C₄, (alcoxy en C₁-C₄)-(alcoxy en C₁-C₂)-alkyle en C₁-C₂, 1-phénylpropén-3-yle, alkyle en C₁-C₆ portant un substituant cyano ou cycloalkyle en C₃-C₆ ; carboxyalkyle en C₁-C₄, (alcoxy en C₁-C₆)-carbonylalkyle en C₁-C₄, halogéno-(alcoxy en C₁-C₆)-carbonylalkyle en C₁-C₄, (alcoxy en C₁-C₄)-(alcoxy en C₁-C₂)-carbonylalkyle en C₁-C₄, (alcoxy en C₁-C₆)-carbonyl-(alcoxy en C₁-C₂)-carbonylalkyle en C₁-C₄, (cycloalkyle en C₃-C₆)-(alcoxy en C₁-C2)-carbonylalkyle en C₁-C₄, (alkyle en C₁-C₅)-aminocarbonylalkyle en C₁-C₄, di-(alkyle en C₁-C₅)-aminocarbonylalkyle en C₁-C₄, cycloalkyle en C₃-C₆, (alkylthio en C₁-C₄)-alkyle en C₁-C₄, benzyle ou benzyle substitué par des halogènes, alkylsulfonyle en C₁-C₄, (alcényle en C₃-C₆)-oxyalkyle en C₁-C₄, (alkyle en C₁-C₈)-carbonyle, 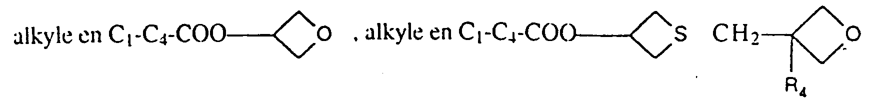 (alkyle en C₁-C₄)-thiocarbonylalkyle en C₁-C₄, ou le groupe 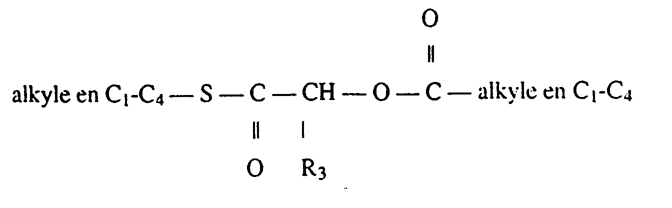 R₃ représente l'hydrogène ou un groupe alkyle en C₁-C₄ et
R₄ représente l'hydrogène ou un groupe alkyle en C₁-C₄.

5. Procédé selon la revendication 1 pour la préparation des composés de formule I dans laquelle R₂ représente l'hydrogène, un groupe alkyle en C₁-C₄, alcényle en C₂-C₄ ou alcynyle en C₂-C₄ ; un groupe alkyle en C₁-C₄, alcényle en C₂-C₄ ou alcynyle en C₃-C₄ substitué par des halogènes ; ou un groupe (alcoxy en C₁-C₂)-alkyle en C₁-C₂.

6. Procédé selon la revendication 1, pour la préparation des composés de formule I dans laquelle R² représente l'hydrogène, un groupe alkyle en C₁-C₄, alcényle en C₂-C₄, un groupe alkyle en C₁-C₄ ou alcényle en C₂-C₄ substitué par le fluor.

7. Procédé selon la revendication 1, pour la préparation des composés de formule I dans laquelle R₂ représente un groupe cycloalkyle en C₃-C₆, alkyle en C₁-C₆ substitué par un groupe cycloalkyle en C₃-C₆ ; un groupe (alkyle en C₁-C₄)-thioalkyle en C₁-C₄, un groupe benzyle éventuellement substitué par des halogènes, un groupe alkylsulfonyle en C₁- C₄, (alcényle en C₃-C₆)-oxyalkyle en C₁-C₄, (alkyle en C₁-C₈)-carbonyle, 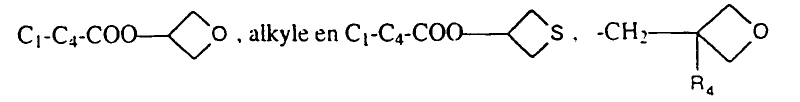 un groupe (alkyle en C₁-C₄)-thiocarbonylalkyle en C₁-C₄ ou le groupe 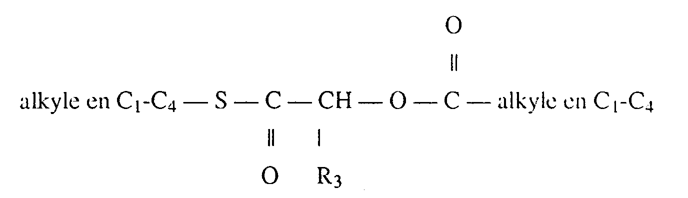 R₃ représente l'hydrogène ou un groupe alkyle en C₁-C₄
R₄ représente l'hydrogène ou un groupe alkyle en C₁-C₄,.

8. Procédé selon la revendication 7, pour la préparation des composés de formule I dans laquelle Y représente l'oxygène, R₁ le fluor et R₃ l'hydrogène ou un groupe méthyle.

9. Procédé selon la revendication 1, pour la préparation des composés de formule I dans laquelle Y représente l'oxygène, R₁ le fluor et R₂ un groupe alkyle en C₁-C₃, CH₂F, CH₂CH₂F, CH₂CH₂CH₂F, CHF₂, un groupe allyle, propargyle, 1-méthyl-3-propynyle, méthoxycarbonylméthyle, éthoxycarbonylméthyle, isopropoxycarbonylméthyle, 1-(méthoxycarbonyl)-éthyle, 1-(éthoxycarbonyl)éthyle ou 1-(iso-propoxycarbonyl)-éthyle.

10. Procédé selon la revendication 1, pour la préparation de la 9-[3-(1-méthyléthyl)-6-fluoro-2(3H)-benzothiazolone-5-yl)-8-thia-1,6-diazabicyclo[4.3.0]nonane]-one.

11. Procédé selon la revendication 1, pour la préparation des composés de formule I dans laquelle R₂ représente un groupe alkyle en C₁-C₆, alcényle en C₂-C₄ ou alcynyle en C₃-C₆ substitué par des halogènes ; un groupe (alcoxy en C₁-C₄)-alkyle en C₁-C₄, un groupe (alcoxy en C₁-C₄)-(alcoxy en C₁-C₂)-alkyle en C₁-C₂, un groupe alkyle en C₁-C₆ substitué par un groupe cyano ou cycloalkyle en C₃-C₆ ; un groupe carboxyalkyle en C₁-C₄, (alcoxy en C₁-C₆)-carbonylalkyle en C₁-C₄, un groupe (alcoxy en C₁-C₄)-(alcoxy en C₁-C₂)-carbonylalkyle en C₁-C₄, (alcoxy en C₁-C₆)-carbonyl-(alcoxy en C₁-C₂)-carbonylalkyle en C₁-C₄, (cycloalkyle en C₃-C₆)-(alcoxy en C₁-C₂)-carbonylalkyle en C₁-C₄, (alkyle en C₁-C₅)-aminocarbonylalkyle en C₁-C₄, di-(alkyle en C₁-C₅)-aminocarbonylalkyle en C₁-C₄, cycloalkyle en C₃-C₆, (alkylthio en C₁-C₄)-alkyle en C₁-C₄, (alcényle en C₃-C₆)-oxyalkyle en C₁-C₄, 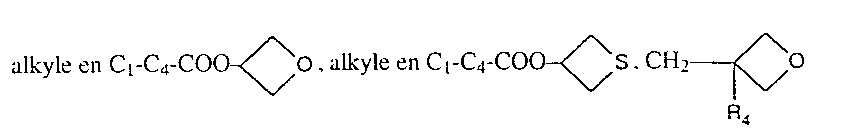 (alkyle en C₁-C₄)-thiocarbonyl-alkyle en C₁ - C₄ ou le groupe

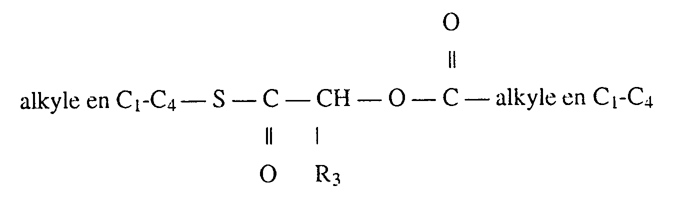

12. Procédé selon la revendication 11, pour la préparation des composés de formule I dans laquelle R₂ représente un groupe (alcoxy en C₁-C₄)-alkyle en C₁-C₄, (alcoxy en C₁-C₄)-(alcoxy en C₁-C₂)-alkyle en C₁-C₂, alkyle en C₁-C₆ substitué par un groupe cyano ; carboxyalkyle en C₁-C₄, (alcoxy en C₁-C₆)-carbonylalkyle en C₁-C₄, (alcoxy en C₁-C₄)-(alcoxy en C₁-C₂)-carbonylalkyle en C₁-C₄, (alcoxy en C₁-C₆)-carbonyl-(alcoxy en C₁-C₂)-carbonylalkyle en C₁-C₄, (cycloalkyle en C₃-C₆)-(alcoxy en C₁-C₂)-carbonyl-alkyle en C₁-C₄, (alkyle en C₁-C₅)-aminocarbonylalkyle en C₁-C₄, di-(alkyle en C₁-C₅)-aminocarbonylalkyle en C₁-C₄ ou cycloalkyle en C₃-C₆.

13. Procédé selon la revendication 12 pour la préparation des composés de formule I dans laquelle R₂ représente un groupe (alcoxy en C₁-C₆)-carbonyl-alkyle en C₁-C₄ ou (alcoxy en C₁-C₄)-(aloxy en C₁-C₂)-carbonyl-alkyle en C₁-C₄.

14. Procédé selon la revendication 11 pour la préparation des composés de formule I dans laquelle R₁ représente le fluor et Y l'oxygène.

15. Un produit herbicide et inhibiteur de la croissance des végétaux, caractérisé en ce qu'il contient un ou plusieurs dérivés de la benzothiazolone répondant à la formule I selon la revendication 1.

16. Produit selon la revendication 15 caractérisé en ce qu'il contient de 0,1 à 95 % d'une substance active de formule I selon la revendication 1.

17. Procédé pour combattre les croissances de végétaux indésirables caractérisé en ce que l'on applique sur les végétaux ou leur habitat une substance active de formule I selon la revendication 1 ou un produit contenant une telle substance active selon la revendication 15, en quantité efficace.

18. Procédé selon la revendication 17, caractérisé en ce que l'on applique une quantité de substance active de 0,001 à 4 kg par ha.

19. Procédé selon la revendication 17, pour la lutte sélective en pré-levée ou en post-levée contre les végétaux adventices dans les cultures de végétaux utiles.

20. Procédé pour inhiber la croissance des végétaux caractérisé en ce que l'on applique sur les végétaux ou leur habitat une substance active de formule I selon la revendication 1 ou un produit contenant une telle substance active selon la revendication 15 en quantité efficace.
